# EUROPEAN PATENT SPECIFICATION

(11) **EP 4 362 858 B1**
(45) Date of publication and mention of the grant of the patent: **09.09.2026**
(21) Application number: 22834028.7
(22) Date of filing: 28.06.2022
(51) Int. Cl.: A61F 2/44, A61F 2/46, A61B 17/70, A61B 34/10, A61B 34/30, G16H 30/40, G16H 40/63, G16H 50/20, G16H 50/50, G16H 50/30, G16H 50/70, G06N 20/00, G16H 10/60, G16H 15/00, G16H 20/40, G16H 30/20, A61B 34/00, A61B 90/00, A61B 90/50

(54) **PATIENT-SPECIFIC ADJUSTMENT OF SPINAL IMPLANTS AND ASSOCIATED SYSTEMS**
PATIENTENSPEZIFISCHE ANPASSUNG VON WIRBELSÄULENIMPLANTATEN SOWIE ZUGEHÖRIGE SYSTEME
AJUSTEMENT SPÉCIFIQUE AU PATIENT D'IMPLANTS RACHIDIENS ET SYSTÈMES ASSOCIÉS

(30) Priority: 28.06.2021 US 202163215784 P
(43) Date of publication of application: 08.05.2024
(73) Proprietor: Carlsmed, Inc., Carlsbad, CA 92008 (US)
(72) Inventor: CORDONNIER, Michael J., Carlsbad, California 92008 (US); CASEY, Niall Patrick, Carlsbad, California 92008 (US); HUSSAIN, Shariq, Vista, California 92081 (US)
(74) Representative: Murgitroyd & Company
(86) International application number: PCT/US2022/035232
(87) International publication number: WO 2023/278385

(56) References cited:
- WO-A1-2017/116346
- WO-A1-2020/055874
- WO-A1-2020/055874
- WO-A1-2020/079598
- US-A1- 2004 171 924
- US-A1- 2005 234 555
- US-A1- 2007 276 369
- US-A1- 2009 234 456
- US-A1- 2010 262 239
- US-A1- 2021 145 519
- US-A1- 2021 145 519
- US-B1- 10 902 944

## Description

### CROSS-REFERENCE TO RELATED APPLICATION

The present application claims priority to U.S. Provisional Patent Application No. 63/215,784, filed June 28, 2021.

### TECHNICAL FIELD

The present disclosure is generally related to designing and implementing medical care, and more particularly to systems and methods for designing and implementing patient-specific adjustment of spinal implants.

### BACKGROUND

Orthopedic implants are used to correct numerous different maladies in a variety of contexts, including spine surgery, hand surgery, shoulder and elbow surgery, total joint reconstruction (arthroplasty), skull reconstruction, pediatric orthopedics, foot and ankle surgery, musculoskeletal oncology, surgical sports medicine, and orthopedic trauma. Spine surgery itself may encompass a variety of procedures and targets, such as one or more of the cervical spine, thoracic spine, lumbar spine, or sacrum, and may be performed to treat a deformity or degeneration of the spine and/or related back pain, leg pain, or other body pain. Common spinal deformities that may be treated using an orthopedic implant include irregular spinal curvature such as scoliosis, lordosis, or kyphosis (hyper- or hypo-), and irregular spinal displacement (e.g., spondylolisthesis). Other spinal disorders that can be treated using an orthopedic implant include osteoarthritis, lumbar degenerative disc disease or cervical degenerative disc disease, lumbar spinal stenosis, and cervical spinal stenosis.
WO2020055874 describes a method for adjusting an adjustable implant that includes broadcasting identification information from an adjustable implant implanted within a subject, receiving wirelessly with the adjustable implant, from a device external to the subject, an adjustment setting configured to be executed by the adjustable implant, receiving wirelessly with the adjustable implant, from a device external to the subject, an instruction to perform the adjustment setting, activating a motor on the adjustable implant to perform the adjustment setting, and sending, to a device external to the subject, a progress of the adjustment setting.
US2005234555 details a prosthetic endplate having articulation surfaces whose relative positions can be post-operatively adjusted.
WO2020079598 describes a method for optimizing orthopedic spinal implant survival using preoperative finite element analysis combined with intraoperative stress analysis. Based on clinically relevant data, finite element analysis, and corrected values of spinal parameters, an acceptable long-term stress score is determined for an appropriate implant, which is selected from a set of potential implants, such that the shape of the implant minimizes predicted stress values.
US10902944 discloses systems and methods for designing and implementing patient-specific surgical procedures and/or medical devices. In some embodiments, a method includes receiving a patient data set of a patient. The patient data set is compared to a plurality of reference patient data sets, wherein each of the plurality of reference patient data sets is associated with a corresponding reference patient. A subset of the plurality of reference patient data sets is selected based, at least partly, on similarity to the patient data set and treatment outcome of the corresponding reference patient. Based on the selected subset, at least one surgical procedure or medical device design for treating the patient is generated.
US2009234456 discusses intervertebral implants and associated methods of implantation and treatment.
US2010262239 describes an orthopedic implant including at least one adjustment mechanism configured for securing to at least one orthopedic structure of a subject, and at least one motor operatively connected to the at least one adjustment mechanism, wherein the at least one motor is configured to move the at least one adjustment mechanism in at least three degrees of freedom.

### SUMMARY

Accordingly there is provided a system and a non-transitory, computer-readable storage medium as detailed in the independent claims. Advantageous features are detailed in the dependent claims.

### BRIEF DESCRIPTION OF THE DRAWINGS

The accompanying drawings illustrate various embodiments of systems, methods, and embodiments of various other aspects of the disclosure. While the surgical methods described herein do not form part of the invention, they are disclosed as they represent useful background for understanding the invention. Any person with ordinary skill in the art will appreciate that the illustrated element boundaries (e.g., boxes, groups of boxes, or other shapes) in the figures represent one example of the boundaries. It may be that in some examples one element may be designed as multiple elements or that multiple elements may be designed as one element. In some examples, an element shown as an internal component of one element may be implemented as an external component in another, and vice versa. Furthermore, elements may not be drawn to scale. Non-limiting and non-exhaustive descriptions are described with reference to the following drawings. The components in the figures are not necessarily to scale, with emphasis instead being placed upon illustrating principles.
Figure 1 is a network connection diagram illustrating a system for providing patient-specific medical care, according to an embodiment.
Figure 2 illustrates a computing device suitable for use in connection with the system of Figure, according to an embodiment.
Figure 3 is a flow diagram illustrating a method for providing patient-specific medical care, according to an embodiment.
Figures 4A-4C illustrate exemplary data sets that may be used and/or generated in connection with the methods described herein, according to an embodiment. Figure 4A illustrates a patient data set. Figure 4B illustrates reference patient data sets. Figure 4C illustrates similarity scores and outcome scores for the reference patient data sets of Figure 4B.
Figure 5 is a flow diagram illustrating another method for providing patient-specific medical care, according to an embodiment.
Figure 6 is a partially schematic illustration of an operative setup and associated computing systems for providing patient-specific medical care, according to an embodiment.
Figures 7A-7D illustrate an exemplary patient data set that may be used and/or generated in connection with the methods described herein, according to an embodiment.
Figures 8A and 8B illustrate an exemplary virtual model of a patient's spine that may be used and/or generated in connection with the methods described herein, according to an embodiment.
Figures 9A-1-9B-2 illustrate an exemplary virtual model of a patient's spine in a pre-operative anatomical configuration and a corrected anatomical configuration. More specifically, Figures 9A-1 and 9A-2 illustrate the pre-operative anatomical configuration of the patient; and Figures 9B-1 and 9B-2 illustrate the corrected anatomical configuration.
Figure 10 illustrates an exemplary surgical plan for a patient-specific surgical procedure that may be used and/or generated in connection with the methods described herein, according to an embodiment.
Figure 11 illustrates an exemplary surgical plan report detailing the surgical plan shown in Figure 10 for surgeon review and that may be used and/or generated in connection with the methods described herein, according to an embodiment.
Figures 12A and 12B illustrate an exemplary patient-specific implant that can be used and/or generated in connection with the methods described herein, according to an embodiment.
Figure 13 illustrates a segment of a patient's spine after several patient-specific implants have been implanted therein, according to an embodiment.
Figures 14A and 14B are schematic anterior and side illustrations, respectively, of a deployed patient-specific intervertebral body fusion device deployed between a first vertebra (e.g., a relatively superior vertebra) and a second vertebra (e.g., a relatively inferior vertebra), according to an embodiment.
Figure 15 is a flow diagram illustrating a process for patient-specific adjustment of spinal implants, according to an embodiment.
Figure 16A shows a patient's spine and a remote device for controlling actuation of intervertebral implants, according to an embodiment.
Figure 16B illustrates an exemplary corrective plan that may be used and/or generated in connection with the systems and methods described herein, according to an embodiment.
Figures 17A-17D show a patient's spine in different configurations, according to an embodiment.
Figure 18 is a side view of an intervertebral body fusion device and a fixation for the spine, according to an embodiment.

### DETAILED DESCRIPTION

The present technology is directed to systems and methods for designing and implementing patient-specific adjustment of spinal implants (the surgical methods do not form part of the invention). Spinal fusion, also called spondylodesis or spondylosyndesis, is a neurosurgical or orthopedic surgical technique that joins two or more vertebrae. Spinal fusion can be used to treat a variety of conditions affecting any level of the spine-lumbar, cervical, and thoracic. In general, spinal fusion is performed to decompress and stabilize the spine, and the result can prevent any movement between the fused vertebrae. Spinal fusion is most commonly performed to relieve the pain and pressure from mechanical pain of the vertebrae or on the spinal cord that results when a disc wears out (e.g., resulting from degenerative disc disease). Other common pathological conditions that are treated by spinal fusion include spinal stenosis, spondylolisthesis, spondylosis, spinal fractures, scoliosis, and kyphosis.

A spine fusion surgery can utilize intervertebral body fusion (IBF) devices. The IBF device can help to restore a height between vertebral bodies, restore lordotic and coronal misalignment, and/or stabilize the spine until bony fusion occurs between vertebral bodies. Example IBF devices can be configured for anterior lumbar interbody fusion (ALIF), lateral lumbar interbody fusion (LLIF), oblique lateral interbody fusion, posterior lumbar interbody fusion (PLIF), or transforaminal lumbar interbody fusion (TLIF). In some embodiments, the IBF device can be a cervical cage. IBF devices can also have multiple expandable mechanisms that provide intraoperative adjustability. In some embodiments, expandable IBF devices also provide adjustability (e.g., pre-, intra-, and/or postoperative adjustability) of, for example, spinal curvature, vertebral heights, lordotic restoration, and/or coronal restoration.

Although the disclosure herein primarily describes systems and methods for treatment planning in the context of orthopedic surgery, the technology may be applied equally to medical treatment and devices in other fields (e.g., other types of surgical practice). Additionally, although many embodiments herein describe systems and methods with respect to implanted devices, while not claimed, the technology may be applied equally to other types of medical devices (e.g., non-implanted devices).

For example, in many of the embodiments disclosed herein, a computer system receives implant sensor readings from one or more implant sensors of a spinal implant configured in a first physical configuration according to a corrective plan for the patient. The implant sensor readings are received after the surgery is performed and are indicative of a load applied by a spine of the patient on the spinal implant. The computer system extracts a feature vector from the implant sensor readings using a machine learning module of the computer system. The feature vector is indicative of a target correction. The computer system generates implant electrical signals using the machine learning module based on the feature vector. The machine learning module is trained based on patient data sets to generate the implant electrical signals to adjust the load, e.g., to achieve the target correction. The computer system transmits the implant electrical signals to the spinal implant to cause the spinal implant to move the spinal implant to a second physical configuration for the target correction. In some embodiments, the spinal implant is adjusted post-operatively in accordance with a predetermined adjustable-implant corrective plan that accounts for one or more of disease progression, additional surgical interventions, aging, sensed metrics, or the like.

In some embodiments, the computer system receives patient data. An anatomical configuration of the patient's spine is determined based on the received patient data. The computer system identifies the target correction based on the anatomical configuration and available adjustability of the spinal implant, wherein the identified target correction is used to extract the feature vector.

In some embodiments, the corrective plan comprises criteria for actuating the spinal implant.

In some embodiments, the computer system receives device sensor readings from one or more device sensors embedded in an intervertebral fusion device implant implanted in the patient during the surgery. The device sensor readings are received after the surgery is performed and before the implant sensor readings are received. The computer system generates device electrical signals using the machine learning module based on the device sensor readings. The machine learning module is trained based on the patient data sets to generate the device sensor readings to reduce physical discomfort caused by the intervertebral fusion device.

In some embodiments, the feature vector is further indicative of at least one of lumbar lordosis (LL), Cobb angles, coronal parameters, sagittal parameters, pelvic parameters, disc height, segment flexibility, bone quality, or rotational displacement of the spine of the patient.

In some embodiments, configuring the spinal implant in the second physical configuration includes adjusting at least one of a screw, a cage, a plate, a rod, a disk, a spacer, an expandable device, a stent, a bracket, a tie, a scaffold, a fixation device, an anchor, a nut, a bolt, a rivet, a connector, a tether, a fastener, or a joint replacement of the spinal implant using the one or more implant actuators.

In some embodiments, configuring the spinal implant in the second physical configuration includes adjusting a reservoir coupled to the spinal implant to modify an amount of at least one of a pharmaceutical, biologic, biochemical, narcotic, or a steroid delivered to the patient.

In some embodiments, a method of providing medical care includes comparing a patient data set of a patient to be treated with multiple reference patient data sets (e.g., data from previously treated patients). The method can include selecting a subset of the reference patient data sets, e.g., based on similarity of the reference patient data set to the patient data set and/or whether the reference patient had a favorable treatment outcome. The selected subset can be used to generate a surgical procedure and/or medical device design that is likely to produce a favorable treatment outcome for the particular patient. In some embodiments, the selected subset is analyzed to identify correlations between patient pathology, surgical procedures, device designs, and/or treatment outcomes, and these correlations are used to determine a personalized treatment protocol with a higher likelihood of success.

In the context of orthopedic surgery, systems with improved computing capabilities (e.g., predictive analytics, machine learning, neural networks, artificial intelligence (Al)) can use large data sets to define improved or optimal surgical interventions and/or implant designs for a specific patient. The patient's entire data can be characterized and compared to aggregated data from groups of prior patients (e.g., parameters, metrics, pathologies, treatments, outcomes). In some embodiments, the systems described herein use this aggregated data to formulate potential treatment solutions (e.g., surgical plans and/or implant designs for spine and orthopedic procedures) and analyze the associated likelihood of success. These systems can further compare potential treatment solutions to determine an optimal patient-specific solution that is expected to maximize the likelihood for a successful outcome.

For example, if a patient presents with a spinal deformity pathology that can be described with data including lumbar lordosis, Cobb angles, coronal parameters (e.g., coronal balance, global coronal balance, coronal pelvic tilt, etc.), sagittal parameters (e.g., pelvic incidence (PI), sacral slope, thoracic kyphosis, etc.) and/or pelvic parameters, an algorithm using these data points as inputs can be used to describe an optimal surgical plan and/or implant design to correct the subject pathology and improve the patient's outcome. As additional data inputs are used to describe the pathology (e.g., disc height, segment flexibility, bone quality, rotational displacement), the algorithm can use these additional inputs to further define an optimal surgical plan and/or implant design for that particular patient and their pathology.

In some embodiments, the present technology can automatically or at least semi-automatically determine a corrected anatomical configuration for a subject patient suffering from one or more deformities. For example, the computing systems described herein can apply mathematical rules for select parameters (e.g., LL, Cobb angles, etc.) and/or identify similar patients by analyzing reference patient data sets, and, based on the rules and/or comparison to other patients, can provide a recommended anatomical configuration that represents the optimal outcome if the subject patient were to undergo surgery. In some embodiments, the systems and methods described herein generate a virtual model of the corrected/recommended anatomical configuration (e.g., for surgeon review).

In some embodiments, the present technology can also automatically or at least semi-automatically generate a surgical plan for achieving a previously identified corrected anatomical configuration for a subject patient. For example, based off the virtual model of the corrected anatomical configuration, the systems and methods herein can determine a type of surgery (e.g., spinal fusion surgery, non-fusion surgery, etc.), a surgical approach (e.g., anterior, posterior, etc.), and/or spinal parameters for the corrected anatomical configuration (e.g., LL, Cobb angles, etc.). The surgical plan can be transmitted to a surgeon for review and approval. In some embodiments, the present technology can also design one or more patient-specific implants for achieving the corrected anatomical configuration via the surgical plan.

In some embodiments, the present technology provides systems and methods that generate multiple anatomical models of the patient. For example, a first model may show the patient's native (e.g., pre-operative) anatomical configuration, and a second model may provide a simulation of the patient's corrected (e.g., post-operative) anatomical configuration. The second virtual model may optionally include one or more virtual implants shown as implanted at one or more target regions of the patient. Spine metrics (e.g., LL, Cobb angles, coronal parameters, sagittal parameters, pelvic parameters, etc.) can also be provided for both the pre-operative anatomical configuration and expected post-operative anatomical configuration.

In some embodiments, the present technology includes generating, designing, and/or providing patient-specific medical procedures for multiple locations within a patient. For example, the present technology can include identifying at least two target regions or sites within a patient (e.g., a first vertebral level and a second vertebral level) for surgical intervention. The present technology can then design at least two patient-specific implants for implantation at the at least two target regions. The at least two patient-specific implants can each be specifically designed for their respective target region, and thus can have different geometries. In some embodiments, the corrected anatomical configuration of the patient is only achieved by implanting each of the at least two patient-specific implants. In the context of spinal surgery, for example, the present technology may provide a first patient-specific interbody device to be implanted between the L2 and L3 vertebrae, a second patient-specific interbody device to be implanted between the L3 and L4 vertebrae, and a third patient-specific interbody device to be implanted between the L4 and L5 vertebrae.

In some embodiments, the present technology can predict, model, or simulate disease progression within a particular patient to aid in diagnosis and/or treatment planning. The simulation can be done to model and/or estimate future anatomical configurations and/or spine metrics of the patient (a) if no surgical intervention occurs, or (b) for a variety of different surgical intervention options. The progression modeling can thus be used to determine the optimal time for surgical intervention and/or to select which surgical intervention provides the best long-term outcomes. In some embodiments, the disease progression modelling is performed using one or more machine learning models trained based on multiple reference patients.

In a particular non-limiting example, the present technology includes a method for providing patient-specific medical care for a subject patient. The method can include receiving a patient-data set for the subject patient that includes one or more images of the patient's spinal region showing the patient's native anatomical configuration. The method can further include determining a corrected anatomical configuration for the subject patient that is different than the native anatomical configuration and creating a virtual model of the corrected anatomical configuration. The method can further include generating a surgical plan and designing one or more patient-specific implants for achieving the corrected anatomical configuration in the subject patient. In representative embodiments, the foregoing method can be performed by a system storing computer-executable instructions that, when executed, cause the system to perform the steps of method.

In a particular non-limiting example, the present technology includes a method for designing a patient-specific orthopedic implant for a subject patient. The method can include receiving a patient data set of the subject patient, the patient data set including spinal pathology data for the subject patient. The patient data set can be compared to multiple reference patient data sets to identify one or more similar patient data sets in the reference patient data sets, with each identified similar patient data set corresponding to a reference patient having similar spinal pathology to the subject patient and who received treatment with an orthopedic implant. The method can further include selecting a subset of the one or more similar patient data sets based on whether the similar patient data sets indicated the reference patient had a favorable outcome following implantation of their orthopedic implant. The method can further include identifying, for at least one similar reference patient of the selected subset, surgical procedure data and design data for the respective orthopedic implant that produced the favorable outcome in the similar reference patient. Based on the design data and the surgical data that produced the favorable outcome in the similar reference patient, the patient-specific orthopedic implant for the subject patient and a surgical procedure for implanting the patient-specific orthopedic implant into the subject patient can be designed. In some embodiments, the method can further include outputting fabrication instructions for causing a manufacturing system to manufacture the patient-specific orthopedic implant according to the generated design. In representative embodiments, the foregoing method can be performed by a system storing computer-executable instructions that, when executed, cause the system to perform the steps of method.

In some embodiments, IBF devices are personalized to patient-specific features and/or concerns in accordance with a pre-operative plan for height restoration, lordotic and coronal correction, and/or optimal endplate coverage. For example, an expandable IBF device in accordance with the present technology can include patient-specific endplates that can achieve optimal surface area contact and/or provide a mechanism to tailor the medical intervention from the IBF device (e.g., tailor the segmental height restoration, the lordotic correction, and/or the coronal correction). In some embodiments, the patient-specific endplates are the result of additive and/or subtractive manufacturing. The patient-specific endplates can then be connected to an expandable mechanism that can also provide a predetermined height restoration, lordotic correction, and/or coronal correction via one or more expansion mechanisms (e.g., an expandable jack, scissor jack mechanism, screw drive mechanism, etc.). In some such embodiments, the expansion mechanism includes one or more joints (e.g., ball joints), hinges, or other connections that can be precisely adjusted to a predetermined angle and then temporarily or permanently locked.

In an example embodiment, the IBF device includes an expansion mechanism configured to be locked at a desired expansion configuration to facilitate the fusion. The expansion mechanism can include a first lockable ball joint on an upper surface of the mechanism and a second lockable ball joint on a lower surface of the mechanism. The IBF device also includes a first endplate connected to the mechanism at the first lockable ball joint. In some embodiments, the first endplate includes a superior surface having one or more patient-specific features configured to engage and mate with the topology of an inferior surface of the superior vertebra. The IBF device also includes a second endplate connected to the mechanism at the second lockable ball joint. In some embodiments, the second endplate includes an inferior surface having one or more patient-specific features configured to engage and mate with the topology of a superior surface of the inferior vertebra.

In some embodiments, the patient-specific features of the first and/or second endplates can improve the match between the IBF device and the vertebrae being fused, thereby increasing the traction of the IBF device at the joint. For example, the one or more patient-specific features can correspond to topographical features on the surfaces of the vertebrae at the vertebral joint to customize the fit of the first and second endplates. In some embodiments, the patient-specific features of the first and/or second endplates include one or more features that help facilitate a prescribed medical treatment. For example, the first and/or second endplates can include a slope that helps provide a lordotic and/or coronal correction to the patient's spine. In some embodiments, the expandable main body includes a screw jack mechanical expansion mechanism. In some embodiments the expandable main body includes a scissor jack mechanical expansion mechanism.

For ease of reference, patient-specific implants are sometimes described herein with reference to top and bottom, upper and lower, upwards and downwards, and/or horizontal plane, x-y plane, vertical, or z-direction relative to the spatial orientation of the embodiments shown in the figures. It is to be understood, however, that the patient-specific implants can be moved to, and used in, different spatial orientations without changing the structure and/or function of the disclosed embodiments of the present technology.

Embodiments of the present disclosure will be described more fully hereinafter with reference to the accompanying drawings in which like numerals represent like elements throughout the several figures, and in which example embodiments are shown. Embodiments of the claims may, however, be embodied in many different forms and should not be construed as limited to the embodiments set forth herein. The examples set forth herein are non-limiting examples and are merely examples among other possible examples.

The words "comprising," "having," "containing," and "including," and other forms thereof, are intended to be equivalent in meaning and be open ended in that an item or items following any one of these words is not meant to be an exhaustive listing of such item or items, or meant to be limited to only the listed item or items.

As used herein and in the appended claims, the singular forms "a," "an," and "the" include plural references unless the context clearly dictates otherwise.

Further, although primarily discussed herein as a method for customizing intervertebral body fusion devices and the resulting IBF devices, one of skill in the art will understand that the scope of the invention is not so limited. For example, the patient-specific customization methods disclosed herein can also be used to customize implants for various other medical procedures, such as for insertion at another joint in a patient's body. Accordingly, the scope of the invention is not confined to any subset of embodiments and is confined only by the limitations set out in the appended claims.

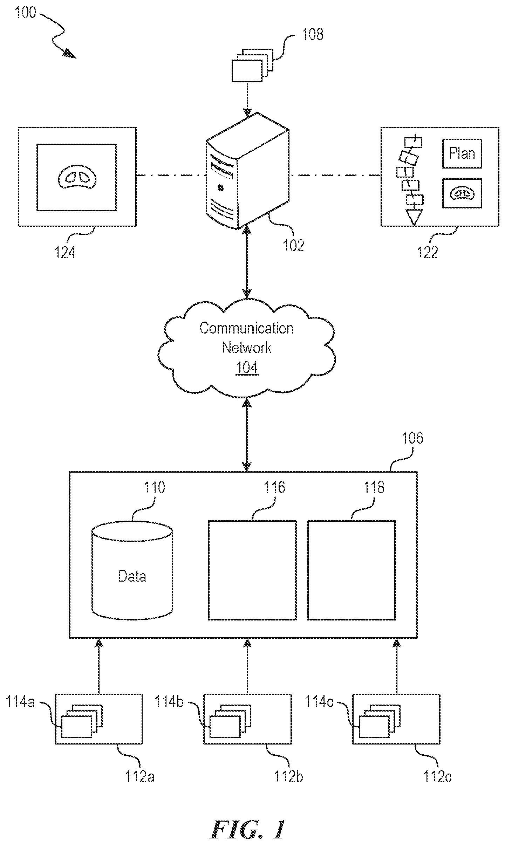

Figure 1 is a network connection diagram illustrating a computing system 100 for providing patient-specific medical care, according to an embodiment. As described in further detail herein, the system 100 is configured to generate a medical treatment plan for a patient. In some embodiments, the system 100 is configured to generate a medical treatment plan for a patient suffering from an orthopedic or spinal disease or disorder, such as trauma (e.g., fractures), cancer, deformity, degeneration, pain (e.g., back pain, leg pain), irregular spinal curvature (e.g., scoliosis, lordosis, kyphosis), irregular spinal displacement (e.g., spondylolisthesis, lateral displacement axial displacement), osteoarthritis, lumbar degenerative disc disease, cervical degenerative disc disease, lumbar spinal stenosis, or cervical spinal stenosis, or a combination thereof. The medical treatment plan can include surgical information, surgical plans, technology recommendations (e.g., device and/or instrument recommendations), and/or medical device designs. For example, the medical treatment plan can include at least one treatment procedure (e.g., a surgical procedure or intervention) and/or at least one medical device (e.g., an implanted medical device (also referred to herein as an "implant" or "implanted device") or implant delivery instrument).

In some embodiments, the system 100 generates a medical treatment plan that is customized for a particular patient or group of patients, also referred to herein as a "patient-specific" or "personalized" treatment plan. The patient-specific treatment plan can include at least one patient-specific surgical procedure and/or at least one patient-specific medical device that are designed and/or optimized for the patient's particular characteristics (e.g., condition, anatomy, pathology, condition, medical history). For example, the patient-specific medical device can be designed and manufactured specifically for the particular patient, rather than being an off-the-shelf device. However, it shall be appreciated that a patient-specific treatment plan can also include aspects that are not customized for the particular patient. For example, a patient-specific or personalized surgical procedure can include one or more instructions, portions, steps, etc. that are non-patient-specific. Likewise, a patient-specific or personalized medical device can include one or more components that are non-patient-specific, and/or can be used with an instrument or tool that is non-patient-specific. Personalized implant designs can be used to manufacture or select patient-specific technologies, including medical devices, instruments, and/or surgical kits. For example, a personalized surgical kit can include one or more patient-specific devices, patient-specific instruments, non-patient-specific technology (e.g., standard instruments, devices, etc.), instructions for use, patient-specific treatment plan information, or a combination thereof.

The system 100 includes a client computing device 102, which can be a user device, such as a smart phone, mobile device, laptop, desktop, personal computer, tablet, phablet, or other such devices known in the art. As discussed further herein, the client computing device 102 can include one or more processors and memory storing instructions executable by the one or more processors to perform the methods described herein. The client computing device 102 can be associated with a healthcare provider that is treating the patient. Although Figure 1 illustrates a single client computing device 102, in alternative embodiments, the client computing device 102 can instead be implemented as a client computing system encompassing multiple computing devices, such that the operations described herein with respect to the client computing device 102 can instead be performed by the computing system and/or the computing devices.

The client computing device 102 is configured to receive a patient data set 108 associated with a patient to be treated. The patient data set 108 can include data representative of the patient's condition, anatomy, pathology, medical history, preferences, and/or any other information or parameters relevant to the patient. For example, the patient data set 108 can include medical history, surgical intervention data, treatment outcome data, progress data (e.g., physician notes), patient feedback (e.g., feedback acquired using quality of life questionnaires, surveys), clinical data, provider information (e.g., physician, hospital, surgical team), patient information (e.g., demographics, sex, age, height, weight, type of pathology, occupation, activity level, tissue information, health rating, comorbidities, health related quality of life (HRQL)), vital signs, diagnostic results, medication information, allergies, image data (e.g., camera images, magnetic resonance imaging (MRI) images, ultrasound images, computerized aided tomography (CAT) scan images, positron emission tomography (PET) images, X-Ray images), diagnostic equipment information (e.g., manufacturer, model number, specifications, user-selected settings/configurations, etc.), or the like. In some embodiments, the patient data set 108 includes data representing one or more of patient identification number (ID), age, gender, body mass index (BMI), LL, Cobb angle(s), PI, disc height, segment flexibility, bone quality, rotational displacement, and/or treatment level of the spine.

The client computing device 102 is operably connected via a communication network 104 to a server 106, thus allowing for data transfer between the client computing device 102 and the server 106. The communication network 104 may be a wired and/or a wireless network. The communication network 104, if wireless, may be implemented using communication techniques such as Visible Light Communication (VLC), Worldwide Interoperability for Microwave Access (WiMAX), Long term evolution (LTE), Wireless local area network (WLAN), Infrared (IR) communication, Public Switched Telephone Network (PSTN), Radio waves, and/or other communication techniques known in the art.

The server 106, which may also be referred to as a "treatment assistance network" or "prescriptive analytics network," can include one or more computing devices and/or systems. As discussed further herein, the server 106 can include one or more processors, and memory storing instructions executable by the one or more processors to perform the methods described herein. In some embodiments, the server 106 is implemented as a distributed "cloud" computing system or facility across any suitable combination of hardware and/or virtual computing resources.

The client computing device 102 and server 106 can individually or collectively perform the various methods described herein for providing patient-specific medical care. For example, some or all of the steps of the methods described herein can be performed by the client computing device 102 alone, the server 106 alone, or a combination of the client computing device 102 and the server 106. Thus, although certain operations are described herein with respect to the server 106, it shall be appreciated that these operations can also be performed by the client computing device 102, and vice-versa.

The server 106 includes at least one database 110 configured to store reference data useful for the treatment planning methods described herein. The reference data can include historical and/or clinical data from the same or other patients, data collected from prior surgeries and/or other treatments of patients by the same or other healthcare providers, data relating to medical device designs, data collected from study groups or research groups, data from practice databases, data from academic institutions, data from implant manufacturers or other medical device manufacturers, data from imaging studies, data from simulations, clinical trials, demographic data, treatment data, outcome data, mortality rates, or the like.

In some embodiments, the database 110 includes multiple reference patient data sets, each patient reference data set associated with a corresponding reference patient. For example, the reference patient can be a patient that previously received treatment or is currently receiving treatment. Each reference patient data set can include data representative of the corresponding reference patient's condition, anatomy, pathology, medical history, disease progression, preferences, and/or any other information or parameters relevant to the reference patient, such as any of the data described herein with respect to the patient data set 108. In some embodiments, the reference patient data set includes pre-operative data, intra-operative data, and/or post-operative data. For example, a reference patient data set can include data representing one or more of patient ID, age, gender, BMI, LL, Cobb angle(s), PI, disc height, segment flexibility, bone quality, rotational displacement, and/or treatment level of the spine. As another example, a reference patient data set can include treatment data regarding at least one treatment procedure performed on the reference patient, such as descriptions of surgical procedures or interventions (e.g., surgical approaches, bony resections, surgical maneuvers, corrective maneuvers, placement of implants or other devices). In some embodiments, the treatment data includes medical device design data for at least one medical device used to treat the reference patient, such as physical properties (e.g., size, shape, volume, material, mass, weight), mechanical properties (e.g., stiffness, strength, modulus, hardness), and/or biological properties (e.g., osteo-integration, cellular adhesion, anti-bacterial properties, anti-viral properties). In yet another example, a reference patient data set can include outcome data representing an outcome of the treatment of the reference patient, such as corrected anatomical metrics, presence of fusion, HRQL, activity level, return to work, complications, recovery times, efficacy, mortality, and/or follow-up surgeries.

In some embodiments, the server 106 receives at least some of the reference patient data sets from multiple healthcare provider computing systems (e.g., systems 112a-112c, collectively 112). The server 106 can be connected to the healthcare provider computing systems 112 via one or more communication networks (not shown). Each healthcare provider computing system 112 can be associated with a corresponding healthcare provider (e.g., physician, surgeon, medical clinic, hospital, healthcare network, etc.). Each healthcare provider computing system 112 can include at least one reference patient data set (e.g., reference patient data sets 114a-114c, collectively 114) associated with reference patients treated by the corresponding healthcare provider. The reference patient data sets 114 can include, for example, electronic medical records, electronic health records, biomedical data sets, etc. The reference patient data sets 114 can be received by the server 106 from the healthcare provider computing systems 112 and can be reformatted into different formats for storage in the database 110. Optionally, the reference patient data sets 114 can be processed (e.g., cleaned) to ensure that the represented patient parameters are likely to be useful in the treatment planning methods described herein.

As described in further detail herein, the server 106 can be configured with one or more algorithms that generate patient-specific treatment plan data (e.g., treatment procedures, medical devices) based on the reference data. In some embodiments, the patient-specific data is generated based on correlations between the patient data set 108 and the reference data. Optionally, the server 106 can predict outcomes, including recovery times, efficacy based on clinical end points, likelihood of success, predicted mortality, predicted related follow-up surgeries, or the like. In some embodiments, the server 106 can continuously or periodically analyze patient data (including patient data obtained during the patient stay) to determine near real-time or real-time risk scores, mortality prediction, etc.

In some embodiments, the server 106 includes one or more modules for performing one or more steps of the patient-specific treatment planning methods described herein. For example, in the depicted embodiment, the server 106 includes a data analysis module 116 and a treatment planning module 118. In alternative embodiments, one or more of these modules may be combined with each other, or may be omitted. Thus, although certain operations are described herein with respect to a particular module or modules, this is not intended to be limiting, and such operations can be performed by a different module or modules in alternative embodiments.

The data analysis module 116 is configured with one or more algorithms for identifying a subset of reference data from the database 110 that is likely to be useful in developing a patient-specific treatment plan. For example, the data analysis module 116 can compare patient-specific data (e.g., the patient data set 108 received from the client computing device 102) to the reference data from the database 110 (e.g., the reference patient data sets) to identify similar data (e.g., one or more similar patient data sets in the reference patient data sets). The comparison can be based on one or more parameters, such as age, gender, BMI, LL, PI, and/or treatment levels. The parameter(s) can be used to calculate a similarity score for each reference patient. The similarity score can represent a statistical correlation between the patient data set 108 and the reference patient data set. Accordingly, similar patients can be identified based on whether the similarity score is above, below, or at a specified threshold value. For example, as described in greater detail below, the comparison can be performed by assigning values to each parameter and determining the aggregate difference between the subject patient and each reference patient. Reference patients whose aggregate difference is below a threshold can be considered to be similar patients.

The data analysis module 116 can further be configured with one or more algorithms to select a subset of the reference patient data sets, e.g., based on similarity to the patient data set 108 and/or treatment outcome of the corresponding reference patient. For example, the data analysis module 116 can identify one or more similar patient data sets in the reference patient data sets, and then select a subset of the similar patient data sets based on whether the similar patient data set includes data indicative of a favorable or desired treatment outcome. The outcome data can include data representing one or more outcome parameters, such as corrected anatomical metrics, presence of fusion, HRQL, activity level, complications, recovery times, efficacy, mortality, or follow-up surgeries. As described in further detail below, in some embodiments, the data analysis module 116 calculates an outcome score by assigning values to each outcome parameter. A patient can be considered to have a favorable outcome if the outcome score is above, below, or at a specified threshold value.

In some embodiments, the data analysis module 116 selects a subset of the reference patient data sets based at least in part on user input (e.g., from a clinician, surgeon, physician, healthcare provider). For example, the user input can be used in identifying similar patient data sets. In some embodiments, weighting of similarity and/or outcome parameters can be selected by a healthcare provider or physician to adjust the similarity and/or outcome score based on clinician input. In further embodiments, the healthcare provider or physician can select the set of similarity and/or outcome parameters (or define new similarity and/or outcome parameters) used to generate the similarity and/or outcome score, respectively.

In some embodiments, the data analysis module 116 includes one or more algorithms used to select a set or subset of the reference patient data sets based on criteria other than patient parameters. For example, the one or more algorithms can be used to select the subset based on healthcare provider parameters (e.g., based on healthcare provider ranking/scores such as hospital/physician expertise, number of procedures performed, hospital ranking, etc.) and/or healthcare resource parameters (e.g., diagnostic equipment, facilities, surgical equipment such as surgical robots), or other non-patient related information that can be used to predict outcomes and risk profiles for procedures for the present healthcare provider. For example, reference patient data sets with images captured from similar diagnostic equipment can be aggregated to reduce or limit irregularities due to variation between diagnostic equipment. Additionally, patient-specific treatment plans can be developed for a particular health-care provider using data from similar healthcare providers (e.g., healthcare providers with traditionally similar outcomes, physician expertise, surgical teams, etc.). In some embodiments, reference healthcare provider data sets, hospital data sets, physician data sets, surgical team data sets, post-treatment data set, and other data sets can be utilized. By way of example, a patient-specific treatment plan to perform a battlefield surgery can be based on reference patient data from similar battlefield surgeries and/or datasets associated with battlefield surgeries. In another example, the patient-specific treatment plan can be generated based on available robotic surgical systems. The reference patient data sets can be selected based on patients that have been operated on using comparable robotic surgical systems under similar conditions (e.g., size and capabilities of surgical teams, hospital resources, etc.).

The treatment planning module 118 is configured with one or more algorithms to generate at least one treatment plan (e.g., pre-operative plans, surgical plans, post-operative plans, etc.) based on the output from the data analysis module 116. In some embodiments, the treatment planning module 118 is configured to develop and/or implement at least one predictive model for generating the patient-specific treatment plan, also known as a "prescriptive model." The predictive model(s) can be developed using clinical knowledge, statistics, machine learning, Al, neural networks, or the like. In some embodiments, the output from the data analysis module 116 is analyzed (e.g., using statistics, machine learning, neural networks, AI) to identify correlations between data sets, patient parameters, healthcare provider parameters, healthcare resource parameters, treatment procedures, medical device designs, and/or treatment outcomes. These correlations can be used to develop at least one predictive model that predicts the likelihood that a treatment plan will produce a favorable outcome for the particular patient. The predictive model(s) can be validated, e.g., by inputting data into the model(s) and comparing the output of the model to the expected output.

In some embodiments, the treatment planning module 118 is configured to generate the treatment plan based on previous treatment data from reference patients. For example, the treatment planning module 118 can receive a selected subset of reference patient data sets and/or similar patient data sets from the data analysis module 116, and determine or identify treatment data from the selected subset. The treatment data can include, for example, treatment procedure data (e.g., surgical procedure or intervention data) and/or medical device design data (e.g., implant design data) that are associated with favorable or desired treatment outcomes for the corresponding patient. The treatment planning module 118 can analyze the treatment procedure data and/or medical device design data to determine an optimal treatment protocol for the patient to be treated. For example, the treatment procedures and/or medical device designs can be assigned values and aggregated to produce a treatment score. The patient-specific treatment plan can be determined by selecting treatment plan(s) based on the score (e.g., higher or highest score; lower or lowest score; score that is above, below, or at a specified threshold value). The personalized patient-specific treatment plan can be based on, at least in part, the patient-specific technologies or patient-specific selected technology.

Alternatively or in combination, the treatment planning module 118 can generate the treatment plan based on correlations between data sets. For example, the treatment planning module 118 can correlate treatment procedure data and/or medical device design data from similar patients with favorable outcomes (e.g., as identified by the data analysis module 116). Correlation analysis can include transforming correlation coefficient values to values or scores. The values/scores can be aggregated, filtered, or otherwise analyzed to determine one or more statistical significances. These correlations can be used to determine treatment procedure(s) and/or medical device design(s) that are optimal or likely to produce a favorable outcome for the patient to be treated.

Alternatively or in combination, the treatment planning module 118 can generate the treatment plan using one or more AI techniques. AI techniques can be used to develop computing systems capable of simulating aspects of human intelligence, e.g., learning, reasoning, planning, problem solving, decision making, etc. AI techniques can include, but are not limited to, case-based reasoning, rule-based systems, artificial neural networks, decision trees, support vector machines, regression analysis, Bayesian networks (e.g., naïve Bayes classifiers), genetic algorithms, cellular automata, fuzzy logic systems, multi-agent systems, swarm intelligence, data mining, machine learning (e.g., supervised learning, unsupervised learning, reinforcement learning), and hybrid systems.

In some embodiments, the treatment planning module 118 generates the treatment plan using one or more trained machine learning models. Various types of machine learning models, algorithms, and techniques are suitable for use with the present technology. In some embodiments, the machine learning model is initially trained on a training data set, which is a set of examples used to fit the parameters (e.g., weights of connections between "neurons" in artificial neural networks) of the model. For example, the training data set can include any of the reference data stored in database 110, such as multiple reference patient data sets or a selected subset thereof (e.g., multiple similar patient data sets).

In some embodiments, the machine learning model (e.g., a neural network or a naïve Bayes classifier) may be trained on the training data set using a supervised learning method (e.g., gradient descent or stochastic gradient descent). The training dataset can include pairs of generated "input vectors" with the associated corresponding "answer vector" (commonly denoted as the target). The current model is run with the training data set and produces a result, which is then compared with the target, for each input vector in the training data set. Based on the result of the comparison and the specific learning algorithm being used, the parameters of the model are adjusted. The model fitting can include both variable selection and parameter estimation. The fitted model can be used to predict the responses for the observations in a second data set called the validation data set. The validation data set can provide an unbiased evaluation of a model fit on the training data set while tuning the model parameters. Validation data sets can be used for regularization by early stopping, e.g., by stopping training when the error on the validation data set increases, as this may be a sign of overfitting to the training data set. In some embodiments, the validation data set error can fluctuate during training, such that ad-hoc rules may be used to decide when overfitting has truly begun. Finally, a test data set can be used to provide an unbiased evaluation of a final model fit on the training data set.

To generate a treatment plan, the patient data set 108 can be input into the trained machine learning model(s). Additional data, such as the selected subset of reference patient data sets and/or similar patient data sets, and/or treatment data from the selected subset, can also be input into the trained machine learning model(s). The trained machine learning model(s) can then calculate whether various candidate treatment procedures and/or medical device designs are likely to produce a favorable outcome for the patient. Based on these calculations, the trained machine learning model(s) can select at least one treatment plan for the patient. In embodiments where multiple trained machine learning models are used, the models can be run sequentially or concurrently to compare outcomes and can be periodically updated using training data sets. The treatment planning module 118 can use one or more of the machine learning models based the model's predicted accuracy score.

The patient-specific treatment plan generated by the treatment planning module 118 can include at least one patient-specific treatment procedure (e.g., a surgical procedure or intervention; not claimed) and/or at least one patient-specific medical device (e.g., an implant or implant delivery instrument). A patient-specific treatment plan can include an entire surgical procedure or portions thereof. Additionally, one or more patient-specific medical devices can be specifically selected or designed for the corresponding surgical procedure, thus allowing for the various components of the patient-specific technology to be used in combination to treat the patient.

In some embodiments, the patient-specific treatment procedure includes an orthopedic surgery procedure (not claimed), such as spinal surgery, hip surgery, knee surgery, jaw surgery, hand surgery, shoulder surgery, elbow surgery, total joint reconstruction (arthroplasty), skull reconstruction, foot surgery, or ankle surgery. Spinal surgery can include spinal fusion surgery, such as PLIF, ALIF, transverse or TLIF, LLIF, direct lateral lumbar interbody fusion (DLIF), or extreme lateral lumbar interbody fusion (XLIF). In some embodiments, the patient-specific treatment procedure includes descriptions of and/or instructions for performing one or more aspects of a patient-specific surgical procedure. For example, the patient-specific surgical procedure can include one or more of a surgical approach, a corrective maneuver, a bony resection, or implant placement.

In some embodiments, the patient-specific medical device design includes a design for an orthopedic implant and/or a design for an instrument for delivering an orthopedic implant. Examples of such implants include, but are not limited to, screws (e.g., bone screws, spinal screws, pedicle screws, facet screws), interbody implant devices (e.g., intervertebral implants, expandable intervertebral implants, etc.), cages, plates, rods, discs, fusion devices, spacers, rods, expandable devices, stents, brackets, ties, scaffolds, fixation devices, anchors, nuts, bolts, rivets, connectors, tethers, fasteners, joint replacements, hip implants, or the like. Examples of instruments include, but are not limited to, screw guides, cannulas, ports, catheters, insertion tools, or the like.

A patient-specific medical device design can include data representing one or more of physical properties (e.g., size, shape, volume, material, mass, weight), mechanical properties (e.g., stiffness, strength, modulus, hardness), and/or biological properties (e.g., osteo-integration, cellular adhesion, anti-bacterial properties, anti-viral properties) of a corresponding medical device. For example, a design for an orthopedic implant can include implant shape, size, material, and/or effective stiffness (e.g., lattice density, number of struts, location of struts, etc.). In some embodiments, the generated patient-specific medical device design is a design for an entire device. Alternatively, the generated design can be for one or more components of a device, rather than the entire device.

In some embodiments, the design is for one or more patient-specific device components that can be used with standard, off-the-shelf components. For example, in a spinal surgery, a pedicle screw kit can include both standard components and patient-specific customized components. In some embodiments, the generated design is for a patient-specific medical device that can be used with a standard, off-the-shelf delivery instrument. For example, the implants (e.g., screws, screw holders, rods) can be designed and manufactured for the patient, while the instruments for delivering the implants can be standard instruments. This approach allows the components that are implanted to be designed and manufactured based on the patient's anatomy and/or surgeon's preferences to enhance treatment. The patient-specific devices described herein are expected to improve delivery into the patient's body, placement at the treatment site, and/or interaction with the patient's anatomy.

In embodiments where the patient-specific treatment plan includes a surgical procedure (not claimed) to implant a medical device, the treatment planning module 118 can also store various types of implant surgery information, such as implant parameters (e.g., types, dimensions), availability of implants, aspects of a pre-operative plan (e.g., initial implant configuration, detection and measurement of the patient's anatomy, etc.), FDA requirements for implants (e.g., specific implant parameters and/or characteristics for compliance with FDA regulations), or the like. In some embodiments, the treatment planning module 118 can convert the implant surgery information into formats useable for machine-learning based models and algorithms. For example, the implant surgery information can be tagged with particular identifiers for formulas or can be converted into numerical representations suitable for supplying to the trained machine learning model(s). The treatment planning module 118 can also store information regarding the patient's anatomy, such as two- or three-dimensional images or models of the anatomy, and/or information regarding the biology, geometry, and/or mechanical properties of the anatomy. The anatomy information can be used to inform implant design and/or placement.

The treatment plan(s) generated by the treatment planning module 118 can be transmitted via the communication network 104 to the client computing device 102 for output to a user (e.g., clinician, surgeon, healthcare provider, patient). In some embodiments, the client computing device 102 includes or is operably coupled to a display 122 for outputting the treatment plan(s), surgical plan(s), corrective plan(s), etc. For example, the surgical plan can be displayed to illustrate a predicted post-operative outcome. The corrective plan can be an adjustable-implant corrective plan displayed to illustrate post-operative implant adjustments to compensate for one or more of disease progression, predicted new disease(s), anatomical changes, future interventions, etc. The display 122 can display adjustability of one or more implants 123 according to the corrective plan.

The display 122 can include a graphical user interface (GUI) for visually depicting various aspects of the treatment plan(s). For example, the display 122 can show various aspects of a surgical procedure to be performed on the patient, such as the surgical approach, treatment levels, corrective maneuvers, tissue resection, and/or implant placement. To facilitate visualization, a virtual model of the surgical procedure can be displayed. As another example, the display 122 can show a design for a medical device to be implanted in the patient, such as a two- or three-dimensional model of the device design. The display 122 can also show patient information, such as two- or three-dimensional images or models of the patient's anatomy where the surgical procedure is to be performed and/or where the device is to be implanted. The client computing device 102 can further include one or more user input devices (not shown) allowing the user to modify, select, approve, and/or reject the displayed treatment plan(s).

In some embodiments, the medical device design(s) generated by the treatment planning module 118 can be transmitted from the client computing device 102 and/or server 106 to a manufacturing system 124 for manufacturing a corresponding medical device. The manufacturing system 124 can be located on site or off site. Onsite manufacturing can reduce the number of sessions with a patient and/or the time to be able to perform the surgery whereas off-site manufacturing can be useful to make the complex devices. Off-site manufacturing facilities can have specialized manufacturing equipment. In some embodiments, more complicated device components can be manufactured off site, while simpler device components can be manufactured on site.

Various types of manufacturing systems are suitable for use in accordance with the embodiments herein. For example, the manufacturing system 124 can be configured for additive manufacturing, such as three-dimensional (3D) printing, stereolithography (SLA), digital light processing (DLP), fused deposition modeling (FDM), selective laser sintering (SLS), selective laser melting (SLM), selective heat sintering (SHM), electronic beam melting (EBM), laminated object manufacturing (LOM), powder bed printing (PP), thermoplastic printing, direct material deposition (DMD), inkjet photo resin printing, or like technologies, or combination thereof. Alternatively or in combination, the manufacturing system 124 can be configured for subtractive (traditional) manufacturing, such as CNC machining, electrical discharge machining (EDM), grinding, laser cutting, water jet machining, manual machining (e.g., milling, lathe/turning), or like technologies, or combinations thereof. The manufacturing system 124 can manufacture one or more patient-specific medical devices based on fabrication instructions or data (e.g., CAD data, 3D data, digital blueprints, stereolithography data, or other data suitable for the various manufacturing technologies described herein). Different components of the system 100 can generate at least a portion of the manufacturing data used by the manufacturing system 124. The manufacturing data can include, without limitation, fabrication instructions (e.g., programs executable by additive manufacturing equipment, subtractive manufacturing equipment, etc.), 3D data, CAD data (e.g., CAD files), CAM data (e.g., CAM files), path data (e.g., print head paths, tool paths, etc.), material data, tolerance data, surface finish data (e.g., surface roughness data), regulatory data (e.g., FDA requirements, reimbursement data, etc.), or the like. The manufacturing system 124 can analyze the manufacturability of the implant design based on the received manufacturing data. The implant design can be finalized by altering geometries, surfaces, etc. and then generating manufacturing instructions. In some embodiments, the server 106 generates at least a portion of the manufacturing data, which is transmitted to the manufacturing system 124.

The manufacturing system 124 can generate CAM data, print data (e.g., powder bed print data, thermoplastic print data, photo resin data, etc.), or the like and can include additive manufacturing equipment, subtractive manufacturing equipment, thermal processing equipment, or the like. The additive manufacturing equipment can be 3D printers, stereolithography devices, DLP devices, FDM devices, SLS devices, SLM devices, EBM devices, LOM devices, powder bed printers, thermoplastic printers, DMD devices, or inkjet photo resin printers, or like technologies. The subtractive manufacturing equipment can be CNC machines, electrical discharge machines, grinders, laser cutters, water jet machines, manual machines (e.g., milling machines, lathes, etc.), or like technologies. Both additive and subtractive techniques can be used to produce implants with complex geometries, surface finishes, material properties, etc. The generated fabrication instructions can be configured to cause the manufacturing system 124 to manufacture the patient-specific orthopedic implant that matches or is therapeutically the same as the patient-specific design. In some embodiments, the patient-specific medical device can include features, materials, and designs shared across designs to simplify manufacturing. For example, deployable patient-specific medical devices for different patients can have similar internal deployment mechanisms but have different deployed configurations. In some embodiments, the components of the patient-specific medical devices are selected from a set of available pre-fabricated components and the selected pre-fabricated components can be modified based on the fabrication instructions or data.

The treatment plans described herein can be performed by a surgeon, a surgical robot, or a combination thereof, thus allowing for treatment flexibility. In some embodiments, the surgical procedure can be performed entirely by a surgeon, entirely by a surgical robot, or a combination thereof. For example, one step of a surgical procedure can be manually performed by a surgeon and another step of the procedure can be performed by a surgical robot. In some embodiments the treatment planning module 118 generates control instructions configured to cause a surgical robot (e.g., robotic surgery systems, navigation systems, etc.) to partially or fully perform a surgical procedure. The control instructions can be transmitted to the robotic apparatus by the client computing device 102 and/or the server 106.

Following the treatment of the patient in accordance with the treatment plan, treatment progress can be monitored over one or more time periods to update the data analysis module 116 and/or treatment planning module 118. Post-treatment data can be added to the reference data stored in the database 110. The post-treatment data can be used to train machine learning models for developing patient-specific treatment plans, patient-specific medical devices, or combinations thereof.

It shall be appreciated that the components of the system 100 can be configured in many different ways. For example, in alternative embodiments, the database 110, the data analysis module 116 and/or the treatment planning module 118 can be components of the client computing device 102, rather than the server 106. As another example, the database 110 the data analysis module 116, and/or the treatment planning module 118 can be located across multiple different servers, computing systems, or other types of cloud-computing resources, rather than at a single server 106 or client computing device 102.

Additionally, in some embodiments, the system 100 can be operational with numerous other computing system environments or configurations. Examples of computing systems, environments, and/or configurations that may be suitable for use with the technology include, but are not limited to, personal computers, server computers, handheld or laptop devices, cellular telephones, wearable electronics, tablet devices, multiprocessor systems, microprocessor-based systems, programmable consumer electronics, network PCs, minicomputers, mainframe computers, distributed computing environments that include any of the above systems or devices, or the like.

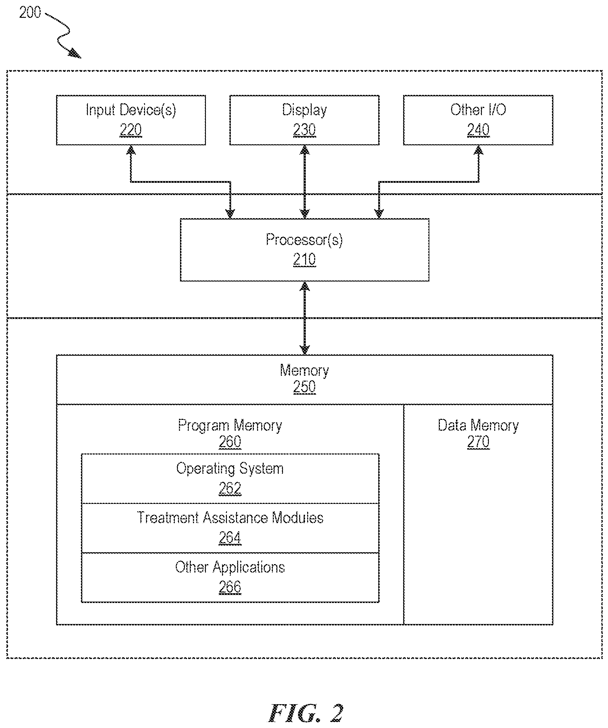

Figure 2 illustrates a computing device 200 suitable for use in connection with the system 100 of Figure 1, according to an embodiment. The computing device 200 can be incorporated in various components of the system 100 of Figure 1, such as the client computing device 102 or the server 106. The computing device 200 includes one or more processors 210 (e.g., CPU(s), GPU(s), HPU(s), etc.). The processor(s) 210 can be a single processing unit or multiple processing units in a device or distributed across multiple devices. The processor(s) 210 can be coupled to other hardware devices, for example, with the use of a bus, such as a PCI bus or SCSI bus. The processor(s) 210 can be configured to execute one more computer-readable program instructions, such as program instructions to carry out of any of the methods described herein.

The computing device 200 can include one or more input devices 220 that provide input to the processor(s) 210, e.g., to notify it of actions from a user of the device 200. The actions can be mediated by a hardware controller that interprets the signals received from the input device and communicates the information to the processor(s) 210 using a communication protocol. Input device(s) 220 can include, for example, a mouse, a keyboard, a touchscreen, an infrared sensor, a touchpad, a wearable input device, a camera- or image-based input device, a microphone, or other user input devices.

The computing device 200 can include a display 230 used to display various types of output, such as text, models, virtual procedures, surgical plans, implants, graphics, and/or images (e.g., images with voxels indicating radiodensity units or Hounsfield units representing the density of the tissue at a location). In some embodiments, the display 230 provides graphical and textual visual feedback to a user. The processor(s) 210 can communicate with the display 230 via a hardware controller for devices. In some embodiments, the display 230 includes the input device(s) 220 as part of the display 230, such as when the input device(s) 220 include a touchscreen or is equipped with an eye direction monitoring system. In alternative embodiments, the display 230 is separate from the input device(s) 220. Examples of display devices include an LCD display screen, an LED display screen, a projected, holographic, or augmented reality display (e.g., a heads-up display device or a head-mounted device), and so on.

Optionally, other I/O devices 240 can also be coupled to the processor(s) 210, such as a network card, video card, audio card, USB, firewire or other external device, camera, printer, speakers, CD-ROM drive, DVD drive, disk drive, or Blu-Ray device. Other I/O devices 240 can also include input ports for information from directly connected medical equipment such as imaging apparatuses, including MRI machines, X-Ray machines, CT machines, etc. Other I/O devices 240 can further include input ports for receiving data from these types of machine from other sources, such as across a network or from previously captured data, for example, stored in a database.

In some embodiments, the computing device 200 also includes a communication device (not shown) capable of communicating wirelessly or wire-based with a network node. The communication device can communicate with another device or a server through a network using, for example, TCP/IP protocols. The computing device 200 can utilize the communication device to distribute operations across multiple network devices, including imaging equipment, manufacturing equipment, etc.

The computing device 200 can include memory 250, which can be in a single device or distributed across multiple devices. Memory 250 includes one or more of various hardware devices for volatile and non-volatile storage, and can include both read-only and writable memory. For example, a memory can comprise random access memory (RAM), various caches, CPU registers, read-only memory (ROM), and writable non-volatile memory, such as flash memory, hard drives, floppy disks, CDs, DVDs, magnetic storage devices, tape drives, device buffers, and so forth. A memory is not a propagating signal divorced from underlying hardware; a memory is thus non-transitory. In some embodiments, the memory 250 is a non-transitory computer-readable storage medium that stores, for example, programs, software, data, or the like. In some embodiments, memory 250 can include program memory 260 that stores programs and software, such as an operating system 262, one or more treatment assistance modules 264, and other application programs 266. The treatment assistance module(s) 264 can include one or more modules configured to perform the various methods described herein (e.g., the data analysis module 116 and/or treatment planning module 118 described with respect to Figure 1). Memory 250 can also include data memory 270 that can include, e.g., reference data, configuration data, settings, user options or preferences, etc., which can be provided to the program memory 260 or any other element of the computing device 200.

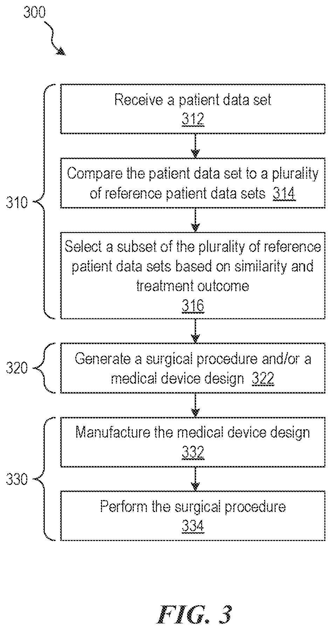

Figure 3 is a flow diagram illustrating a method 300 for providing patient-specific medical care, according to an embodiment. The method 300 can include a data phase 310, a modeling phase 320, and an execution phase 330. The data phase 310 can include collecting data of a patient to be treated (e.g., pathology data), and comparing the patient data to reference data (e.g., prior patient data such as pathology, surgical, and/or outcome data). For example, a patient data set can be received (block 312). The patient data set can be compared to multiple reference patient data sets (block 314), e.g., in order to identify one or more similar patient data sets in the reference patient data sets. Each of the reference patient data sets can include data representing one or more of age, gender, BMI, LL, Cobb angle(s), PI, disc height, segment flexibility, bone quality, rotational displacement, or treatment level of the spine.

A subset of the reference patient data sets can be selected (block 316), e.g., based on similarity to the patient data set and/or treatment outcomes of the corresponding reference patients. For example, a similarity score can be generated for each reference patient data set, based on the comparison of the patient data set and the reference patient data set. The similarity score can represent a statistical correlation between the patient data and the reference patient data set. One or more similar patient data sets can be identified based, at least partly, on the similarity score.

In some embodiments, each patient data set of the selected subset includes and/or is associated with data indicative of a favorable treatment outcome (e.g., a favorable treatment outcome based on a single target outcome, aggregate outcome score, outcome thresholding). The data can include, for example, data representing one or more of corrected anatomical metrics, presence of fusion, health related quality of life, activity level, or complications. In some embodiments, the data is or includes an outcome score, which can be calculated based on a single target outcome, an aggregate outcome, and/or an outcome threshold.

Optionally, the data analysis phase 310 can include identifying or determining, for at least one patient data set of the selected subset (e.g., for at least one similar patient data set), surgical procedure data and/or medical device design data associated with the favorable treatment outcome. The surgical procedure data can include data representing one or more of a surgical approach, a corrective maneuver, a bony resection, or implant placement. The at least one medical device design can include data representing one or more of physical properties, mechanical properties, or biological properties of a corresponding medical device. In some embodiments, the at least one patient-specific medical device design includes a design for an implant or an implant delivery instrument.

In the modeling phase 320, a surgical procedure and/or medical device design is generated (block 322). The generating step can include developing at least one predictive model based on the patient data set and/or selected subset of reference patient data sets (e.g., using statistics, machine learning, neural networks, Al, or the like). The predictive model can be configured to generate the surgical procedure and/or medical device design.

In some embodiments, the predictive model includes one or more trained machine learning models that generate, at least partly, the surgical procedure and/or medical device design. For example, the trained machine learning model(s) can determine multiple candidate surgical procedures and/or medical device designs for treating the patient. Each surgical procedure can be associated with a corresponding medical device design. In some embodiments, the surgical procedures and/or medical device designs are determined based on surgical procedure data and/or medical device design data associated with favorable outcomes, as previously described with respect to the data analysis phase 310. For each surgical procedure and/or corresponding medical device design, the trained machine learning model(s) can calculate a probability of achieving a target outcome (e.g., favorable or desired outcome) for the patient. The trained machine learning model(s) can then select at least one surgical procedure and/or corresponding medical device design based, at least partly, on the calculated probabilities.

The execution phase 330 can include manufacturing the medical device design (block 332). In some embodiments, the medical device design is manufactured by a manufacturing system configured to perform one or more of additive manufacturing, 3D printing, stereolithography, DLP, FDM, SLS, SLM, EBM, LOM, PP, thermoplastic printing, DMD, or inkjet photo resin printing. The execution phase 330 can optionally include generating fabrication instructions configured to cause the manufacturing system to manufacture a medical device having the medical device design.

The execution phase 330 can include performing the surgical procedure (block 334). The surgical procedure can involve implanting a medical device having the medical device design into the patient. The surgical procedure can be performed manually, by a surgical robot, or a combination thereof. In embodiments where the surgical procedure is performed by a surgical robot, the execution phase 330 can include generating control instructions configured to cause the surgical robot to perform, at least partly, the patient-specific surgical procedure.

The method 300 can be implemented and performed in various ways. In some embodiments, one or more steps of the method 300 (e.g., the data phase 310 and/or the modeling phase 320) can be implemented as computer-readable instructions stored in memory and executable by one or more processors of any of the computing devices and systems described herein (e.g., the system 100), or a component thereof (e.g., the client computing device 102 and/or the server 106). Alternatively, one or more steps of the method 300 (e.g., the execution phase 330) can be performed by a healthcare provider (e.g., physician, surgeon), a robotic apparatus (e.g., a surgical robot), a manufacturing system (e.g., manufacturing system 124), or a combination thereof. In some embodiments, one or more steps of the method 300 are omitted (e.g., the execution phase 330).

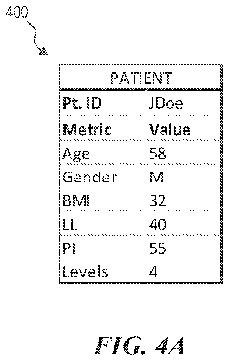

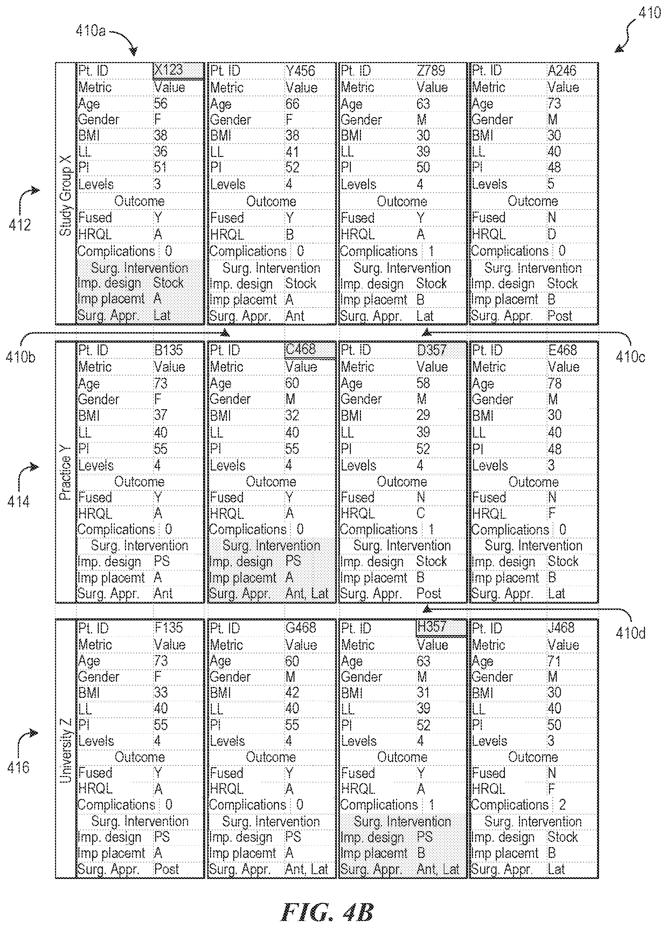

Figures 4A-4C illustrate exemplary data sets that may be used and/or generated in connection with the methods described herein (e.g., the data analysis phase 310 described with respect to Figure 3), according to an embodiment. Figure 4A illustrates a patient data set 400 of a patient to be treated. The patient data set 400 can include a patient ID and multiple pre-operative patient metrics (e.g., age, gender, BMI, LL, PI, and treatment levels of the spine (levels)). Figure 4B illustrates multiple reference patient data sets 410. In the depicted embodiment, the reference patient data sets 410 include a first subset 412 from a study group (Study Group X), a second subset 414 from a practice database (Practice Y), and a third subset 416 from an academic group (University Z). In alternative embodiments, the reference patient data sets 410 can include data from other sources, as previously described herein. Each reference patient data set can include a patient ID, multiple pre-operative patient metrics (e.g., age, gender, BMI, LL, PI, and treatment levels of the spine (levels)), treatment outcome data (Outcome) (e.g., presence of fusion (fused), HRQL, complications), and treatment procedure data (Surg. Intervention) (e.g., implant design, implant placement, surgical approach).

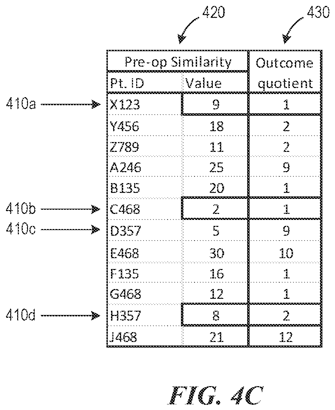

Figure 4C illustrates comparison of the patient data set 400 to the reference patient data sets 410. As previously described, the patient data set 400 can be compared to the reference patient data sets 410 to identify one or more similar patient data sets from the reference patient data sets. In some embodiments, the patient metrics from the reference patient data sets 410 are converted to numeric values and compared the patient metrics from the patient data set 400 to calculate a similarity score 420 ("Pre-op Similarity") for each reference patient data set. Reference patient data sets having a similarity score below a threshold value can be considered to be similar to the patient data set 400. For example, in the depicted embodiment, reference patient data set 410a has a similarity score of 9, reference patient data set 410b has a similarity score of 2, reference patient data set 410c has a similarity score of 5, and reference patient data set 410d has a similarity score of 8. Because each of these scores are below the threshold value of 10, reference patient data sets 410a-d are identified as being similar patient data sets.

The treatment outcome data of the similar patient data sets 410a-d can be analyzed to determine surgical procedures and/or implant designs with the highest probabilities of success. For example, the treatment outcome data for each reference patient data set can be converted to a numerical outcome score 430 ("Outcome Quotient") representing the likelihood of a favorable outcome. In the depicted embodiment, reference patient data set 410a has an outcome score of 1, reference patient data set 410b has an outcome score of 1, reference patient data set 410c has an outcome score of 9, and reference patient data set 410d has an outcome score of 2. In embodiments where a lower outcome score correlates to a higher likelihood of a favorable outcome, reference patient data sets 410a, 410b, and 410d can be selected. The treatment procedure data from the selected reference patient data sets 410a, 410b, and 410d can then be used to determine at least one surgical procedure (e.g., implant placement, surgical approach) and/or implant design that is likely to produce a favorable outcome for the patient to be treated.

In some embodiments, a method for providing medical care to a patient is provided. The method can include comparing a patient data set to reference data. The patient data set and reference data can include any of the data types described herein. The method can include identifying and/or selecting relevant reference data (e.g., data relevant to treatment of the patient, such as data of similar patients and/or data of similar treatment procedures), using any of the techniques described herein. A treatment plan can be generated based on the selected data, using any of the techniques described herein. The treatment plan can include one or more treatment procedures (e.g., surgical procedures, instructions for procedures, models or other virtual representations of procedures), one or more medical devices (e.g., implanted devices, instruments for delivering devices, surgical kits), or a combination thereof.

In some embodiments, a system for generating a medical treatment plan is provided. The system can compare a patient data set to multiple reference patient data sets, using any of the techniques described herein. A subset of the reference patient data sets can be selected, e.g., based on similarity and/or treatment outcome, or any other technique as described herein. A medical treatment plan can be generated based at least in part on the selected subset, using any of the techniques described herein. The medical treatment plan can include one or more treatment procedures, one or more medical devices, or any of the other aspects of a treatment plan described herein, or combinations thereof.

In further embodiments, a system is configured to use historical patient data. The system can select historical patient data to develop or select a treatment plan, design medical devices, or the like. Historical data can be selected based on one or more similarities between the present patient and prior patients to develop a prescriptive treatment plan designed for desired outcomes. The prescriptive treatment plan can be tailored for the present patient to increase the likelihood of the desired outcome. In some embodiments, the system can analyze and/or select a subset of historical data to generate one or more treatment procedures, one or more medical devices, or a combination thereof. In some embodiments, the system can use subsets of data from one or more groups of prior patients, with favorable outcomes, to produce a reference historical data set used to, for example, design, develop or select the treatment plan, medical devices, or combinations thereof.

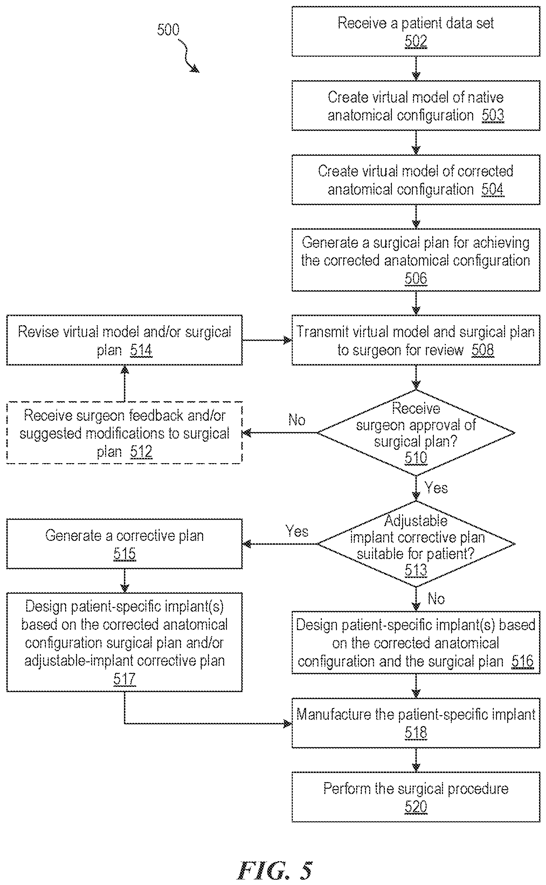

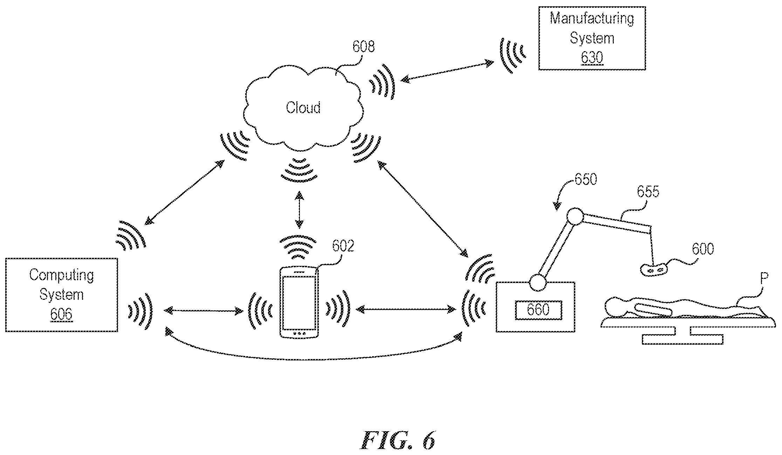

Figure 5 is a flow diagram illustrating a method 500 for providing patient-specific medical care, according to another embodiment of the present technology. The method 500 can begin in step 502 by receiving a patient data set for a particular patient in need of medical treatment. The patient data set can include data representative of the patient's condition, anatomy, pathology, symptoms, medical history, preferences, and/or any other information or parameters relevant to the patient. For example, the patient data set 808 can include surgical intervention data, treatment outcome data, progress data (e.g., surgeon notes), patient feedback (e.g., feedback acquired using quality of life questionnaires, surveys), clinical data, patient information (e.g., demographics, sex, age, height, weight, type of pathology, occupation, activity level, tissue information, health rating, comorbidities, health related quality of life (HRQL)), vital signs, diagnostic results, medication information, allergies, diagnostic equipment information (e.g., manufacturer, model number, specifications, user-selected settings/configurations, etc.) or the like. The patient data set can also include image data, such as camera images, MRI images, ultrasound images, CAT scan images, PET images, X-Ray images, and the like. In some embodiments, the patient data set includes data representing one or more of patient identification number (ID), age, gender, BMI, LL, Cobb angle(s), PI, disc height, segment flexibility, bone quality, rotational displacement, and/or treatment level of the spine. The patient data set can be received at a server, computing device, or other computing system. For example, in some embodiments the patient data set can be received by the server 106 shown in Figure 1 or the computing system 606 described below with respect to Figure 6. In some embodiments, the computing system that receives the patient data set in step 502 also stores one or more software modules (e.g., the data analysis module 116 and/or the treatment planning module 118, shown in Figure 1, or additional software modules for performing various operations of the method 500). Additional details for collecting and receiving the patient data set are described below with respect to Figures 6-7D.

In some embodiments, the received patient data set can include disease metrics such as LL, Cobb angles, coronal parameters (e.g., coronal balance, global coronal balance, coronal pelvic tilt, etc.), sagittal parameters (e.g., PI, sacral slope, thoracic kyphosis, etc.) and/or pelvic parameters. The disease metrics can include micro-measurements (e.g., metrics associated with specific or individual segments of the patient's spine) and/or macro-measurements (e.g., metrics associated with multiple segments of the patient's spine). In some embodiments, the disease metrics are not included in the patient data set, and the method 500 includes determining (e.g., automatically determining) one or more of the disease metrics based on the patient image data, as described below.

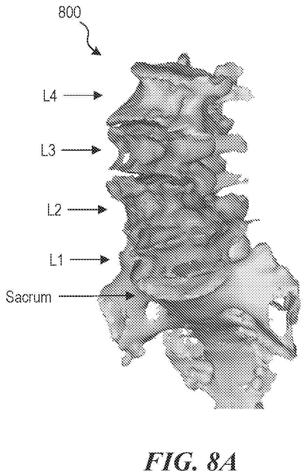

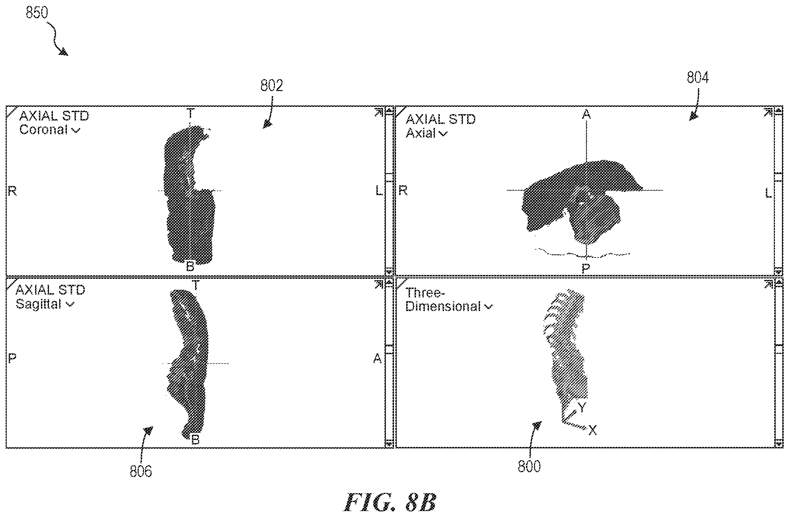

Once the patient data set is received in step 502, the method 500 can continue in step 503 by creating a virtual model of the patient's native anatomical configuration (also referred to as "pre-operative anatomical configuration"). The virtual model can be based on the image data included in the patient data set received in step 502. For example, the same computing system that received the patient data set in step 502 can analyze the image data in the patient data set to generate a virtual model of the patient's native anatomical configuration. The virtual model can be a two- or three-dimensional visual representation of the patient's native anatomy. The virtual model can include one or more regions of interest, and may include some or all of the patient's anatomy within the regions of interest (e.g., any combination of tissue types including, but not limited to, bony structures, cartilage, soft tissue, vascular tissue, nervous tissue, etc.). As a non-limiting example, the virtual model can include a visual representation of the patient's spinal cord region, including some or all of the sacrum, lumbar region, thoracic region, and/or cervical region. In some embodiments, the virtual model includes soft tissue, cartilage, and other non-bony structures. In other embodiments, the virtual model only includes the patient's bony structures. An example of a virtual model of the native anatomical configuration is described below with respect to Figures 8A and 8B. In some embodiments, the method 500 can optionally omit creating a virtual model of the patient's native anatomy in step 503, and proceed directly from step 502 to step 504.

In some embodiments, the computing system that generated the virtual model in step 502 can also determine (e.g., automatically determine or measure) one or more disease metrics of the patient based on the virtual model. For example, the computing system may analyze the virtual model to determine the patient's pre-operative LL, Cobb angles, coronal parameters (e.g., coronal balance, global coronal balance, coronal pelvic tilt, etc.), sagittal parameters (e.g., PI, sacral slope, thoracic kyphosis, etc.) and/or pelvic parameters. The disease metrics can include micro-measurements (e.g., metrics associated with specific or individual segments of the patient's spine) and/or macro-measurements (e.g., metrics associated with multiple segments of the patient's spine).

The method 500 can continue in step 504 by creating a virtual model of a corrected anatomical configuration (which can also be referred to herein as the "planned configuration," "optimized geometry," "post-operative anatomical configuration," or "target outcome") for the patient. For example, the computing system can, using the analysis procedures described previously, determine a "corrected" or "optimized" anatomical configuration for the particular patient that represents an ideal surgical outcome for the particular patient. This can be done, for example, by analyzing multiple reference patient data sets to identify post-operative anatomical configurations for similar patients who had a favorable post-operative outcome, as previously described in detail with respect to Figures 1-4C (e.g., based on similarity of the reference patient data set to the patient data set and/or whether the reference patient had a favorable treatment outcome). This may also include applying one or more mathematical rules defining optimal anatomical outcomes (e.g., positional relationships between anatomic elements) and/or target (e.g., acceptable) post-operative metrics/design criteria (e.g., adjust anatomy so that the post-operative sagittal vertical axis is less than 7mm, the post-operative Cobb angle less than 10 degrees, etc.). Target post-operative metrics can include, but are not limited to, target coronal parameters, target sagittal parameters, target PI angle, target Cobb angle, target shoulder tilt, target iliolumbar angle, target coronal balance, target Cobb angle, target lordosis angle, and/or a target intervertebral space height. The different between the native anatomical configuration and the corrected anatomical configuration may be referred to as a "patient-specific correction" or "target correction."

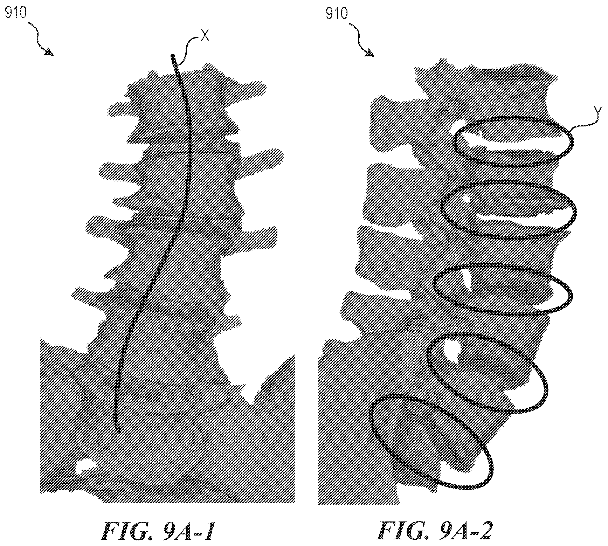

Once the corrected anatomical configuration is determined, the computing system can generate a two- or three-dimensional visual representation of the patient's anatomy with the corrected anatomical configuration. As with the virtual model created in step 503, the virtual model of the patient's corrected anatomical configuration can include one or more regions of interest, and may include some or all of the patient's anatomy within the regions of interest (e.g., any combination of tissue types including, but not limited to, bony structures, cartilage, soft tissue, vascular tissue, nervous tissue, etc.). As a non-limiting example, the virtual model can include a visual representation of the patient's spinal cord region in a corrected anatomical configuration, including some or all of the sacrum, lumbar region, thoracic region, and/or cervical region. In some embodiments, the virtual model includes soft tissue, cartilage, and other non-bony structures. In other embodiments, the virtual model only includes the patient's bony structures. An example of a virtual model of the native anatomical configuration is described below with respect to Figures 9A-1-9B-2.

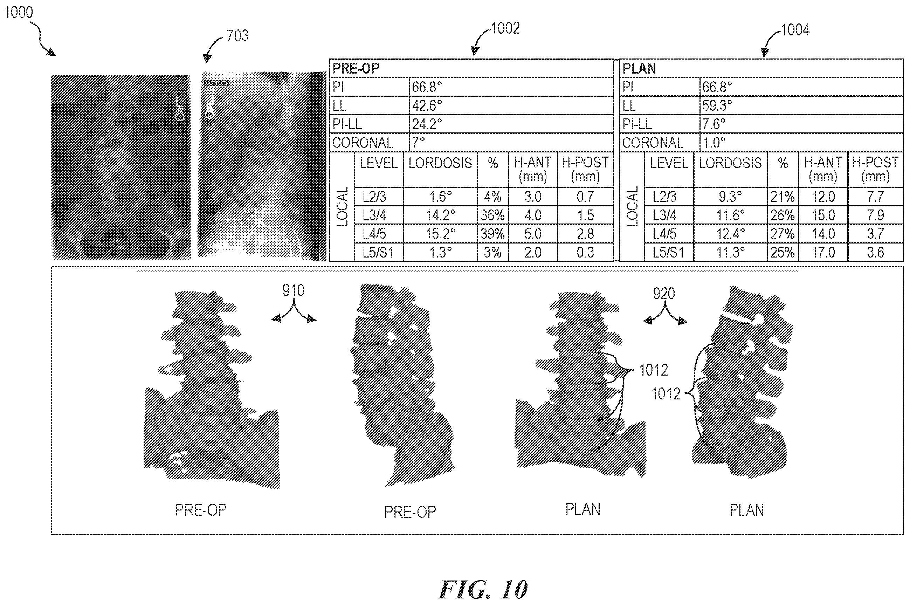

The method 500 can continue in step 506 by generating (e.g., automatically generating) a surgical plan for achieving the corrected anatomical configuration shown by the virtual model. The surgical plan can include pre-operative plans, operative plans, post-operative plans, and/or specific spine metrics associated with the optimal surgical outcome. For example, the surgical plans can include a specific surgical procedure for achieving the corrected anatomical configuration. In the context of spinal surgery, the surgical plan may include a specific fusion surgery (e.g., PLIF, ALIF, TLIF, LLIF, DLIF, XLIF, etc.) across a specific range of vertebral levels (e.g., L1-L4, L1-L5, L3-T12, etc.). Of course, other surgical procedures may be identified for achieving the corrected anatomical configuration, such as non-fusion surgical approaches and orthopedic procedures for other areas of the patient. The surgical plan may also include one or more expected spine metrics (e.g., LL, Cobb angles, coronal parameters, sagittal parameters, and/or pelvic parameters) corresponding to the expected post-operative patient anatomy. The surgical plan can be generated by the same or different computing system that created the virtual model of the corrected anatomical configuration. In some embodiments, the surgical plan can also be based on one or more reference patient data sets as previously described with respect to Figures 1-4C. In some embodiments, the surgical plan can also be based at least in part on surgeon-specific preferences and/or outcomes associated with a specific surgeon performing the surgery. In some embodiments, more than one surgical plan is generated in step 506 to provide a surgeon with multiple options. An example of a surgical plan is described below with respect to Figure 10.

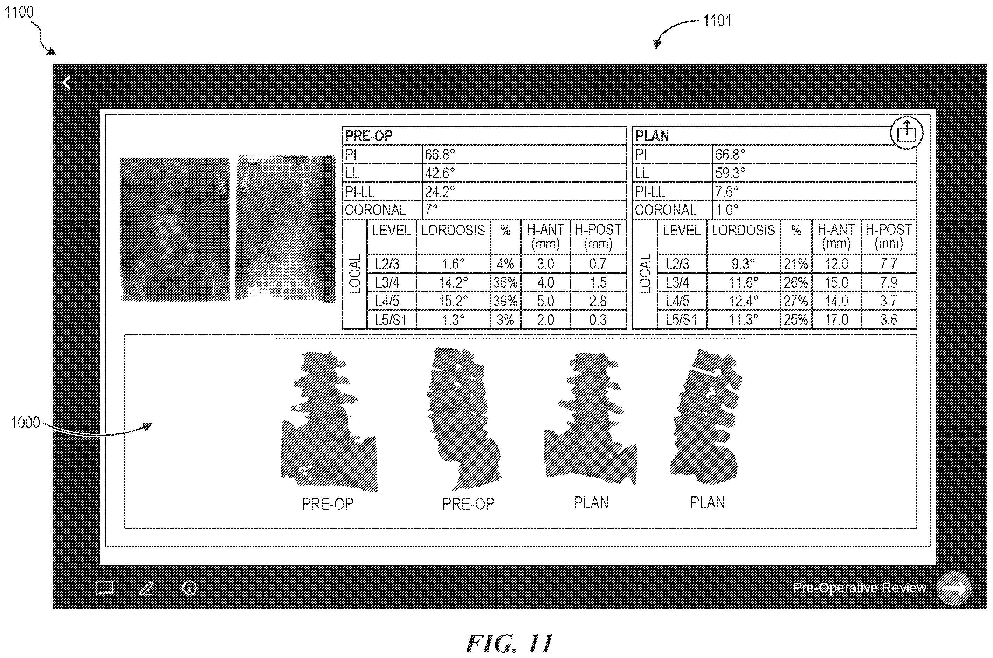

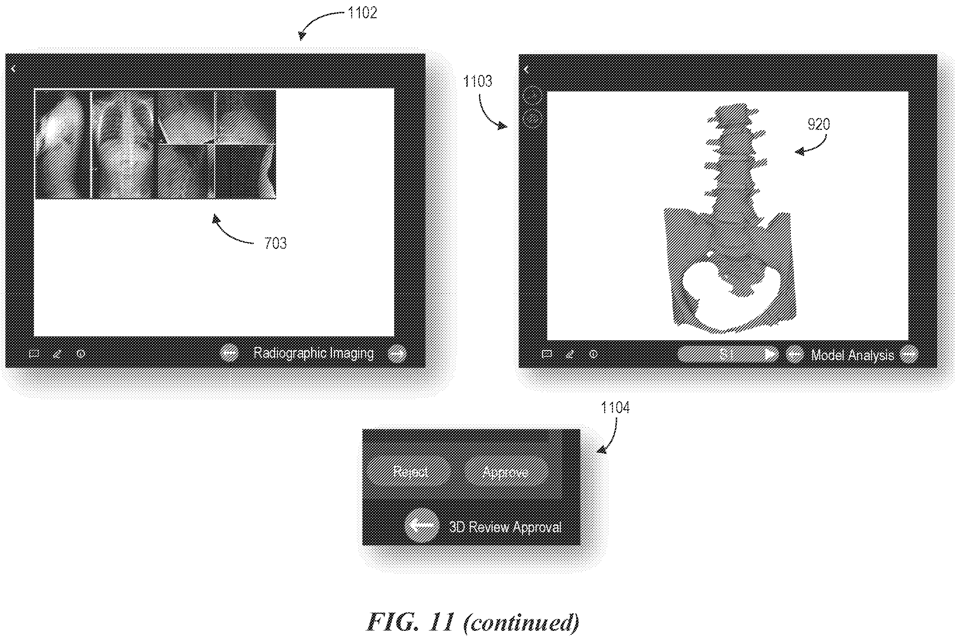

After the virtual model of the corrected anatomical configuration is created in step 504 and the surgical plan is generated in step 506, the method 500 can continue in step 508 by transmitting the virtual model of the corrected anatomical configuration and the surgical plan for surgeon review. In some embodiments, the virtual model and the surgical plan are transmitted as a surgical plan report, an example of which is described with respect to Figure 11. In some embodiments, the same computing system used in steps 502-506 can transmit the virtual model and surgical plan to a computing device for surgeon review (e.g., the client computing device 102 described in Figure 1 or the computing device 602 described below with respect to Figure 6). This can include directly transmitting the virtual model and the surgical plan to the computing device or uploading the virtual model and the surgical plan to a cloud or other storage system for subsequent downloading. Although step 508 describes transmitting the surgical plan and the virtual model to the surgeon, one skilled in the art will appreciate from the disclosure herein that images of the virtual model may be included in the surgical plan transmitted to the surgeon, and that the actual model need not be included (e.g., to decrease the file size being transmitted). Additionally, the information transmitted to the surgeon in step 508 may include the virtual model of the patient's native anatomical configuration (or images thereof) in addition to the virtual model of the corrected anatomical configuration. In embodiments in which more than one surgical plan is generated in step 506, the method 500 can include transmitting more than one surgical plan to the surgeon for review and selection.

The surgeon can review the virtual model and surgical plan and, in step 510, either approve or reject the surgical plan (or, if more than one surgical plan is provided in step 508, select one of the provided surgical plans). If the surgeon does not approve the surgical plan in step 510, the surgeon can optionally provide feedback and/or suggested modifications to the surgical plan (e.g., by adjusting the virtual model or changing one or more aspects about the plan). Accordingly, the method 500 can include receiving (e.g., via the computing system) the surgeon feedback and/or suggested modifications. If surgeon feedback and/or suggested modifications are received in step 512, the method 500 can continue in step 514 by revising (e.g., automatically revising via the computing system) the virtual model and/or surgical plan based at least in part on the surgeon feedback and/or suggested modifications received in step 512. In some embodiments, the surgeon does not provide feedback and/or suggested modifications if they reject the surgical plan. In such embodiments, step 512 can be omitted, and the method 500 can continue in step 514 by revising (e.g., automatically revising via the computing system) the virtual model and/or the surgical plan by selecting new and/or additional reference patient data sets. The revised virtual model and/or surgical plan can then be transmitted to the surgeon for review. Steps 508, 510, 512, and 514 can be repeated as many times as necessary until the surgeon approves the surgical plan. Although described as the surgeon reviewing, modifying, approving, and/or rejecting the surgical plan, in some embodiments the surgeon can also review, modify, approve, and/or reject the corrected anatomical configuration shown via the virtual model.

Once surgeon approval of the surgical plan is received in step 510, the method 500 can continue to step 513 by determining whether a suitable adjustable-implant corrective plan ("corrective plan") for providing one or more post-operative implant adjustments can be generated based on the approved surgical plan. For example, the computing system can perform one or more simulations in which one or more implants perform post-operative adjustments designed to achieve simulated outcomes (e.g., outcomes of the surgical plan, corrective plan, etc.). If the simulated outcomes meet suitability criteria (e.g., user inputted criteria, threshold metrics from the surgical plan, etc.), the computing system can determine that the corrective plan is suitable for the surgical plan. In some embodiments, the computing system can request modification to the surgical plan in order to generate corrective plans for the modified surgical plan. This provides flexibility to coordinate surgical plans and adjustable implant corrective plans.

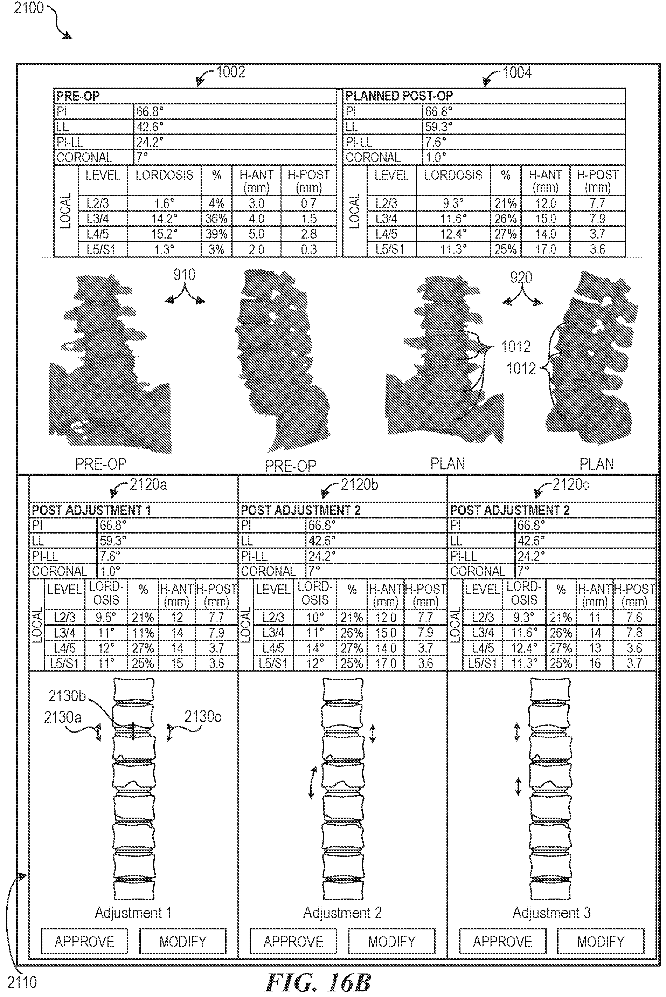

If the computing system determines that a suitable corrective plan can be generated, the method 500 can continue to step 515. The computing system can analyze, for example, one or more surgical plans, designs of adjustable implants, selectable sensor readings, and/or design parameters to generate a corrective plan that meets one or more outcome criteria. In some embodiments, reference data sets can be used to determine a patient-specific post-operative corrective plan. For example, step 513 can include one or more acts of step 316 of Figure 3 and select a subset of reference data sets with adjustable-implant data based on similarity criteria. This allows the computing system to use reference data that meets criteria for designing implants (e.g., non-adjustable implants, non-adjustable implants, etc.). In some embodiments, the physician can input design parameters, including, without limitation, ranges or values for disk heights, anterior disk heights, posterior disk heights, lordosis angles, and other parameters disclosed herein. Example design parameters for adjustable implants and physician approval are discussed in connection with Figure 16B and can be used to generate patient-specific post-operative corrective plans in the form of corrective plans (e.g., implant specific corrective plans, multi-implant corrective plans, etc.) used to perform single level or multi-level post-operative spinal adjustments.

In some embodiments, the computing system can generate a corrective plan based on physician inputted design parameters by, for example, selecting levels for treatment, number of implants (e.g., per level, per spinal segment, etc.), and designs for the respective implants. The corrective plan can include post-operative adjustments that can be, for example, incorporated into post-operative therapy or other plans disclosed herein. In some embodiments, the computing system can determine, for example, types of adjustable implants (e.g., expandable disks, rods, expandable cages, etc.), range of motion for individual implants, sensing capabilities of implants, or the like based on computer generated simulations using, for example, three-dimensional models of the patient's anatomy. The physician can approve or revise the corrective plan.

The method 500 can proceed to step 517 to design (e.g., via the same computing system that performed one or more of steps 502-514) patient-specific implant(s) based on the surgical plan and the corrective plan. The patient-specific implant(s) can have post-implantation configurations for achieving targeted corrections, such as corrections in the surgical plan. The adjustability of the patient-specific implants can then be designed based on the corrective plan, which can include, without limitation, one or more implant configurations (e.g., undeployed or collapsed configurations, partially deployed or expanded configurations, fully deployed or fully expanded configurations), ranges of motion, relationships between implants (e.g., configuration relationships, loading relationships, etc.), sensing capabilities, or the like. In some embodiments, the computer system can, for example, simulate disease progressions, natural age-related anatomical changes, target spinal configurations, or other anatomical changes to design implants capable of achieving outcomes via post-operative in-vivo adjustments. In some embodiments, the computer system can simulate multiple scenarios of anatomical changes. The scenarios can be ranked to prioritize and/or select design parameters. The system can request physician input to, for example, select the rankings. In some embodiments, the computer system can score and rank the scenarios. The highest-ranking scenario can be selected to design implants that meet a confidence score threshold.

The method 500 can continue in step 518 by manufacturing the patient-specific implant. The implant can be manufactured using additive manufacturing techniques, such as 3D printing, stereolithography, DLP, FDM, SLS, SLM, EBM, LOM, PP, thermoplastic printing, DMD, or inkjet photo resin printing, or like technologies, or combination thereof. Alternatively or additionally, the implant can be manufactured using subtractive manufacturing techniques, such as CNC machining, EDM, grinding, laser cutting, water jet machining, manual machining (e.g., milling, lathe/turning), or like technologies, or combinations thereof. The implant may be manufactured by any suitable manufacturing system (e.g., the manufacturing system 124 shown in Figure 1 or the manufacturing system 630 described below with respect to Figure 6). In some embodiments, the implant is manufactured by the manufacturing system executing the computer-readable fabrication instructions generated by the computing system in step 516.

Once the implant is manufactured in step 518, the method 500 can continue in step 520 by implanting the patient-specific implant into the patient. The surgical procedure can be performed manually, by a robotic surgical platform (e.g., a surgical robot), or a combination thereof. In embodiments in which the surgical procedure is performed at least in part by a robotic surgical platform, the surgical plan can include computer-readable control instructions configured to cause the surgical robot to perform, at least partly, the patient-specific surgical procedure. Additional details regarding a robotic surgical platform are described below with respect to Figure 6.

At step 512, if the suitable corrective plan is not identified for the approved surgical plan, the method 500 can proceed to step 516 to design (e.g., via the same computing system that performed steps 502-514) patient-specific implant(s) based on the corrected anatomical configuration and the surgical plan. For example, the patient-specific implant can be specifically designed such that, when it is implanted in the particular patient, it directs the patient's anatomy to occupy the corrected anatomical configuration (e.g., transforming the patient's anatomy from the native anatomical configuration to the corrected anatomical configuration). The patient-specific implant can be designed such that, when implanted, it causes the patient's anatomy to occupy the corrected anatomical configuration for the expected service life of the implant (e.g., 5 years or more, 10 years or more, 20 years or more, 50 years or more, etc.). In some embodiments, the patient-specific implant is designed solely based on the virtual model of the corrected anatomical configuration and/or without reference to pre-operative patient images.

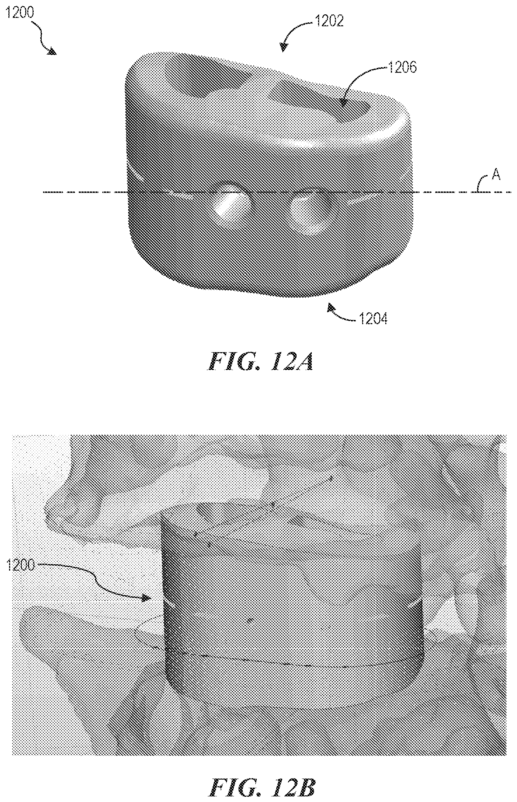

The patient-specific implant can be any of the implants described herein. For example, the patient-specific implant can include one or more of screws (e.g., bone screws, spinal screws, pedicle screws, facet screws), interbody implant devices (e.g., intervertebral implants), cages, plates, rods, discs, fusion devices, spacers, rods, expandable devices, stents, brackets, ties, scaffolds, fixation device, anchors, nuts, bolts, rivets, connectors, tethers, fasteners, joint replacements (e.g., artificial discs), hip implants, or the like. A patient-specific implant design can include data representing one or more of physical properties (e.g., size, shape, volume, material, mass, weight), mechanical properties (e.g., stiffness, strength, modulus, hardness), and/or biological properties (e.g., osteo-integration, cellular adhesion, anti-bacterial properties, anti-viral properties) of the implant. For example, a design for an orthopedic implant can include implant shape, size, material, and/or effective stiffness (e.g., lattice density, number of struts, location of struts, etc.). An example of a patient-specific implant designed via the method 500 is described below with respect to Figures 12A and 12B.

In some embodiments, designing the implant in step 516 can optionally include generating fabrication instructions for manufacturing the implant. For example, the computing system may generate computer-executable fabrication instructions that that, when executed by a manufacturing system, cause the manufacturing system to manufacture the implant.

In some embodiments, the patient-specific implant is designed at step 516 only after the surgeon has reviewed and approved the virtual model with the corrected anatomical configuration and the surgical plan. Accordingly, in some embodiments, the implant design is neither transmitted to the surgeon with the surgical plan in step 508, nor manufactured before receiving surgeon approval of the surgical plan. Without being bound by theory, waiting to design the patient-specific implant until after the surgeon approves the surgical plan may increase the efficiency of the method 500 and/or reduce the resources necessary to perform the method 500.

The method 500 can then proceed to steps 518 and 520 as discussed above. The method 500 can be implemented and performed in various ways. In some embodiments, steps 502-516 can be performed by a computing system (e.g., the computing system 606 described below with respect to Figure 6) associated with a first entity, step 518 can be performed by a manufacturing system associated with a second entity, and step 520 can be performed by a surgical provider, surgeon, and/or robotic surgical platform associated with a third entity. Any of the foregoing steps may also be implemented as computer-readable instructions stored in memory and executable by one or more processors of the associated computing system(s).

Figure 6 is a schematic illustration of an operative setup including select systems and devices that can be used to provide patient-specific medical care, such as for performing the method 500 described with respect to Figure 5. As shown, the operative setup includes a computing device 602, a computing system 606, a cloud 608, a manufacturing system 630, and a robotic surgical platform 650. The computing device 602 can be a user device, such as a smart phone, mobile device, laptop, desktop, personal computer, tablet, phablet, or other such devices known in the art. In operation, a user (e.g., a surgeon) can collect, retrieve, review, modify, or otherwise interact with a patient data set using the computing device 602. The computing system 606 can include any suitable computing system configured to store one or more software modules for identifying reference patient data sets, determining patient-specific surgical plans, generating virtual models of patient anatomy, designing patient-specific implants, or the like. The one or more software modules can include algorithms, machine-learning models, Al architectures, or the like for performing select operations. The cloud 608 can be any suitable network and/or storage system, and may include any combination of hardware and/or virtual computing resources. The manufacturing system 630 can be any suitable manufacturing system for producing patient-specific implants, including any of those previously described herein. The robotic surgical platform 650 (referred to herein as "the platform 650") can be configured to perform or otherwise assist with one or more aspects of a surgical procedure.

In a representative operation, the computing device 602, the computing system 606, the cloud 608, the manufacturing system 630, and the platform 650 can be used to provide patient-specific medical care, such as to perform the method 500 described with respect to Figure 5. For example, the computing system 606 can receive a patient data set from the computing device 602 (e.g., step 502 of the method 500). In some embodiments, the computing device 602 can directly transmit the patient data set to the computing system 606. In other embodiments, the computing device 602 can upload the patient data set into the cloud 608, and the computing system 606 can download or otherwise access the patient data set from the cloud. Once the computing system 606 receives the patient data set, the computing system 606 can create a virtual model of the patient's native anatomical configuration (e.g., step 503 of the method 500), create a virtual model of the corrected anatomical configuration (e.g., step 504 of the method 500), and/or generate a surgical plan for achieving the corrected anatomical configuration (e.g., step 506 of the method 500). The computing system can perform the foregoing operations via the one or more software modules, which in some embodiments include machine learning models or other Al architectures. Once the virtual models and the surgical plan are created, the computing system 606 can transmit the virtual models and the surgical plan to the surgeon for review (e.g., step 508 of the method 500). This can include, for example, directly transmitting the virtual models and the surgical plan to the computing device 602 for surgeon review. In other embodiments, this can include uploading the virtual models and the surgical plan to the cloud 608. A surgeon can then download or otherwise access the virtual models and the surgical plan from the cloud 608 using the computing device 602.

The surgeon can use the computing device 602 to review the virtual models and the surgical plan. The surgeon can also approve or reject the surgical plan and provide any feedback regarding the surgical plan using the computing device 602. The surgeon's approval, rejection, and/or feedback regarding the surgical plan can be transmitted to, and received by, the computing system 606 (e.g., steps 510 and 512 of the method 500). The computing system 606 can than revise the virtual model and/or the surgical plan (e.g., step 514 of the method 500). The computing system 606 can transmit the revised virtual model and surgical plan to the surgeon for review (e.g., by uploading it to the cloud 608 or directly transmitting it to the computing device 602).

The computing system 606 can also design the patient-specific implant based on the corrected anatomical configuration and the surgical plan (e.g., step 516 of the method 500) using, the one or more software modules. In some embodiments, software modules rely on one or more algorithms, machine learning models, or other Al architectures to design the implant. Once the computing system 606 designs the patient-specific implant, the computing system 606 can upload the design and/or manufacturing instructions to the cloud 608. The computing system 606 may also create fabrication instructions (e.g., computer-readable fabrication instructions) for manufacturing the patient-specific implant. In such embodiments, the computing system 606 can upload the fabrication instructions to the cloud 608.

The manufacturing system 630 can download or otherwise access the design and/or fabrication instructions for the patient-specific implant from the cloud 608. The manufacturing system can then manufacture the patient-specific implant (e.g., step 518 in the method 500) using additive manufacturing techniques, subtractive manufacturing techniques, or other suitable manufacturing techniques.

The robotic surgical platform 650 can perform or otherwise assist with one or more aspects of the surgical procedure (e.g., step 520 of the method 500). For example, the platform 650 can prepare tissue for an incision, make an incision, make a resection, remove tissue, manipulate tissue, perform a corrective maneuver, deliver the implant to a target site, deploy the implant at the target site, adjust the implant at the target site, manipulate the implant once it is implanted, secure the implant at the target site, explant the implant, suture tissue, etc. The platform 650 can therefore include one or more arms 655 and end effectors for holding various surgical tools (e.g., graspers, clips, needles, needle drivers, irrigation tools, suction tools, staplers, screw driver assemblies, etc.), imaging instruments (e.g., cameras, sensors, etc.), and/or medical devices (e.g., the implant 600) and that enable the platform 650 to perform the one or more aspects of the surgical plan. Although shown as having one arm 655, one skilled in the art will appreciate that the platform 650 can have multiple arms (e.g., two, three, four, or more) and any number of joints, linkages, motors, and degrees of freedom. In some embodiments, the platform 650 may have a first arm dedicated to holding one or more imaging instruments, while the remainder of the arms hold various surgical tools. In some embodiments, the tools can be releasably secured to the arms such that they can be selectively interchanged before, during, or after an operative procedure. The arms can be moveable through a variety of ranges of motion (e.g., degrees of freedom) to provide adequate dexterity for performing various aspects of the operative procedure.

The platform 650 can include a control module 660 for controlling operation of the arm(s) 655. In some embodiments, the control module 660 includes a user input device (not shown) for controlling operation of the arm(s) 655. The user input device can be a joystick, a mouse, a keyboard, a touchscreen, an infrared sensor, a touchpad, a wearable input device, a camera- or image-based input device, a microphone, or other user input devices. A user (e.g., a surgeon) can interact with the user input device to control movement of the arm(s) 655.

In some embodiments, the control module 660 includes one or more processors for executing machine-readable operative instructions that, when executed, automatically control operation of the arm 655 to perform one or more aspects of the surgical procedure. In some embodiments, the control module 660 may receive the machine-readable operative instructions (e.g., from the cloud 608) specifying one or more steps of the surgical procedure that, when executed by the control module 660, cause the platform 650 to perform the one or more steps of the surgical procedure. For example, the machine-readable operative instructions may direct the platform 650 to prepare tissue for an incision, make an incision, make a resection, remove tissue, manipulate tissue, perform a corrective maneuver, deliver the implant 600 to a target site, deploy the implant 600 at the target site, adjust a configuration of the implant 600 at the target site, manipulate the implant 600 once it is implanted, secure the implant 600 at the target site, explant the implant 600, suture tissue, and the like. The operative instructions may therefore include particular instructions for articulating the arm 655 to perform or otherwise aid in the delivery of the patient-specific implant.

In some embodiments, the platform 650 can generate (e.g., as opposed to simply receiving) the machine-readable operative instructions based on the surgical plan. For example, the surgical plan can include information about the delivery path, tools, and implantation site. The platform 650 can analyze the surgical plan and develop executable operative instructions for performing the patient-specific procedure based on the capabilities (e.g., configuration and number of robotic arms, functionality of end effectors, guidance systems, visualization systems, etc.) of the robotic system. This enables the operative setup shown in Figure 6 to be compatible with a wide range of different types of robotic surgery systems.

The platform 650 can include one or more communication devices (e.g., components having VLC, WiMAX, LTE, WLAN, IR communication, PSTN, Radio waves, Bluetooth, and/or Wi-Fi operability) for establishing a connection with the cloud 608 and/or the computing device 602 for accessing and/or downloading the surgical plan and/or the machine-readable operative instructions. For example, the cloud 608 can receive a request for a particular surgical plan from the platform 650 and send the plan to the platform 650. Once identified, the cloud 608 can transmit the surgical plan directly to the platform 650 for execution. In some embodiments, the cloud 608 can transmit the surgical plan to one or more intermediate networked devices (e.g., the computing device 602) rather than transmitting the surgical plan directly to the platform 650. A user can review the surgical plan using the computing device 602 before transmitting the surgical plan to the platform 650 for execution. Additional details for identifying, storing, downloading, and accessing patient-specific surgical plans are described in U.S. Application No. 16/990,810, filed August 11, 2020.

The platform 650 can include additional components not expressly shown in Figure 6. For example, in various embodiments the platform 650 may include one or more displays (e.g., LCD display screen, an LED display screen, a projected, holographic, or augmented reality display (e.g., a heads-up display device or a head-mounted device)), one or more I/O devices (e.g., a network card, video card, audio card, USB, firewire or other external device, camera, printer, speakers, CD-ROM drive, DVD drive, disk drive, or Blu-Ray device), and/or a memory (e.g., RAM, various caches, CPU registers, ROM, and writable non-volatile memory, such as flash memory, hard drives, floppy disks, CDs, DVDs, magnetic storage devices, tape drives, device buffers, and so forth). In some embodiments, the foregoing components can be generally similar to the like components described in detail with respect to computing device 200 in Figure 2.

Without being bound by theory, using a robotic surgical platform to perform various aspects of the surgical plans described herein is expected to provide several advantages over conventional operative techniques. For example, use of robotic surgical platforms may improve surgical outcomes and/or shorten recovery times by, for example, decreasing incision size, decreasing blood loss, decreasing a length of time of the operative procedure, increasing the accuracy and precision of the surgery (e.g., the placement of the implant at the target location), and the like. The platform 650 can also avoid or reduce user input errors, e.g., by including one or more scanners for obtaining information from instruments (e.g., instruments with retrieval features), tools, the patient specific implant 600 (e.g., after the implant 600 has been gripped by the arm 655), etc. The platform 650 can confirm use of proper instruments prior to and during the surgical procedure. If the platform 650 identifies an incorrect instrument or tool, an alert can be sent to a user that another instrument or tool should be installed. The user can scan the new instrument to confirm that the instrument is appropriate for the surgical plan. In some embodiments, the surgical plan includes instructions for use, a list of instruments, instrument specifications, replacement instruments, and the like. The platform 650 can perform pre- and post-surgical checking routines based on information from the scanners.

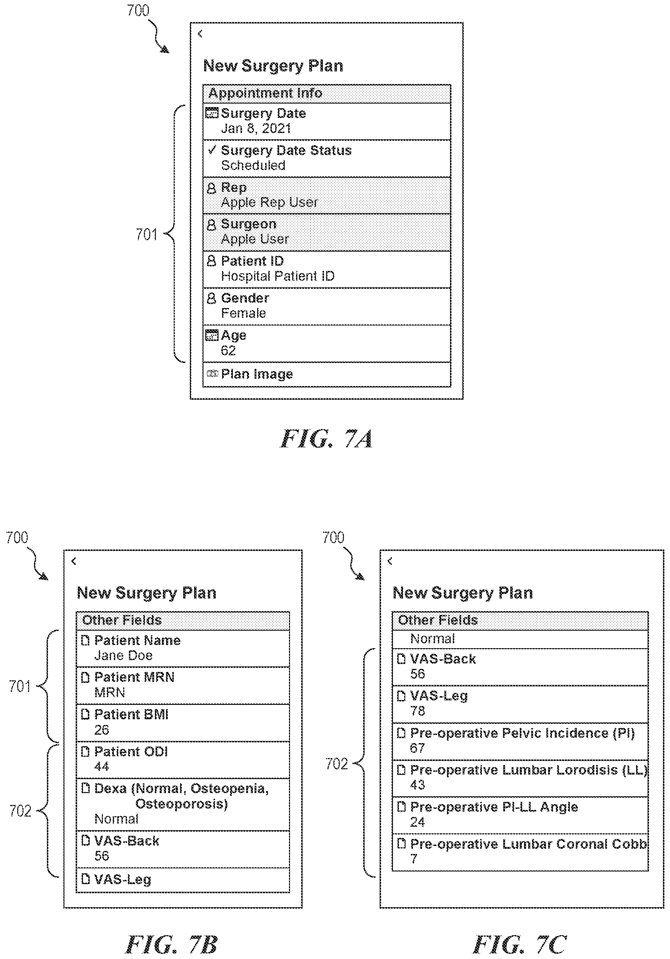

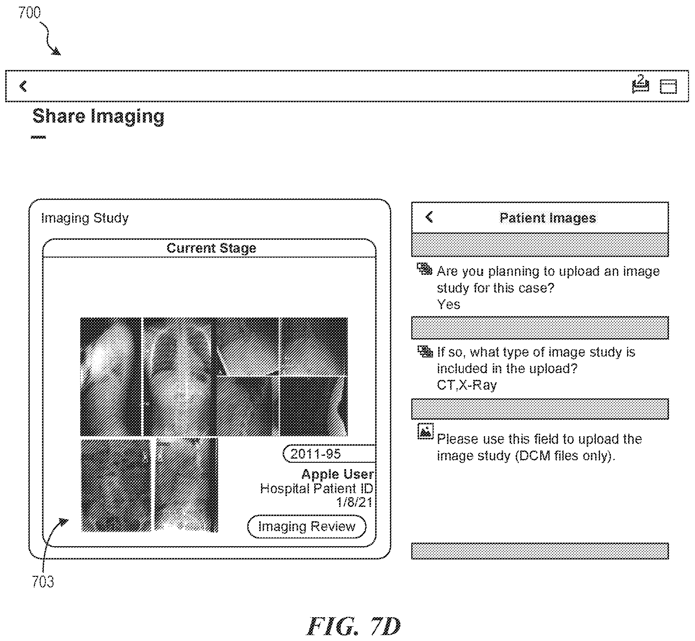

Figures 7A-13 further illustrate select aspects of providing patient-specific medical care, e.g., in accordance with the method 500. For example, Figures 7A-7D illustrate an example of a patient data set 700 (e.g., as received in step 502 of the method 500). The patient data set 700 can include any of the information previously described with respect to the patient data set. For example, the patient data set 700 includes patient information 701 (e.g., patient identification no., patient MRN, patient name, sex, age, BMI, surgery date, surgeon, etc., shown in Figures 7A and 7B), diagnostic information 702 (e.g., Oswestry Disability Index (ODI), VAS-back score, VAS-leg score, Pre-operative pelvic incidence, pre-operative lumbar lordosis, pre-operative PI-LL angle, pre-operative lumbar coronal cobb, etc., shown in Figures 7B and 7C), and image data 703 (x-ray, CT, MRI, etc., shown in Figure 7D). In the illustrated embodiment, the patient data set 700 is collected by a healthcare provider (e.g., a surgeon, a nurse, etc.) using a digital and/or fillable report that can be accessed using a computing device (e.g., the computing device 602 shown in Figure 6). In some embodiments, the patient data set 700 can be automatically or at least partially automatically generated based on digital medical records of the patient. Regardless, once collected, the patient data set 700 can be transmitted to the computing system configured to generate the surgical plan for the patient (e.g., the computing system 606 shown in Figure 6).

Figures 8A and 8B illustrate an example of a virtual model 800 of a patient's native anatomical configuration (e.g., as created in step 503 of the method 500). In particular, Figure 8A is an enlarged view of the virtual model 800 of the patient's native anatomy and shows the patient's native anatomy of their lower spinal cord region. The virtual model 800 is a three-dimensional visual representation of the patient's native anatomy. In the illustrated embodiment, the virtual model includes a portion of the spinal column extending from the sacrum to the L4 vertebral level. Of course, the virtual model can include other regions of the patient's spinal column, including cervical vertebrae, thoracic vertebrae, lumbar vertebrae, and the sacrum. The illustrated virtual model 800 only includes bony structures of the patient's anatomy, but in other embodiments may include additional structures, such as cartilage, soft tissue, vascular tissue, nervous tissue, etc.

Figure 8B illustrates a virtual model display 850 (sometimes referred to herein as the "display 850") showing different views of the virtual model 800. The virtual model display 850 includes a three-dimensional view of the virtual model 800, one or more coronal cross-section(s) 802 of the virtual model 800, one or more axial cross section(s) 804 of the virtual model 800, and/or one or more sagittal cross-section(s) 806 of the virtual model 800. Of course, other views are possible and can be included on the virtual model display 850. In some embodiments, the virtual model 800 may be interactive such that a user can manipulate the orientation or view of the virtual model 800 (e.g., rotate), change the depth of the displayed cross-sections, select and isolate specific bony structures, or the like.

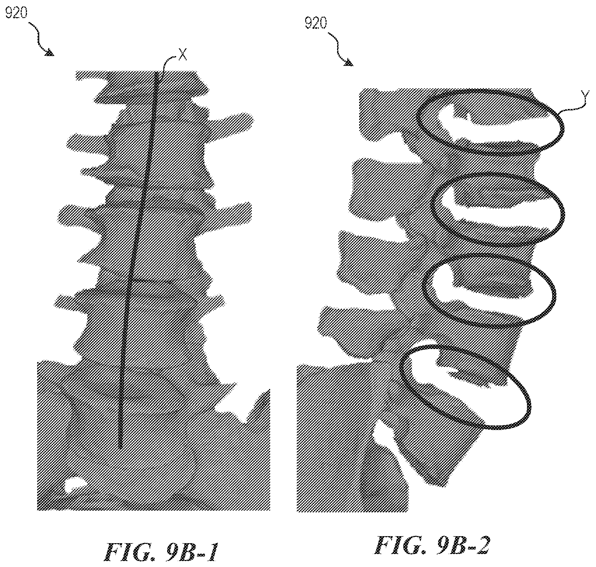

Figures 9A-1-9B-2 demonstrate an example of a virtual model of a patient's native anatomical configuration (e.g., as created in step 503 of the method 500) and a virtual model of the patient's corrected anatomical configuration (e.g., as created in step 504 of the method 500). In particular, Figures 9A-1 and 9A-2 are anterior and lateral views, respectively, of a virtual model 910 showing a native anatomical configuration of a patient, and Figures 9B-1 and 9B-2 are anterior and lateral views, respectively, of a virtual model 920 showing the corrected anatomical configuration for the same patient. Referring first to Figure 9A-1, the anterior view of the virtual model 910 illustrates the patient has abnormal curvature (e.g., scoliosis) of their spinal column. This is marked by line X, which follows a rostral-caudal axis of the spinal column. Referring next to Figure 9A-1, the lateral view of the virtual model 910 illustrates the patient has collapsed discs or decreased spacing between adjacent vertebral endplates, marked by ovals Y. Figures 9B-1 and 9B-2 illustrate the corrected virtual model 920 accounting for the abnormal anatomical configurations shown in Figures 9A-1 and 9A-2. For example, Figure 9B-1, which is an anterior view of the virtual model 920, illustrates the patient's spinal column having corrected alignment (e.g., the abnormal curvature has been reduced). This correction is shown by line X, which also follows a rostral-caudal axis of the spinal column. Figure 9B-2, which is a lateral view of the virtual model 920, illustrates the patient's spinal column having restored disc height (e.g., increased spacing between adjacent vertebral endplates), also marked by ovals Y. The lines X and the ovals Y are provided in Figures 9A-1-9B-2 to more clearly demonstrate the correction between the virtual models 910 and 920, and are not necessarily included on the virtual models generated in accordance with the present technology.

Figure 10 illustrates an example of a surgical plan 1000 (e.g., as generated in step 506 of the method 500). The surgical plan 1000 can include pre-operative patient metrics 1002, predicted post-operative patient metrics 1004, one or more patient images (e.g., the patient images 703 received as part of the patient data set), the virtual model 910 (which can be the model itself or one or more images derived from the model) of the patient's native anatomical configuration (e.g., pre-operative patient anatomy), and/or the virtual model 920 (which can be the model itself or one or more images derived from the model) of the patient's corrected anatomical configuration (e.g., predicted post-operative patient anatomy). The virtual model 920 of the predicted post-operative patient anatomy can optionally include one or more implants 1012 shown as implanted in the patient's spinal cord region to demonstrate how patient anatomy will look following the surgery. Although four implants 1012 are shown in the virtual model 920, the surgical plan 1000 may include more or fewer implants 1012, including one, two, three, five, six, seven, eight, or more implants 1012.

The surgical plan 1000 can include additional information beyond what is illustrated in Figure 10. For example, the surgical plan 1000 may include pre-operative instructions, operative instructions, and/or post-operative instructions. Operative instructions can include one or more specific procedures to be performed (e.g., PLIF, ALIF, TLIF, LLIF, DLIF, XLIF, etc.) and/or one or more specific targets of the operation (e.g., fusion of vertebral levels L1-L4, anchoring screw to be inserted in lateral surface of L4, etc.). Although the surgical plan 1000 is demonstrated in Figure 10 as a visual report, the surgical plan 1000 can also be encoded in computer-executable instructions that, when executed by a processor connected to a computing device, cause the surgical plan 1000 to be displayed by the computing device. In some embodiments, the surgical plan 1000 may also include machine-readable operative instructions for carrying out the surgical plan. For example, the surgical plan can include operative instructions for a robotic surgical platform to carry out one or more steps of the surgical plan 1000.

Figure 11 provides a series of images illustrating an example of a patient surgical plan report 1100 that includes the surgical plan 1000 and that may be transmitted to a surgeon for review and approval (e.g., as transmitted in step 508 of the method 500). The surgical plan report 1100 can include a multi-page report detailing aspects of the surgical plan 1000. For example, the multi-page report may include a first page 1101 demonstrating an overview of the surgical plan 1000 (e.g., as shown in Figure 10), a second page 1102 illustrating patient images (e.g., such as the patient images 703 received in step 502 and shown in Figure 7D), a third page 1103 illustrating an enlarged view of the virtual model of the corrected anatomical configuration (e.g., the virtual model 920 shown in Figure 9), and a fourth page 1104 prompting the surgeon to either approve or reject the surgical plan 900. Of course, additional information about the surgical plan can be presented with the report 1100 in the same or different formats. In some embodiments, if the surgeon rejects the surgical plan 1000, the surgeon can be prompted to provide feedback regarding the aspects of the surgical plan 1000 the surgeon would like adjusted.

The patient surgical plan report 1100 can be presented to the surgeon on a digital display of a computing device (e.g., the client computing device 102 shown in Figure 1 or the computing device 602 shown in Figure 6). In some embodiments, the report 1100 is interactive and the surgeon can manipulate various aspects of the report 1100 (e.g., adjust views of the virtual model, zoom-in, zoom-out, annotate, etc.). However, even if the report 1100 is interactive, the surgeon generally cannot directly change the surgical plan 1000. Rather, the surgeon may provide feedback and suggested changes to the surgical plan 1000, which can be sent back to the computing system that generated the surgical plan 1000 for analysis and refinement.

Figure 12A illustrates an example of a patient-specific implant 1200 (e.g., as designed in step 516 and manufactured in step 518 of the method 500), and Figure 12B illustrates the implant 1200 implanted in the patient. The implant 1200 can be any orthopedic or other implant specifically designed to induce the patient's body to conform to the previously identified corrected anatomical configuration. In the illustrated embodiment, the implant 1200 is an vertebral interbody device having a first (e.g., upper) surface 1202 configured to engage an inferior endplate surface of a superior vertebral body and a second (e.g., lower) surface 1204 configured to engage a superior endplate surface of an inferior vertebral body. The first surface 1202 can have a patient-specific topography designed to match (e.g., mate with) the topography of the inferior endplate surface of the superior vertebral body to form a generally gapless interface therebetween. Likewise, the second surface 1204 can have a patient-specific topography designed to match or mate with the topography of the superior endplate surface of the inferior vertebral body to form a generally gapless interface therebetween. The implant 1200 may also include a recess 1206 or other feature configured to promote bony ingrowth. Because the implant 1200 is patient-specific and designed to induce a geometric change in the patient, the implant 1200 is not necessarily symmetric, and is often asymmetric. For example, in the illustrated embodiment, the implant 1200 has a non-uniform thickness such that a plane defined by the first surface 1202 is not parallel to a central longitudinal axis A of the implant 1200. Of course, because the implants described herein, including the implant 1200, are patient-specific, the present technology is not limited to any particular implant design or characteristic. Additional features of patient-specific implants that can be designed and manufactured in accordance with the present technology are described in U.S. Patent Application Nos. 16/987,113 and 17/100,396.

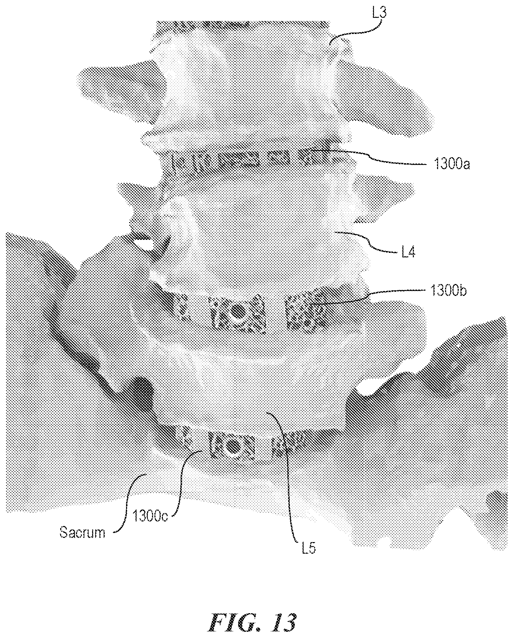

The patient-specific medical procedures described herein can involve implanting more than one patient-specific implant into the patient to achieve the corrected anatomical configuration (e.g., a multi-site procedure). Figure 13, for example, illustrates a lower spinal cord region having three patient specific implants 1300a-1300c implanted at different vertebral levels. More specifically, a first implant 1300a is implanted between the L3 and L4 vertebral bodies, a second implant 1300b is implanted between the L4 and L5 vertebral bodies, and a third implant 1300c is implanted between the L5 vertebral body and the sacrum. Together, the implants 1300a-c can cause the patient's spinal cord region to assume the previously identified corrected anatomical configuration (e.g., transforming the patient's anatomy from its pre-operative diseased configuration to the post-operative optimized configuration). In some embodiments, more or fewer implants are used to achieve the corrected anatomical configuration. For example, in some embodiments one, two, four, five, six, seven, eight, or more implants are used to achieve the corrected anatomical configuration. In embodiments involving more than one implant, the implants do not necessarily have the same shape, size, or function. In fact, the multiple implants will often have different geometries and topographies to correspond to the target vertebral level at which they will be implanted. As also shown in Figure 13, the patient-specific medical procedures described herein can involve treating the patient at multiple target regions (e.g., multiple vertebral levels).

In addition to designing patient-specific medical care based off reference patient data sets, the systems and methods of the present technology may also design patient-specific medical care based off disease progression for a particular patient. In some embodiments, the present technology therefore includes software modules (e.g., machine learning models or other algorithms) that can be used to analyze, predict, and/or model disease progression for a particular patient. The machine learning models can be trained based off multiple reference patient data sets that includes, in addition to the patient data described with respect to Figure 1, disease progression metrics for each of the reference patients. The progression metrics can include measurements for disease metrics over a period of time. Suitable metrics may include spinopelvic parameters (e.g., LL, pelvic tilt, sagittal vertical axis (SVA), cobb angle, coronal offset, etc.), disability scores, functional ability scores, flexibility scores, VAS pain scores, or the like. The progression of the metrics for each reference patient can be correlated to other patient information for the specific reference patient (e.g., age, sex, height, weight, activity level, diet, etc.).

In some embodiments, the present technology includes a disease progression module that includes an algorithm, machine learning model, or other software analytical tool for predicting disease progression in a particular patient. The disease progression module can be trained based on reference patient data sets that includes patient information (e.g., age, sex, height, weight, activity level, diet) and disease metrics (e.g., diagnosis, spinopelvic parameters such as LL, pelvic tilt, SVA, cobb angle, coronal offset, etc., disability scores, functional ability scores, flexibility scores, VAS pain scores, etc.). The disease metrics can include values over a period of time. For example, the reference patient data may include values of disease metrics on a daily, weekly, monthly, bi-monthly, yearly, or other basis. By measuring the metrics over a period of time, changes in the values of the metrics can be tracked as an estimate of disease progression and correlated to other patient data.

In some embodiments, the disease progression module can therefore estimate the rate of disease progression for a particular patient. The progression may be estimated by providing estimated changes in one or more disease metrics over a period of time (e.g., X% increase in a disease metric per year). The rate can be constant (e.g., 5% increase in pelvic tilt per year) or variable (e.g., 5% increase in pelvic tilt for a first year, 10% increase in pelvic tilt for a second year, etc.). In some embodiments, the estimated rate of progression can be transmitted to a surgeon or other healthcare provider, who can review and update the estimate, if necessary.

As a non-limiting example, a particular patient who is a fifty-five-year-old male may have an SVA value of 6mm. The disease progression module can analyze patient reference data sets to identify disease progression for individual reference patients having one or more similarities with the particular patient (e.g., individual patients of the reference patients who have an SVA value of about 6 mm and are approximately the same age, weight, height, and/or sex of the patient). Based on this analysis, the disease progression module can predict the rate of disease progression if no surgical intervention occurs (e.g., the patient's VAS pain scores may increase 5%, 10%, or 15% annually if no surgical intervention occurs, the SVA value may continue to increase by 5% annually if no surgical intervention occurs, etc.).

The systems and methods described herein can also generate models/simulations based on the estimated rates of disease progression, thereby modeling different outcomes over a desired period of time. Additionally, the models/simulations can account for any number of additional diseases or conditions to predict the patient's overall health, mobility, or the like. These additional diseases or conditions can, in combination with other patient health factors (e.g., height, weight, age, activity level, etc.) be used to generate a patient health score reflecting the overall health of the patient. The patient health score can be displayed for surgeon review and/or incorporated into the estimation of disease progression. Accordingly, the present technology can generate one or more virtual simulations of the predicted disease progression to demonstrate how the patient's anatomy is predicted to change over time. Physician input can be used to generate or modify the virtual simulation(s). The present technology can generate one or more post-treatment virtual simulations based on the received physician input for review by the healthcare provider, patient, etc.

In some embodiments, the present technology can also predict, model, and/or simulate disease progression based on one or more potential surgical interventions. For example, the disease progression module may simulate what a patient's anatomy may look like 1, 2, 5, or 10 years post-surgery for several surgical intervention options. The simulations may also incorporate non-surgical factors, such as patient age, height, weight, sex, activity level, other health conditions, or the like, as previously described. Based on these simulations, the system and/or a surgeon can select which surgical intervention is best suited for long-term efficacy. These simulations can also be used to determine patient-specific corrections that compensate for the projected diseases progression.

Accordingly, in some embodiments, multiple disease progression models (e.g., two, three, four, five, six, or more) are simulated to provide disease progression data for several different surgical intervention options or other scenarios. For example, the disease progression module can generate models that predict post-surgical disease progression for each of three different surgical interventions. A surgeon or other healthcare provider can review the disease progression models and, based on the review, select which of the three surgical intervention options is likely to provide the patient with the best long-term outcome. Of course, selecting the optimal intervention can also be fully or semi-automated, as described herein.

Based off of the modeled disease progression, the systems and methods described herein can also (i) identify the optimal time for surgical intervention, and/or (ii) identify the optimal type of surgical procedure for the patient. In some embodiments, the present technology therefore includes an intervention timing module that includes an algorithm, machine learning model, or other software analytical tool for determining the optimal time for surgical intervention in a particular patient. This can be done, for example, by analyzing patient reference data that includes (i) pre-operative disease progression metrics for individual reference patients, (ii) disease metrics at the time of surgical intervention for individual reference patients, (iii) post-operative disease progression metrics for individual reference patients, and/or (iv) scored surgical outcomes for individual reference patients. The intervention timing module can compare the disease metrics for a particular patient to the reference patient data sets to determine, for similar patients, the point of disease progression at which surgical intervention produced the most favorable outcomes.

As a non-limiting example, the reference patient data sets may include data associated with reference patients' SVA. The data can include (i) SVA values for individual patients over a period of time before surgical intervention (e.g., how fast and to what degree the SVA value changed), (ii) SVA of the individual patients at the time of surgical intervention, (iii) the change in SVA after surgical intervention, and (iv) the degree to which the surgical intervention was successful (e.g., based on pain, quality of life, or other factors). Based on the foregoing data, the intervention timing module can, based on a particular patient's SVA value, identify at which point surgical intervention will have the highest likelihood of producing the most favorable outcome. Of course, the foregoing metric is provided by way of example only, and the intervention timing module can incorporate other metrics (e.g., LL, pelvic tilt, SVA, cobb angle, coronal offset, disability scores, functional ability scores, flexibility scores, VAS pain scores) instead of or in combination with SVA to predict the time at which surgical intervention has the highest probability of providing a favorable outcome for the particular patient.

The intervention timing module may also incorporate one or more mathematical rules based on value thresholds for various disease metrics. For example, the intervention timing module may indicate surgical intervention is necessary if one or more disease metrics exceed a predetermined threshold or meet some other criteria. Representative thresholds that indicate surgical intervention may be necessary include SVA values greater than 7mm, a mismatch between lumbar lordosis and pelvic incidence greater than 10 degrees, a cobb angle of greater than 10 degrees, and/or a combination of cobb angle and LL/PI mismatch greater than 20 degrees. Of course, other threshold values and metrics can be used; the foregoing are provided as examples only and in no way limit the present disclosure. In some embodiments, the foregoing rules can be tailored to specific patient populations (e.g., for males over 50 years of age, an SVA value greater than 7 mm indicates the need for surgical intervention). If a particular patient does not exceed the thresholds indicating surgical intervention is recommended, the intervention timing module may provide an estimate for when the patient's metrics will exceed one or more thresholds, thereby providing the patient with an estimate of when surgical intervention may become recommended.

The present technology may also include a treatment planning module that can identify the optimal type of surgical procedure for the patient based on the disease progression of the patient. The treatment planning module can be an algorithm, machine learning model, or other software analytical tool trained or otherwise based on multiple reference patient data sets, as previously described. The treatment planning module may also incorporate one or more mathematical rules for identifying surgical procedures. As a non-limiting example, if a LL/PI mismatch is between 10 and 20 degrees, the treatment planning module may recommend an anterior fusion surgery, but if the LL/PI mismatch is greater than 20 degrees, the treatment planning module may recommend both anterior and posterior fusion surgery. As another non-limiting example, if a SVA value is between 7 mm and 15mm, the treatment planning module may recommend posterior fusion surgery, but if the SVA is above 15 mm, the treatment planning module may recommend both posterior fusion surgery and anterior fusion surgery. Of course, other rules can be used; the foregoing are provided as examples only and in no way limit the present disclosure.

Without being bound by theory, incorporating disease progression modeling into the patient-specific medical procedures described herein may even further increase the effectiveness of the procedures. For example, in many cases it may be disadvantageous operate after a patient's disease progresses to an irreversible or unstable state. However, it may also be disadvantageous to operate too early, before the patient's disease is causing symptoms and/or if the patient's disease may not progress further. The disease progression module and/or the intervention timing module can therefore help identify the window of time during which surgical intervention in a particular patient has the highest probability of providing a favorable outcome for the patient.

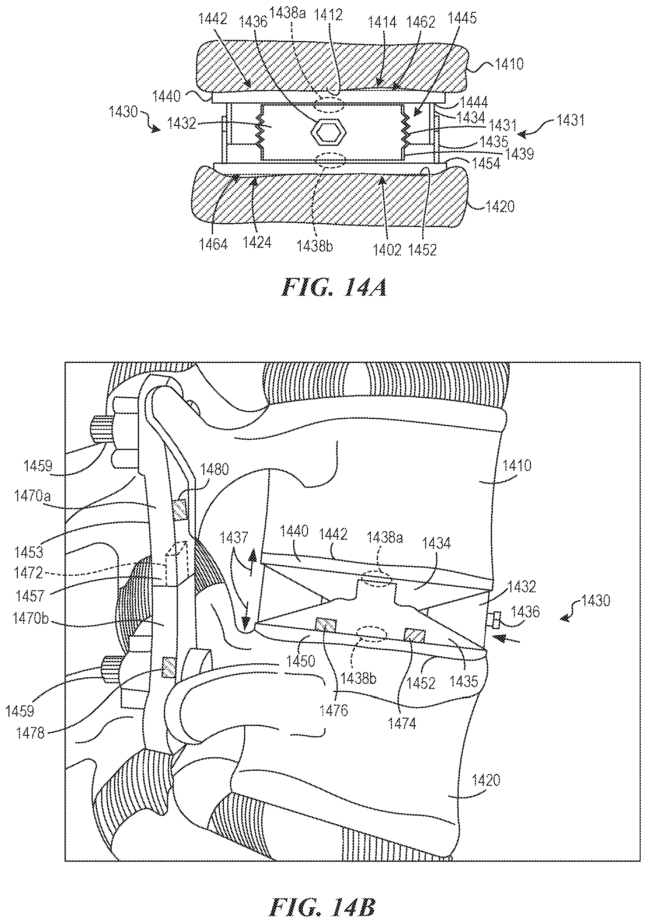

Figures 14A and 14B are schematic anterior and side illustrations, respectively, of a deployed patient-specific IBF device 1430 ("device 1430") deployed between a first vertebra 1410 (e.g., a relatively superior vertebra) and a second vertebra 1420 (e.g., a relatively inferior vertebra) in accordance with some embodiments of the present technology. The device 1430 can be in a collapsed or low-profile configuration for delivery to the disc space between the first and second vertebrae 1410, 1420. For example, the collapsed device 1430 can be inserted manually with surgical navigation or via a surgical robot and then expanded at the implantation site. Once deployed, as described in more detail below, the device 1430 can provide one or more adjustments, corrections (e.g., corrections to the alignment of the first and second vertebrae 1410, 1420, spinal segments, etc.), or the like. Figure 14B shows the device 1430 fully expanded along a vertical axis, indicated by arrows 1437, to space apart the first and second vertebrae 1410, 1420. As discussed in more detail below, the device 1430 can be contoured and/or otherwise customized to match the contours of the first and second vertebrae 1410, 1420. An auxiliary implant 1453 is also illustrated. In some embodiments, the auxiliary implant 1453 is patient-specific and includes a rod 1457 curved to provide spacing between vertebrae. Fasteners 1459 can couple the rod 1457 to the vertebrae. Auxiliary patient-specific implants can include, without limitation, rod and screw systems, interspinous spacers, or other orthopedic implants. In some embodiments, the device 1430 can also be used with non-patient-specific devices and implants. The systems disclosed herein can design implants for implantation at different locations to provide a specific treatment.

In the illustrated embodiment of Figure 14A, the device 1430 includes an expansion body or main body 1431 operable to controllably expand or deploy the device 1430. The main body 1431 has a deployed or expanded configuration selected based on the treatment to be performed. The internal components of the main body 1431 can be designed and manufactured to achieve a desired treatment plan. Central reservoir 1432 can be configured to expand under hydraulic or pneumatic pressure. The patient-specific expansion can be selected based, at least in part, on the design of other components of the device 1430. The device 1430 can have a patient-specific design selected to, for example, enhance fusion (e.g., bone growth to the vertebral bodies), enhance fixation between the vertebral bodies, limit stresses in the vertebral bodies, or the like. For example, the device 1430 can have a volume or receiving window 1445 for receiving material, such as material that promotes bone ingrowth.

The main body 1431 can be configured to expand from a collapsed configuration to an expanded configuration (illustrated in Figures 14A and 14B) and can include one or more expansion mechanisms (e.g., screw jack mechanisms, wedges, scissor mechanisms, etc.), angled or sloped surfaces, inflatable members, or other components for causing deployment. Additionally, the main body 1431 can include linkages, pin connections, linkage assemblies, or other components for connecting various other components. In some embodiments, the device 1430 includes a drive feature 1436 (e.g., a drive head, a screw head, a bolt head, etc.) coupleable to a drive instrument. The drive feature 1436 can be connected to one or more drive elements (e.g., threaded bodies, wedge members, drive shafts, etc.) of the device 1430. In some embodiments, the drive feature 1436 can be rotated in opposite directions to controllably expand or collapse the device 1430. The main body 1431 can include an outer covering or bellows 1439 that surrounds internal movable components. In other embodiments, the internal moving components can be exposed to the surrounding environment and upper and lower components 1434, 1435 can inhibit or limit movement of tissue between components of the main body 1431.

As further illustrated in Figures 14A and 14B, the device 1430 can further include a first endplate 1440 (e.g., a superior endplate) and a lockable joint 1438a connecting the first endplate 1440 to the main body 1431. The device 1430 can further include a second endplate 1450 (e.g., an inferior endplate) connected to the main body 1431 via a lockable joint 1438b. The lockable joints 1438a, 1438b can be selectively transitioned between an unlocked configuration and a locked configuration. In the unlocked configuration, the lockable joints 1438a, 1438b enable the first endplate 1440 and the second endplate 1450 to move relative to the main body 1431. In the locked configuration, the lockable joints 1438a, 1438b prevent or at least reduce the first endplate 1440 and the second endplate 1450 from moving relative to the main body. Accordingly, in the unlocked configuration, the lockable joints 1438a, 1438b can be configured to provide a desired range of motion that permits the endplates 1440, 1450 to conform to patient anatomy. In some embodiments, the lockable joints 1438a, 1438b are ball joints, hinges, tethers, or other connections that allow relative movement between the endplates 1440, 1450. The lockable joints 1438a, 1438b can be connected to opposite ends of the main body 1431 such that the lockable joints 1438a, 1438b are moved along with the respective endplates 1440, 1450, respectively, during expansion of the device 1430. In some embodiments, the maximum range of motion of the lockable joints 1438a, 1438b is selected based on a desired range of motion for the spinal segment. In some embodiments, the position, configuration, and/or motion provided by the lockable joints 1438a, 1438b after locking can be selected based on the desired range of motion.

The first endplate 1440 includes a first surface 1442 (e.g., a superior surface) that mates with an inferior surface 1412 of the first vertebra, and a second surface 1444 (e.g., an inferior surface) that mates with the lockable joint 1438a and/or the upper component 1434. Further, as illustrated in Figures 14A and 14B, the first surface 1442 is customized to the patient-specific topology of the inferior vertebral surface 1412 of the first vertebra 1410. For example, as illustrated with respect to Figure 14A, the inferior vertebral surface 1412 can include patient-specific feature 1414, such as the illustrated recessed region, valley, or divot. A flat endplate that was not customized to the patient-specific topology of the inferior vertebral surface 1412 would result in a gap 1462 at the patient-specific feature 1414 (i.e., the gap 1462 where the first endplate 1440 does not contact the first vertebra 1410). However, in the illustrated embodiment, the contoured first surface 1442 matches the inferior vertebral surface 1412 to increase the area of contact, thereby limiting or reducing stresses, such as stresses in the first vertebra 1410 and/or the device 1430. Further, as a result of the more complete contact made by the first endplate 1440, the device 1430 is expected to have more optimal surface area contact with the first vertebra 1410 to improve the traction of the device 1430 and/or improve the expected outcome for a medical treatment using the device 1430.

The contoured first surface 1442 of the device 1430 can also reduce or limit motion between the first vertebra 1410 and the device 1430. The reduced motion can help reduce spinal fusion time. In some embodiments, the contoured first surface 1442 can have a thickened or protruding region that is substantially geometrically concurrent to the patient-specific feature 1414 along the inferior vertebral surface 1412. This further helps the endplate 1440 to seat against the first vertebra 1410. When an axial load is applied to device 1430, the customized mating at the interface can limit, reduce, or substantially prevent relative movement between device 1430 and the first vertebra 1410. In some procedures, the device 1430 can be configured to provide a generally gapless interface when the device 1430 is in a fully expanded, implanted configuration.

Similarly, the second endplate 1450 includes a first surface 1452 (e.g., an inferior surface) that mates with a superior surface 1422 of the second vertebra 1420 and a second surface 1454 (e.g., a superior surface) that mates with the lockable joint 1438b and/or the lower component 1435. Further, as illustrated in Figures 14A and 14B, the first surface 1452 is customized to the patient-specific topology of the superior surface 1422 of the second vertebra 1420. For example, as illustrated with respect to Figure 14A, the superior surface 1422 can include multiple patient-specific features 1424, such as the illustrated valleys or divots. An endplate that was not customized to the patient-specific topology of the superior surface 1422 would accordingly include one or more gaps 1464 corresponding to the patient-specific features 1424 where the second endplate 1450 does not contact the second vertebra 1420. In contrast, the patient-specific topology of the second endplate 1452 can be contoured to occupy the gap 1464 and therefore contact the superior surface 1422 of the second vertebra 1420 at the patient-specific features 1424. As a result of the more complete contact made by the second endplate 1450, the device 1430 is expected to have more optimal surface area contact with the second vertebra 1420 to improve the traction of the device 1430 and/or improve the expected outcome for a medical treatment using the device 1430.

In some embodiments, the first and second endplates 1440, 1450 can additionally, or alternatively, be customized to a medical treatment prescribed for the patient. In some embodiments, the first and second endplates 1440, 1450 are configured to help provide a height restoration, lordotic correction, and/or coronal correction. For example, the first and second endplates 1440, 1450 can vary in thickness in an x-y plane (e.g., thereby containing a slope) to help provide the lordotic and/or coronal corrections. In some embodiments, the height restoration, lordotic correction, and/or coronal correction provided by the first and second endplates 1440, 1450 can be patient-specific (e.g., based on the amount of prescribed corrections and/or factors specific to the patient, described in more detail below).

In some embodiments, the first and second endplates 1440, 1450 can additionally, or alternatively, be customized to account for load bearing strengths, toughness, fatigue characteristics, or properties that may vary along the endplates 1440, 1450. For example, the first and second endplates 1440, 1450 can compensate for strong and/or weak zones identified in the first and second vertebrae 1410, 1420 that are specific to the patient. In some embodiments, the first and second endplates 1440, 1450 can be configured to apply more force to identified strong or high load bearing zones (e.g., zones comprising bone or tissue with a relative high yield strength, fracture toughness, etc.) in the first and second vertebrae 1410, 1420 and/or apply less force (or no force) to identified weak zones (e.g., zones comprising bone or tissue with a relative low yield strength, fracture toughness, etc.).

In some embodiments, the first and second endplates 1440, 1450 can additionally, or alternatively, be customized to achieve a desired fit. The desired fit can be designed to, for example, reduce motion between the device 1430 and the vertebral bodies, facilitate seating during the implantation procedure, increase friction, or the like. The first and second endplates 1440, 1450 can include anchors, texturing, protrusions, or other suitable elements selected to provide the desired fit.

In addition to (or in alternative to) the patient-specific features of the first and second endplates 1440, 1450, the device 1430 can be configured to provide a precise, predetermined height restoration, lordosis angle correction, and/or coronal angle correction. In some embodiments, the device 1430 can be intraoperatively adjusted. For example, a surgical instrument can be connected to the first and second endplates 1440, 1450 to intraoperatively adjust the lockable joints 1438 until a predetermined lordotic and/or coronal segmental correction is provided by the slope of the first and second endplates 1440, 1450, then the lockable joints 1438 can be locked. In another example, a surgical instrument can be intraoperatively connected to the drive feature 1436 of the device 1430 to expand the device 1430 until a predetermined height restoration is achieved. Once the predetermined height restoration is achieved, the device 1430 can be locked at the configuration that provides the predetermined height restoration. In some embodiments, the device 1430 is pre-operatively adjusted and locked, then inserted to achieve the predetermined correction. In some embodiments, one or more components of the device 1430 are pre-operatively adjusted and locked while other components are intraoperatively adjusted. For example, in some embodiments, the lockable joints 1438a, 1438b can be pre-operatively adjusted and locked to achieve the predetermined angulation of the first and second endplates 1440, 1450, while the device 1430 is expanded in-situ to provide the predetermined height restoration.

In some intraoperative embodiments, the device 1430 is inserted and adjusted to achieve the optimal height and angular correction under surgical navigation guidance. For example, a surgical instrument can be used to monitor the expansion and/or angular correction of the device 1430. In some embodiments, the device 1430 includes lockable mechanical and/or electrical stoppers (not shown) that can be pre-operatively set to stop the expansion and/or angle correction at predetermined points. For example, the device 1430 can include a lockable mechanical mechanism that prevents the device 1430 from expanding past the predetermined height restoration. In these embodiments, the intraoperative adjustments can be accurately adjusted to the predetermined configuration without additional surgical instrumentation by adjusting until the lockable stoppers prevent further adjustment.

Without being bound by theory, the device 1430 is expected to provide several advantages over conventional IBF devices. First, the device 1430 can be configured for two types of adjustments: (1) increasing the space/distance between the first endplate 1440 and the second endplate 1450 (e.g., expansion), such as to restore appropriate intervertebral spacing, and (2) selectively and independently changing the angle of the first endplate 1440 and the second endplate 1450 relative to the main body 1431 via manipulation of the lockable joints 1438a, 1438b, such as to restore appropriate intervertebral alignment. Second, as set forth in detail above, the device 1430 is designed with patient-specific features that are expected to improve performance of the device by improving fit, optimizing load-bearing regions, reducing the likelihood of an overcorrection, or the like. Of course, other advantages of the device 1430 and the present technology will be apparent to those skilled in the art based on this Detailed Description and the Figures. The present technology is therefore not limited by the foregoing advantages. Indeed, additional examples and features of expandable IBF devices are described in U.S. Patent Application No. 17/835,777.

In additional to the foregoing, in some embodiments, as shown in Figure 14B, the device 1430 includes one or more sensors 1476 (sometimes referred to as device sensors), e.g., embedded in, or otherwise coupled to, the device 1430. The sensors 1476 can be pressure sensors, temperature sensors, transducers, accelerometers, fluid sensors, etc. The sensors 1476 measure, when the device 1430 is implanted in the patient, at least one of a pressure exerted by the body of the patient on the device 1430, a temperature of the body of the patient, movement of the body of the patient, etc. In some embodiments, the device 1430 includes one or more actuators 1474 embedded in the device 1430. The actuators 1474 receive electrical signals generated off-site (e.g., from a controller (not shown) positioned external to the patient) and move parts of the device 1430 to place the device 1430 in different physical configurations as described above. For example, the actuators 1474 can provide one or more adjustments or corrections to the physical configuration of the device 1430, expand the device 1430 along the vertical axis, (indicated by arrows 1437 to space apart the first and second vertebrae 1410, 1420), rotate the spatial orientation of the first endplate 1440 and/or the second endplate 1450 relative to the main body 1431 (via the first and second lockable joints 1438a, 1438b), contour and/or otherwise position the device 1430 to match the contours of the first and second vertebrae 1410, 1420, etc.

In some embodiments, as shown in Figure 14B, the auxiliary implant 1453 includes one or more sensors 1480 (sometimes referred to as implant sensors) embedded in the auxiliary implant 1453. The auxiliary implant 1453 is sometimes referred to as a "spinal implant." The sensors 1480 can be pressure sensors, temperature sensors, transducers, accelerometers, fluid sensors, etc. Similar to the sensors 1480 on the device 1430, the sensors 1480 measure, when the spinal implant 1453 is implanted in the patient, at least one of a pressure exerted by the body of the patient on the spinal implant 1453, a temperature of the body of the patient, movement of the body of the patient, etc. However, unlike the device 1430, the spinal implant 1453 is not positioned in a disc space between the first vertebra 1410 and the second vertebra 1420, and therefore the sensors 1480 are positioned to measure one or more physiologic parameters at a position spaced apart from the device 1430. In some embodiments, the parameter(s) measured by the sensors 1476 on the device 1430 and the sensors 1480 on the spinal implant 1453 are compared to aid in determining an appropriate adjustment to the device 1430 and/or the spinal implant 1453. For example, the sensors 1476 may detect a first pressure value indicative of a first load on the device 1430, and the sensors 1480 may detect a second pressure value indicative of a second load on the spinal implant 1453. If the relationship between the first load and the second load is outside a predetermined range (e.g., within 50% of, with 75% of, within 80% of a threshold or range) or does not meet another predetermined metric (e.g., the first load being greater than the second load by particular value, such as at least 10% greater than, at least 20% greater than, at least 50% greater than, etc.), then one or both of the device 1430 and the spinal implant 1453 can be adjusted until the sensed parameters satisfy the predetermined range or other metric.

Accordingly, in some embodiments, the spinal implant 1453 includes one or more actuators 1478 (sometimes referred to as implant actuators) embedded in the spinal implant 1453. The actuators 1478 receive electrical signals generated off-site (e.g., from a controller (not shown) positioned external to the patient) and move parts of the spinal implant 1453 to place the spinal implant 1453 in different physical configurations, as described below.

For example, in some embodiments, the rod 1457 includes a first moveable portion 1470a that can be moved relative to a second moveable portion 1470b of the rod 1457 using the one or more actuators 1478 embedded in the spinal implant 1453. In some embodiments, the rod 1457 includes an inner member 1472 that can be moved relative to the fasteners 1459 that couple the rod 1457 to the vertebrae, such that the rod 1457 telescopes. For example, the one or more actuators 1478 are operable to controllably expand or deploy the spinal implant 1453. The spinal implant 1453 has a deployed or expanded configuration selected based on the treatment to be performed. The patient-specific expansion can be selected based, at least in part, on the design of other components of the spinal implant 1453. The rod 1457 and the device 1430 can be configured to provide a desired range of motion. In some embodiments, the maximum range of motion of the rod 1457 is selected based on a desired range of motion for the device 1430.

The spinal implant 1453 can be configured to expand from a collapsed configuration to an expanded configuration (illustrated in Figure 14B) and can include one or more expansion mechanisms (e.g., screw jack mechanisms, wedges, scissor mechanisms, etc.), angled or sloped surfaces, inflatable members, or other components for causing deployment. Additionally, the spinal implant 1453 can include linkages, pin connections, linkage assemblies, or other components for connecting various other components. In some embodiments, the spinal implant 1453 includes a drive feature (e.g., a drive head, a screw head, a bolt head, etc.) coupleable to a drive instrument.

In some embodiments, a computer system can be coupled to the spinal implant 1453 and/or the device 1430 in either a wired or wireless configuration. The computer system can be the same as or similar to the computing system 100 illustrated and described in more detail with reference to Figure 1. The computer system can be implemented using any of the components illustrated and described in more detail with reference to Figures 1 and 2. The computer system is used to program and adjust the physical configuration of the spinal implant 1453 and/or the device 1430 during and/or after surgery to balance the load exerted by the body of the patient on the spinal implant 1453 and/or the device 1430. The computer system thus adjusts mechanical properties of the spinal implant 1453 and/or the device 1430. During surgery, the spinal implant 1453 and/or the device 1430 is positioned within the patient without loads being applied (i.e., not simulating actual loading when the patient is standing). The initial position of the spinal implant 1453 and/or the device 1430 is sometimes referred to as a "small delivery configuration."

After surgery, the spinal implant 1453 and/or the device 1430 is in a load bearing configuration. The computer system adjusts the configuration of the spinal implant 1453 and/or the device 1430 while the patient is awake after surgery to reduce pain and/or physical discomfort experienced by the patient in real-time; additionally, the configuration can be adjusted to provide an improved biomechanical system including alignment, balance, or spacing. In some embodiments, for example, the computer system receives implant sensor readings from the one or more implant sensors 1480 embedded in the spinal implant 1453. The spinal implant 1453 is configured in a first physical configuration. The implant sensor readings are received after the surgery is performed and the patient is awake. For example, the implant sensor readings are digital and/or analog signals implemented using the methods illustrated and described in more detail with reference to Figure 2. The implant sensor readings are indicative of a load applied by a spine of the patient on the spinal implant 1453. The load can cause physical discomfort to the patient when the spinal implant 1453 is configured in the first physical configuration.

In some embodiments, the computer system receives device sensor readings from the one or more device sensors 1476 embedded in the intervertebral fusion device 1430 implanted in the patient during the surgery. The device sensor readings are received after the surgery is performed and before the implant sensor readings are received. This is because the fusion device 1430 can be adjusted shortly after surgery (before vertebrae have fused) and the rod 1457 can be adjusted months later. Accordingly, in some embodiments whether the device 1430 or the rod 1457 is adjusted depends at last in part on an amount of time elapsed following the surgery. For example, if the computer system determines an adjustment is necessary to reduce patient discomfort, improve load bearing, or the like, the computer system can also determine whether to adjust the device 1430 or the rod 1457. In some embodiments, if the adjustment is to be performed intraoperatively and/or before a certain time has elapsed post-surgery, such as within 3 days, within 1 week, within 2 weeks, etc., the computer system can adjust the device 1430. If the adjustment is to be performed postoperatively and/or after a certain amount of time has elapsed post-surgery, such as more than 3 days post-surgery, more than 1 week post-surgery, more than 2 weeks post-surgery, etc., the computer system can adjust the rod 1457. In this way, the computer system can utilize time elapsed since surgery to help determine appropriate adjustments to reduce patient discomfort, improve load bearing, and otherwise improve long-term surgical outcomes. In some embodiments, the device 1430 and/or the rod 1457 can be adjusted based upon, for example, a surgical plan, a corrective plan, a post-operative therapy plan, or the like. For example, the computer system can generate a corrective plan with a post-operative adjustment protocol, as described with reference to Figure 5. The device 1430 and/or the rod 14572 can be programmed to perform one or more post operative adjustment(s) according to a predetermined schedule. The schedule can include adjustments at specific time(s), adjustments based on sensor reading(s), physician input, etc. In some embodiments, the device 1430 and/or the rod 157 can perform intra- or post-operative adjustments based on real-time collected data to achieve one or more targeted outcomes, such as range of motion, pelvic parameters within targeted ranges, or the like. The device 1430 and/or the rod 157 can confirm adjustments achieve based on their sensor readings and can communicate with each other to monitor and/or confirm spinal corrections.

The computer system extracts a feature vector from the implant sensor readings using a machine learning module of the computer system. The feature vector is indicative of the physical discomfort caused by the load. In some embodiments, the feature vector is further indicative of at least one of lumbar lordosis, Cobb angles, coronal parameters, sagittal parameters, pelvic parameters, disc height, segment flexibility, bone quality, or rotational displacement of the spine of the patient. The use of machine learning to implement the embodiments disclosed herein is illustrated and described in more detail with reference to Figure 1. The feature vector includes features that are individual measurable properties or characteristics of the raw implant sensor readings. For example, the features can be numeric or structural. In one embodiment, the machine learning module applies dimensionality reduction (e.g., via linear discriminant analysis (LDA), principle component analysis (PCA), or the like) to reduce the amount of data in the features for content items to a smaller, more representative set of data.

The computer system generates implant electrical signals using the machine learning module based on the feature vector. The machine learning module is trained based on patient data sets to generate the implant electrical signals to balance the load, such that the physical discomfort caused by the load is reduced. The use of patient data sets is illustrated and described in more detail with reference to Figures 4A-4C and 7A-7D. In some embodiments, the computer system generates device electrical signals using the machine learning module based on the device sensor readings. The machine learning module is trained based on the patient data sets to generate the device sensor readings to reduce physical discomfort caused by the intervertebral fusion device 1430. The computer system transmits the implant electrical signals to the one or more implant actuators 1478 embedded in the spinal implant 1453 to cause the one or more implant actuators 1478 to configure the spinal implant 1453 in a second physical configuration, such that the load is balanced. The machine learning or artificial intelligence module can be run in a feedback loop, such that a first patient is treated and the machine learning module learns to treat the first patient and other patients.

In some embodiments, configuring the spinal implant 1453 in the second physical configuration includes adjusting at least one of a screw, a cage, a plate, the rod 1457, a disk, a spacer, an expandable device, a stent, a bracket, a tie, a scaffold, a fixation device, an anchor, a nut, a bolt, a rivet, a connector, a tether, a fastener, or a joint replacement of the spinal implant using the one or more implant actuators. In other embodiments, the spinal implant 1453 includes a reservoir containing at least one of a narcotic or a steroid. Alternatively, the reservoir can be coupled to the spinal implant 1453. Configuring the spinal implant 1453 in the second physical configuration can include adjusting the reservoir to modify an amount of the narcotic or a steroid delivered to the patient. The adjustment of biochemical properties provides pain management, infection reduction, and a favorable biological response.

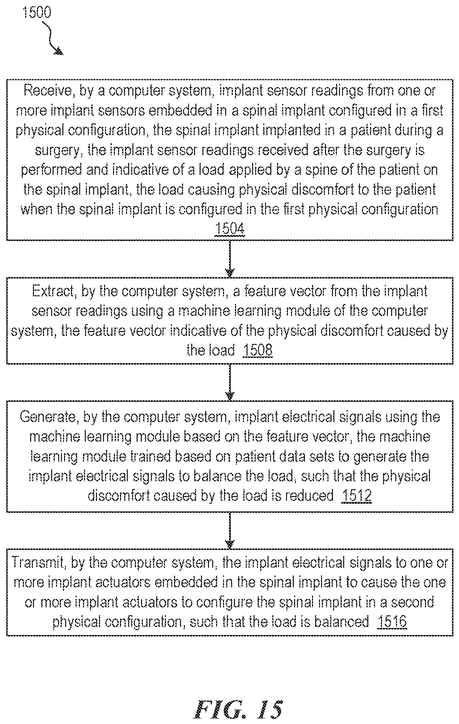

Figure 15 is a flow diagram illustrating a process 1500 for patient-specific adjustment of spinal implants. In some embodiments, the process 1500 of Figure 15 is performed by a computer system, e.g., the example computing system 100 illustrated and described in more detail with reference to Figure 1. Particular entities, for example, the data analysis module 116, illustrated and described in more detail with reference to Figure 1, perform some or all of the steps of the process in other embodiments. Likewise, embodiments may include different and/or additional steps, or perform the steps in different orders.

The computer system receives (1504) implant sensor readings from one or more implant sensors embedded in a spinal implant configured in a first physical configuration. The implant sensors are the same as or similar to the sensors 1476 and/or 1480 illustrated and described in more detail with reference to Figures 14A and 14B. The spinal implant can be the same as or similar to the spinal implant 1453 and/or the device 1430 illustrated and described in more detail with reference to Figures 14A and 14B. The spinal implant is implanted in a patient during a surgery. The implant sensor readings are received after the surgery is performed and are indicative of a load applied by a spine of the patient on the spinal implant. The load causes physical discomfort to the patient when the spinal implant is configured in the first physical configuration.

The computer system extracts (1508) a feature vector from the implant sensor readings using a machine learning module of the computer system. The feature vector is indicative of the physical discomfort caused by the load. In some embodiments, the feature vector is further indicative of at least one of lumbar lordosis, Cobb angles, coronal parameters, sagittal parameters, pelvic parameters, disc height, segment flexibility, bone quality, or rotational displacement of the spine of the patient. The use of machine learning to implement the embodiments disclosed herein is illustrated and described in more detail with reference to Figure 1.

The computer system generates (1512) implant electrical signals using the machine learning module based on the feature vector. The machine learning module is trained based on patient data sets to generate the implant electrical signals to balance the load, such that the physical discomfort caused by the load is reduced. The use of patient data sets is illustrated and described in more detail with reference to Figures 4A-4C and 7A-7D. In some embodiments, the computer system generates device electrical signals using the machine learning module based on the device sensor readings. The machine learning module is trained based on the patient data sets to generate the device sensor readings to reduce physical discomfort caused by the intervertebral fusion device 1430.

The computer system transmits (1516) the implant electrical signals to one or more implant actuators embedded in the spinal implant to cause the one or more implant actuators to configure the spinal implant in a second physical configuration, such that the load is balanced. The implant actuators can be the same as or similar to the actuators 1474 and/or 1478 illustrated and described in more detail with reference to Figures 14A and 14B. The machine learning or artificial intelligence module can be run in a feedback loop, such that a first patient is treated and the machine learning module learns to treat other patients.

As one skilled in the art will appreciate from the disclosure herein, the device 1430 is provided as a simple schematic example of a patient-specific IBF device. Because the patient-specific implants described herein are designed to match individual patient anatomy, the size, shape, and geometry of the patient-specific implant will vary according to individual patient anatomy. The present technology is thus not limited to any particular IBF device or implant design and can therefore include other implants beyond those illustrated or described herein, including replacements for other discs or joints not expressly described herein. In various embodiments, a spinal procedure may include implantation of implants at one or multiple levels.

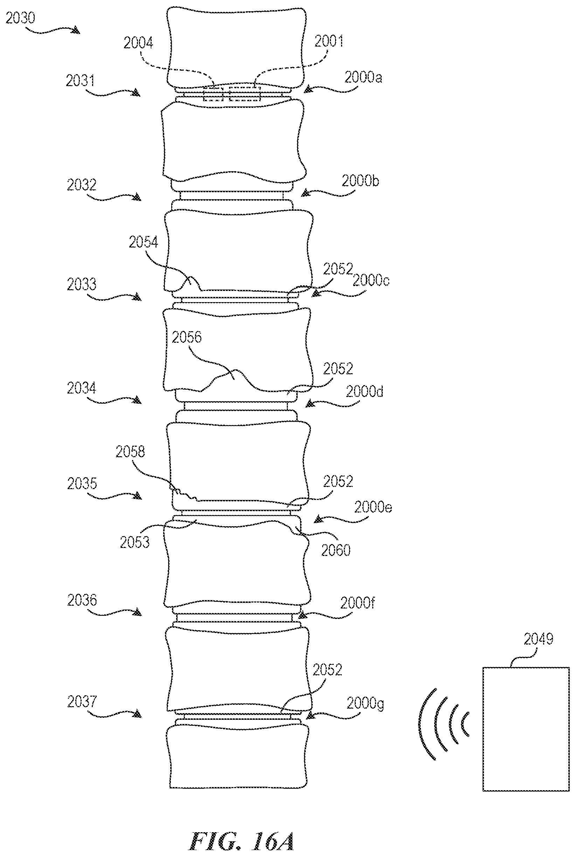

Figure 16A shows a patient's spine 2030 with intervertebral implants located at each individual level. The implants 2000a-g (collectively, "implants 2000") can be tailored to fit with anatomical features at the individual levels. Non-invasive post-operative spine adjustments can be performed using a remote device or controller 2049 that controls actuation of the implants 2000. The remote device 2049 can communicate wirelessly with selected implants or all of the implants.

An implant 2000a is implanted at a level 2031 with normal endplates free from any defect in the surface topology. The endplates of the implant 2000a can have convex shapes that match the illustrated concave endplates of the adjacent vertebrae at the level 2031. The implant 2000a can have an actuation mechanism 2001 that can be powered by an externally applied field (e.g., magnetic field or another field) provided by the remote device 2049. The actuation mechanism 2001 can include inductively rechargeable power sources, actuation elements, processors, transmitters/receivers, etc. The position, number, and capabilities of the actuation mechanisms can be selected based on the available adjustability (e.g., range expansion/contraction, drive force, etc.).

The implants 2000 can have patient specific features. An implant 2000b is implanted at a level 2032 with a severe concave shape in the superior and inferior vertebra. The implant 2000b has large convex contours that match the corresponding concave shape of the superior vertebra. An implant 2000c is implanted at a level 2033 with a superior endplate having a focal defect adjacent, but not on, a longitudinal side of the superior vertebra. The implant 2000d has an upper endplate 2052 with a contouring feature 2056 generally corresponding to the focal defect to better fit the superior endplate. Focal defects in a patient's spine can range from relatively small cavities (e.g., as shown at the level 2033) to relatively large valleys (e.g., as shown at the level 2034). Further, focal defects can include protrusions (not shown) where excess bone and/or cartilage is collected, requiring concave contouring features in the endplates of the implants to match them. An implant 2000e is implanted a level 2035 with corner defects in the superior and inferior vertebrae. Corner defects are located at least partially on longitudinal sides of the vertebrae. Corner defects can include missing corners that are cut off at varying angles, protrusions (not shown) at the corners, and/or rough topology at the corners (e.g., on the missing corner, on the protrusion, and/or on the otherwise normal surface of the corner). The implant 2000e has an upper endplate 2052 with a periphery contour 2058 configured to fit the corner defect in the superior endplate and a lower endplate 2053 with a periphery contour 2060 configured to fit the corner defect in the inferior endplate. Other adjacent levels, such as level 2036, can be formed by endplates with relatively smooth and planar or straight topologies. In such embodiments, an implant 2000f with relatively smooth contouring can be implanted at level 2036.

An implant 2000g is implanted at level 2037 with a superior vertebra having erosive defects on the inferior surface of the superior vertebra. The external device 2049 can command the implant 2000g to move to a target position. As illustrated, erosive defects can span the entire surface of a vertebra and include multiple valleys and peaks therein. In some patients, erosive defects can be contained to a focal region and/or a corner region of a surface. In some patients, erosive defects can include one or more deep valleys and/or one or more tall peaks. As illustrated, the implant 2000g can have an upper endplate 2052 configured to mate with the erosive defects in the superior vertebra.

Figure 16B illustrates an exemplary corrective plan 2100 (e.g., as generated in step 515 of the method 500) for a patient-specific surgical procedure that may be used and/or generated in connection with the methods described herein, according to an embodiment. The corrective plan 2100 can be an adjustable-implant corrective plan that incorporates all or some of surgical plans or other plans disclosed herein. The corrective plan 2100 can include, without limitation, intra- and/or pre-operative patient metrics (e.g., pre-operative patient metrics 1002 discussed in connection with Figures 10-13), predicted post-operative patient metrics (e.g., predicted post-operative patient metrics 1004 discussed in connection with Figures 10-13), and adjustment metrics 2110.

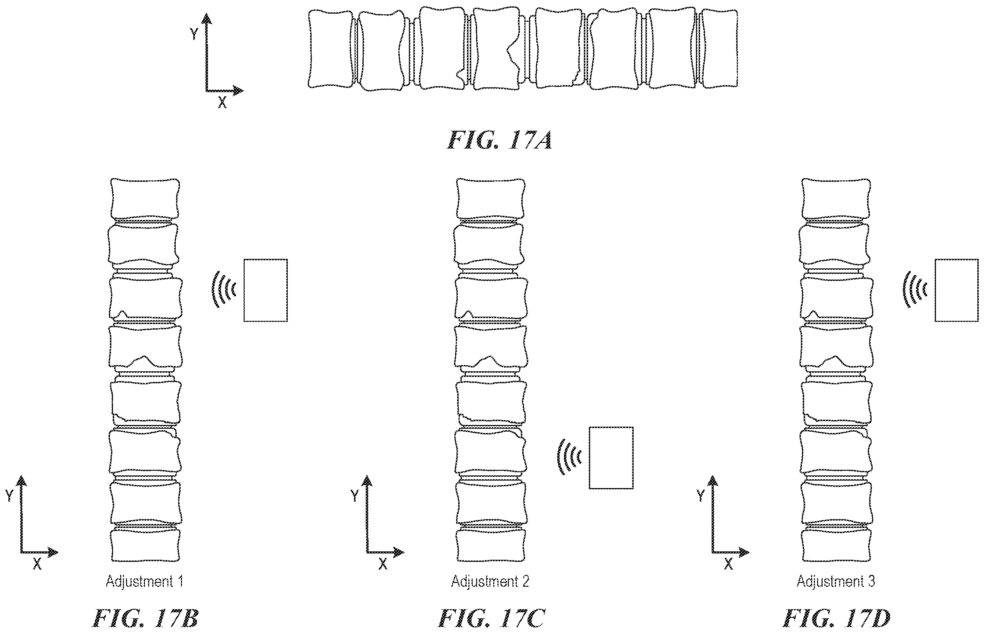

The adjustment metrics 2110 can include any number of planned adjustments to an adjustable spinal implant. The illustrated corrective plan 2100 includes planned adjustments 2120a, 2120b, 2120c (collectively "adjustments 2120"). Each adjustment 2120 can include associated post-adjustment metrics reviewable by the physician. For example, the physician can review and approve these metrics by selecting an approve button. The computing system can then design the adjustable implants based on the approved adjustment (e.g., design the adjustable implant to have an adjustable range of motion capable of accommodating the approved adjustment(s)). If the physician wants to modify adjustments, the physician can select the modify button. The physician can then input one or more parameters or metrics for adjustment. The computing system can update the spinal model accordingly to the inputted parameters or metrics. Arrows can (e.g., arrows 2130a, 2130b, 2130c) indicate adjustments, such as range of motion, adjustment values, etc. Adjustments 2 and 3 include adjustment indicators (illustrated as arrows) showing planned adjustments, such as the adjustments discussed in connection with Figures 17A-17D. The physician can approve/select individual target intra-operative configurations and/or post-operative configurations for different loading conditions.

Figures 17A-17Dshow a patient's spine in different orientations resulting in different loading of implants. Figure 17A shows the patient's spine in a generally horizontally orientation. For example, the implants can be implanted when the patient's body is generally horizontal so that the spine is generally unloaded. During surgery, it may be difficult to determine how loading of spine will compare to the predicted loading. Accordingly, the implants can be reconfigured post-operatively to move the spine to a target post-operative configuration. Figures 17B-17D show adjustment for the post-operative patient's spine in a vertical orientation (e.g., sitting or standing), although post-operative adjustments can also be performed with the patient in other orientations. This allows for post-operative adjustments based on post-operative loading, dynamic visualization, etc.

In spinal fusion procedures, implants can be adjusted shortly after surgery (e.g., hours, days, etc.) to position at vertebrae for fusion. In spinal alignment procedures, the implants can be vertebral discs adjusted periodically to compensate for patient improvement, disease progression, etc. For example, Adjustments 1-3 of Figures 17B-17D can be performed monthly, yearly, or at physician determined intervals. The number of adjustment sessions, period between adjustment sessions, and alterations to the spine can be selected based on a treatment plan, patient recovery, or the like. For example, the system of Figure 1 and computer device of Figure 2 can be used to generate correction and adjustments plans. For example, a computer system can be used to determine a corrected anatomical configuration of a patient for achieving a target treatment outcome. The computer system can predict disease progression for a disease affecting the patient's spine based on a patient data set of a patient using at least one machine learning model. The computer system can identify the actuatable implant configured to be implanted in the patient to achieve the corrected anatomical configuration. The actuatable implant is movable between multiple configurations to compensate for the predicted disease progression based on the target treatment outcome. The at least one machine learning model can determine whether to reconfigure the at least one device based on post-adjustment images. The post-adjustment images can include dynamic sit/stand x-ray images, and in some adjustment procedures, the spine can be visualized (e.g., using fluoroscopy) while invasively or non-invasively actuating the actuatable implant.

In some embodiments, one or more anatomical corrections for the patient are generated based on pre-adjustment images and a patient-specific pre-surgical correction plan. A computer system can generate a series of corrected anatomical models representing anatomical changes over a period of time based on a patient-specific correction to the native anatomy and a predicted disease progression. The corrected anatomical models can be viewed and modified by a user as part of the pre-surgical correction plan. The pre-surgical correction plan can be generated by comparing a patient data set to a plurality of reference patient data sets to identify one or more similar patient data sets in the plurality of reference patient data sets, and each similar patient data set corresponds to a reference patient that (a) has similar spinal pathology data as the patient and/or (b) received treatment with an post-operative adjustable orthopedic implant. In some embodiments, a virtual model of the spine is generated. The predicted disease progression using the virtual model. An actuatable implant can be designed to fit the virtual model throughout the predicted disease progression. Simulations can be modified and rerun based on the post-operative adjustments (see Figures 17A-17D). Additional implants configured to cooperate with the actuatable implant can be designed to achieve the target treatment outcome and be configured for multi-level adjustments. The plans disclosed herein an provide results (e.g., analytics for each level, overall spine correction score, etc.) from simulations of the multi-level adjustments.

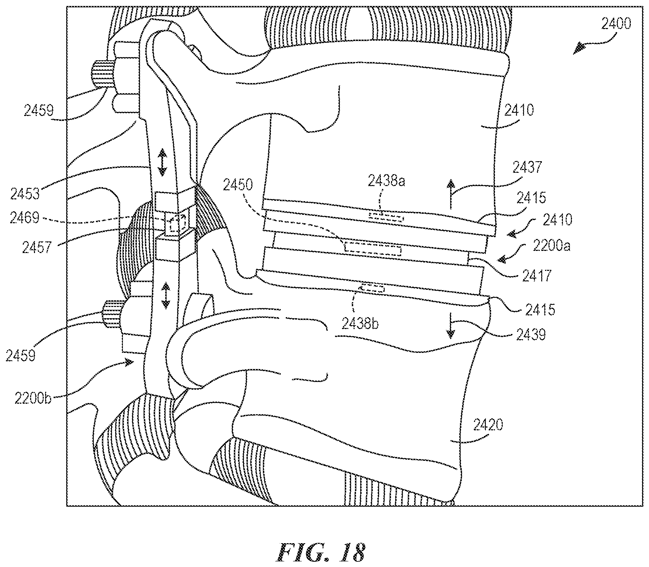

Figure 18 shows an implant system 2400 including implants 2200a, 2200b (also referred to herein as "patient devices" 2200a, 2200b) that can selectively and independently be non-invasively reconfigured. A computer system can receive a patient data set of a patient and can compare the patient data set to a plurality of reference patient data sets to identify one or more similar patient data sets in the plurality of reference patient data sets. A subset of the one or more similar patient data sets can be selected such that each similar patient data set of the selected subset includes data indicative of a favorable treatment outcome. The system can identify for at least one similar patient data set of the selected subset, surgical procedure data and medical device design data and implant adjustment data associated with the favorable treatment outcome. Based on the medical device design data and implant adjustment data surgical procedure data and the medical device design data, the computer system generates at least one patient-specific surgical procedure and at least one patient-specific medical device design for the patient. At least one patient-specific medical device design is configured to be actuated non-invasively using an internal actuator 2450 (shown in phantom line).

In some embodiments, a series of corrected anatomical models represent predicted anatomical changes over a period of time based on a patient-specific correction to the patient's native anatomy and a predicted disease progression. A plurality of treatment locations can be identified along the patient's spine. The implants 2200a, 2200b can be designed for each treatment location based on the patient-specific correction and to compensate for the anatomical changes by post-operative adjustments to the implants. The physician can review the corrected anatomical models and can modify/approve the corrected anatomical models, as discussed above.

The implants 2200a, 2200b can be configured to implement a corrective treatment plan(s), correction, and/or to correct for deformities of the spine described by, without limitation, lumbar lordosis, Cobb angles, coronal parameters (e.g., coronal balance, global coronal balance, coronal pelvic tilt, etc.), sagittal parameters (e.g., pelvic incidence, sacral slope, thoracic kyphosis, etc.), pelvic parameters, combinations thereof, or the like. The implant system 2400 can store, for example, algorithms that use collected data (e.g., sensor readings, device setting/configuration data, etc.) as inputs used to determine the pathology (e.g., discs height, segment flexibility, bone quality, rotational displacement), and the algorithm can use these additional inputs to further define optimal implant configurations for the current pathology. To treat multiple disease/conditions, the implant system 2400 can predict outcomes for one or more conditions/diseases based on adjustments. The predicted outcomes can then be ranked, categorized, weighted, aggregated, etc. to evaluate the patient's condition/state and determine whether to adjust implant configurations, adjustment protocols, or patient monitoring plans, etc. This allows conditions/diseases to be ranked and prioritized. In some embodiments, the implants can collect data to track fatigue or service life. In some embodiments, the implant system 2400 uses sensor readings for diagnostics.

The patient devices 2200a, 2200b can be moved concurrently or sequentially to provide targeted corrections. Additional sensor data can be collected during this process to monitor adjustments. If applied loads reach maximum allowable values, the patient devices 2200a, 2200b can reduce actuation speeds or determine an alternative target configuration. The implant system 2400 then determines configurations for one or more of the patient devices 2200a, 2200b to provide the target anatomical configuration or correction. In some implementations, the implant system 2400 can generate the target anatomical configuration or correction based on sensor readings. If the patient devices 2200a, 2200b detect values (e.g., strains, loads, forces, pressures, etc.) that are outside of an acceptable range, the patient devices 2200a, 2200b move to another configuration to keep the detected values within the range, thereby limiting or avoiding unwanted states or conditions.

If a threshold correction cannot be achieved, a notification can be transmitted to a remote device to alert the patient and/or healthcare provider. Additional treatments or surgical procedures can then be performed. In some embodiments, the patient devices 2200a, 2200b are programmed with one or more target anatomical configuration for the patient, such a spine curvature, vertebral spacing, etc. The patient devices 2200a, 2200b can communicate with each other to monitor the current anatomical configuration of the patient. If the anatomical configuration is outside an acceptable range or above/below a predetermined value, the patient devices 2200, 2200b can automatically move anatomical features (e.g., vertebral bodies) to an acceptable configuration for height restoration, angle correction (e.g., lordosis angle correction, coronal angle correction, etc.), or the like.

The difference between the native anatomical configuration and the corrected anatomical configuration may be referred to as a "patient-specific correction" or "target correction." The embodiments, features, systems, devices, materials, methods, and techniques described herein may, in certain embodiments, be applied to or used in connection with any one or more of the embodiments, features, systems, or devices. In some embodiments, mathematical rules defining patient-specific corrections, optimal anatomical outcomes (e.g., positional relationships between anatomic elements) and/or post-operative metrics/design criteria (e.g., adjusted anatomies) are used so that post-operative metrics are within an acceptable range for a patient state (e.g., laying down, standing vertical, etc.). Target post-operative metrics can include, but are not limited to, target coronal parameters, target sagittal parameters, target pelvic incidence angle, target Cobb angle, target shoulder tilt, target iliolumbar angle, target coronal balance, target lordosis angle, and/or a target intervertebral space height. For example, the sagittal vertical axis can be less than a set value (e.g., 6 mm, 7 mm, 9 mm, etc.) and the post-operative Cobb angle less than a set value (e.g., 8 degrees, 9 degrees, 10 degrees, etc.), etc., when the patient stands vertically.

The implanted patient device 2200a can be an artificial disk with sensors 2438a, 2438b (collectively "sensors 438") configured to measure pressures, loads, or forces applied by the first vertebra 410 (e.g., a relatively superior vertebra) and a second vertebra 2420 (e.g., a relatively inferior vertebra). The patient device 2200a can transmit sensor readings from the sensors 2438a, 2438b to the patient device 2200b, illustrated as an extendable rod assembly with sensors (e.g., force sensors, pressure sensors, etc.) and a controller 2469. The patient devices 2200a, 2200b can receive signals transmitted from a controller positioned external to the patient, and in response move to another configuration based on the received signals. In other embodiments, the patient device 2200a generates and transmits commands to the patient device 2200b, or vice versa. Both patient devices 2200a and 2200b can expand to increase the height of an intervertebral space 2410 to, for example, reduce or eliminate nerve compress while maintaining target characteristics (e.g., curvature, alignment, etc.) of the spine. The amount of expansion provided by each device can be substantially the same (e.g., to maintain current spine curvature along that section of the spine) or substantially different (e.g., to alter the spine curvature along that section of the spine), etc. In some embodiments, the characteristics of the patient device 2200a are controlled based on the sensor readings. Such characteristics include compressibility, range of articulation, etc.

The implanted patient devices 2200 may have modules or programs that incorporate one or more mathematical rules based on ranges or thresholds for various disease metrics. For example, an intervention timing module may indicate surgical intervention is necessary if one or more disease metrics exceed a predetermined threshold or meet some other criteria. Representative thresholds that indicate adjustment may be necessary include SVA values greater than 7 mm, a mismatch between lumbar lordosis and pelvic incidence greater than 10 degrees, a Cobb angle of greater than 10 degrees, and/or a combination of Cobb angle and LL/PI mismatch greater than 20 degrees. In some embodiments, indications of adjustment can be based on a percentage change over a period of time. For example, if the SVA value increases by 20% over a period of time, the patient devices 200 can reconfigure themselves to lower the SVA value within 10% of a target SVA value. Other threshold values and metrics can be used; the foregoing are provided as examples only and in no way limit the present disclosure. In some embodiments, the foregoing rules can be tailored to specific patient populations (e.g., for males over 50 years of age or females over 40 years of age, etc.). If a particular patient does not exceed the thresholds indicating adjustment is recommended, the implanted patient devices 2200a, 2200b may provide an estimate for when the patient's metrics will exceed one or more thresholds, thereby providing the patient with an estimate of when adjustment may become recommended. The estimations can be transmitted to an external device for viewing.

The present technology may also include a treatment planning module that can identify the optimal type of correction based on the disease progression of the patient. The treatment planning module can be an algorithm, machine learning model, or other software analytical tool trained or otherwise based on a plurality of reference patient data sets, as previously described. The treatment planning module may also incorporate one or more mathematical rules for identifying adjustments to counter or slow disease progression. As a non-limiting example, if a LL/PI mismatch is between 5 and 10 degrees, the treatment planning module may recommend adjustment to lower LL/PI mismatch below 5 degrees.

The patient device 2200 can include patient-specific endplates 2415 and a main body 2417. The endplates can be configured to engage the vertebral bodies 2410 and 2420, respectively. The main body 2417 can include electronics (e.g., processors, storage devices, communication devices, etc.), sensors 2438a, 2438b, and actuation devices 2450. In some embodiments, an actuation device is fluidically driven and the sensors 2438a, 2438b are pressure sensors.

In some embodiments, the implant 2000a and/or the implant 2000b can also be used with non-patient-specific devices and implants. The systems disclosed herein can include any number of implants designed for implantation at different locations to provide a specific treatment.

In some procedures, the patient device 2200a can be in a collapsed or low-profile configuration between the first and second vertebrae 2410, 2420. For example, the device 2200a can be inserted manually in the collapsed configuration with surgical navigation or via a surgical robot and then expanded at the implantation site. Once deployed/expanded, as described in more detail below, the device 2200a can provide one or more adjustments, corrections (e.g., corrections to the alignment of the first and second vertebrae 2410, 2420, spinal segments, etc.), or the like. Figure 18 shows the implant 2200a fully expanded along a vertical axis, indicated by arrows 2437, 2439, to hold apart the first and second vertebrae 2410, 2420. When the implant 2200a is implanted, the patient can be laying generally horizontal so that the spine is generally unloaded. After surgery, the patient can stand vertically or perform one or more tasks while the sensors 2438a, 2438b continuously or periodically collect data for adjustment of the patient device 2200a. An external device such as a controller or other computing system can control data collection, including sampling rates, detection schedules, etc. The system 2400 can transmit the data to the remote device or network. The remote device or network can determine one or more settings (e.g., height, stiffness, position of endplates, etc.) based on the received data. The settings are sent to the implants 2200a, 2200b so that the implants move to respective new target configurations. Accordingly, the implants 2200a, 2200b can also be referred to as "networked" implants. The process can be performed any number of times (e.g., monthly, yearly, etc.) for treatment adjustability.

The implants 2200a, 2200b can be programmed to detect adverse events, such as excessive loading, displacement (e.g., lateral movement), or the like. The implants 2200a, 2200b can automatically control adjustment to compensate for the adverse event. For example, if posterior loading exceeds a threshold value, the implant 2200a can command the fixation rod implant 2200b to lengthen to reduce the posterior loading on the implant 2200a. The number, configurations, and capabilities of the patient devices 2200a, 2200b can be selected based on the treatment. For example, an intervertebral device (e.g., cage, artificial disc, etc.) can be at each level for treatment.

The networked systems and devices disclosed herein can include a data storage element storing patient-specific data, a retrieval feature for accessing patient-specific data, or the like. A data storage module having a memory storing data and a retrieval module configured to transmit the patient-specific surgical plan from the data storage module to a surgical platform can be configured to execute one or more aspects of the patient-specific surgical plan. Patient-specific data is therefore linked to the patient-specific implant. Data can be accessed after the implant is implanted. Data can be used to confirm aspects of the implant/surgery (e.g., is the implant correctly positioned) and be combined, aggregated, and analyzed with post-implantation data (e.g., state of implant data, configuration data, sensor data, etc.). U.S. App. No. 16/990,810 discloses features, systems, devices, materials, and methods that can be incorporated into or used with the networked systems and devices disclosed herein. U.S. App. No. 16/990,810.

In some embodiments, the present technology can also predict, model, and/or simulate disease progression. For example, a disease progression module may simulate what a patient's anatomy may look like one, two, five, or 10 years post-surgery for several surgical intervention options. The simulations may also incorporate non-surgical factors, such as patient age, height, weight, sex, activity level, other health conditions, or the like, as previously described. Based on these simulations, the system and/or a surgeon can select which surgical intervention is best suited for long-term efficacy. These simulations can also be used to determine patient-specific corrections that compensate for the projected diseases progression. The networked systems and devices can generate data to monitor and predict disease progression. In some embodiments, one or more of the implantable devices includes a disease progression module for local analysis of data. In other embodiments, a remote computing device can include the disease progression module. As the implanted networked systems adjust corrections, the disease progression module can continuously or periodically predict disease progression.

The systems disclosed herein can also include multiple disease progression models (e.g., two, three, four, five, six, or more) that are simulated to provide disease progression data for several different surgical intervention options or other scenarios. For example, the disease progression module can generate models that predict post-surgical disease progression for each of three different surgical interventions. A surgeon or other healthcare provider can review the disease progression models and, based on the review, select which of the three surgical intervention options is likely to provide the patient with the best long-term outcome. Of course, selecting the optimal adjustments can also be fully or semi-automated, as described herein. The implanted networked system can be programmed with the multiple disease progression models. The disease progression models can be modified based on the collected data and healthcare provider, etc.

In some embodiments, networked implants can be used to correct numerous different maladies in a variety of contexts, including spine surgery, hand surgery, shoulder and elbow surgery, total joint reconstruction (arthroplasty), skull reconstruction, pediatric orthopedics, foot and ankle surgery, musculoskeletal oncology, surgical sports medicine, or orthopedic trauma. The implants can dynamically correct for irregular spinal curvature, such as scoliosis, lordosis, or kyphosis (hyper- or hypo-), and irregular spinal displacement (e.g., spondylolisthesis). As such, corrections can be varied over time to compensate for disease progress and growth of the patient (e.g., devices implanted when patient is not fully grown, etc.). The networked devices can be designed to treat osteoarthritis, lumbar degenerative disk disease or cervical degenerative disk disease, lumbar spinal stenosis, or cervical spinal stenosis.

As one skilled in the art will appreciate, any of the software functions described previously may be combined or distributed into one or more software functions or devices for performing the operations described herein. Accordingly, any of the operations described herein can be performed by any of the computing devices or systems described herein, unless expressly noted otherwise.

As one skilled in the art will appreciate, any of the software modules described previously may be combined into a single software module for performing the operations described herein. Likewise, the software modules can be distributed across any combination of the computing systems and devices described herein, and are not limited to the express arrangements described herein. Accordingly, any of the operations described herein can be performed by any of the computing devices or systems described herein, unless expressly noted otherwise.

### Conclusion

The foregoing detailed description has set forth various embodiments of the devices and/or processes via the use of block diagrams, flowcharts, and/or examples. Insofar as such block diagrams, flowcharts, and/or examples contain one or more functions and/or operations, it will be understood by those within the art that each function and/or operation within such block diagrams, flowcharts, or examples can be implemented, individually and/or collectively, by a wide range of hardware, software, firmware, or virtually any combination thereof. In some embodiments, several portions of the subject matter described herein may be implemented via Application Specific Integrated Circuits (ASICs), Field Programmable Gate Arrays (FPGAs), digital signal processors (DSPs), or other integrated formats. However, those skilled in the art will recognize that some aspects of the embodiments disclosed herein, in whole or in part, can be equivalently implemented in integrated circuits, as one or more computer programs running on one or more computers (e.g., as one or more programs running on one or more computer systems), as one or more programs running on one or more processors (e.g., as one or more programs running on one or more microprocessors), as firmware, or as virtually any combination thereof, and that designing the circuitry and/or writing the code for the software and or firmware would be well within the skill of one of skill in the art in light of this disclosure. In addition, those skilled in the art will appreciate that the mechanisms of the subject matter described herein are capable of being distributed as a program product in a variety of forms, and that an illustrative embodiment of the subject matter described herein applies regardless of the particular type of signal bearing medium used to actually carry out the distribution. Examples of a signal bearing medium include, but are not limited to, the following: a recordable type medium such as a floppy disk, a hard disk drive, a CD, a DVD, a digital tape, a computer memory, etc.; and a transmission type medium such as a digital and/or an analog communication medium (e.g., a fiber optic cable, a waveguide, a wired communications link, a wireless communication link, etc.).

Those skilled in the art will recognize that it is common within the art to describe devices and/or processes in the fashion set forth herein, and thereafter use engineering practices to integrate such described devices and/or processes into data processing systems. That is, at least a portion of the devices and/or processes described herein can be integrated into a data processing system via a reasonable amount of experimentation. Those having skill in the art will recognize that a typical data processing system generally includes one or more of a system unit housing, a video display device, a memory such as volatile and non-volatile memory, processors such as microprocessors and DSPs, computational entities such as operating systems, drivers, graphical user interfaces, and applications programs, one or more interaction devices, such as a touch pad or screen, and/or control systems including feedback loops and control motors (e.g., feedback for sensing position and/or velocity, control motors for moving and/or adjusting components and/or quantities). A typical data processing system may be implemented utilizing any suitable commercially available components, such as those typically found in data computing/communication and/or network computing/communication systems.

The herein described subject matter sometimes illustrates different components contained within, or connected with, different other components. It is to be understood that such depicted architectures are merely examples, and that in fact many other architectures can be implemented which achieve the same functionality. In a conceptual sense, any arrangement of components to achieve the same functionality is effectively "associated" such that the desired functionality is achieved. Hence, any two components herein combined to achieve a particular functionality can be seen as "associated with" each other such that the desired functionality is achieved, irrespective of architectures or intermediate components. Likewise, any two components so associated can also be viewed as being "operably connected," or "operably coupled," to each other to achieve the desired functionality, and any two components capable of being so associated can also be viewed as being "operably couplable" to each other to achieve the desired functionality. Specific examples of operably couplable include but are not limited to physically mateable and/or physically interacting components and/or wirelessly interactable and/or wirelessly interacting components and/or logically interacting and/or logically interactable components.

The ranges disclosed herein also encompass any and all overlap, sub-ranges, and combinations thereof. Language such as "up to," "at least," "greater than," "less than," "between," or the like includes the number recited. Numbers preceded by a term such as "approximately," "about," and "substantially" as used herein include the recited numbers (e.g., about 10%=10%), and also represent an amount close to the stated amount that still performs a desired function or achieves a desired result. For example, the terms "approximately," "about," and "substantially" may refer to an amount that is within less than 10% of, within less than 5% of, within less than 1% of, within less than 0.1% of, and within less than 0.01% of the stated amount.

From the foregoing, it will be appreciated that various embodiments of the present disclosure have been described herein for purposes of illustration, and that various modifications may be made without departing from the scope of the present disclosure. Accordingly, the various embodiments disclosed herein are not intended to be limiting.

## Claims

1. A system (100), comprising:
one or more computer processors (210); and
a non-transitory, computer-readable storage medium (250) storing computer instructions, which when executed by the one or more computer processors (210), cause the one or more computer processors to:
receive first implant sensor readings from one or more first implant sensors (1480) of a first implant device (1453) implanted in a patient and configured in a first physical configuration, the first implant sensor readings indicative of a first load applied by a spine of the patient on the first implant device (1453);
receive second implant sensor readings from one or more second implant sensors (1476) of a second intervertebral implant device (1430) implanted in the patient and configured in a second physical configuration, the second implant sensor readings indicative of a second load applied by the spine of the patient on the second intervertebral implant device (1430);
determine whether the first load and the second load comply with a predetermined metric;
extract, in response to determining that the first load or the second load do not meet the predetermined metric, a feature vector from the first and/or second implant sensor readings using a machine learning module (116, 264) of the system, the feature vector indicative of a target correction according to a corrective plan for balancing the first load and the second load in compliance with the predetermined metric;
generate implant electrical signals using the machine learning module (116, 264) and based on the feature vector, the machine learning module trained based on patient data sets to generate the implant electrical signals to adjust the first load or the second load; and
transmit the implant electrical signals to at least one of one or more first implant actuators (1478) of the first implant device (1453) and one or more second implant actuators (1474) of the second intervertebral implant device (1430) to move at least one of the first implant device or the second intervertebral implant device to a different physical configuration for the target correction.

2. The system of claim 1, wherein the feature vector is further indicative of at least one of lumbar lordosis, Cobb angles, coronal parameters, sagittal parameters, pelvic parameters, disc height, segment flexibility, bone quality, or rotational displacement of the spine of the patient.

3. The system of claim 1, wherein configuring the first implant device in the different physical configuration comprises:
adjusting at least one of a screw, a cage, a plate, a rod, a disk, a spacer, an expandable device, a stent, a bracket, a tie, a scaffold, a fixation device, an anchor, a nut, a bolt, a rivet, a connector, a tether, a fastener, or a joint replacement of the first implant device using the one or more first implant actuators.

4. The system of claim 1 wherein configuring the first implant device in the different physical configuration comprises:
adjusting a reservoir coupled to the first implant device to modify an amount of at least one of a pharmacological, a biological, a biochemical, a narcotic, or a steroid delivered to the patient.

5. The system of claim 1 wherein the computer instructions, when executed by the one or more computer processors, further cause the one or more computer processors to:
receive patient data;
determine an anatomical configuration of the patient's spine based on the patient data, and
identify the target correction based on the anatomical configuration and available adjustability of the first implant device and the second intervertebral implant device, wherein the target correction is used to extract the feature vector.

6. The system of claim 1, wherein the corrective plan comprises criteria for actuating one or more first implant actuators of the first implant device.

7. A non-transitory, computer-readable storage medium (250) storing computer instructions, which when executed by one or more computer processors (210), cause the one or more computer processors to:
receive first implant sensor readings from one or more first implant sensors (1480) of a first implant device (1453) implanted in a patient and configured in a first physical configuration, the first implant sensor readings indicative of a first load applied by a spine of the patient on the first implant device;
receive second implant sensor readings from one or more second implant sensors (1476) of a second intervertebral implant device (1430) implanted in the patient and configured in a second physical configuration, the second implant sensor readings indicative of a second load applied by the spine of the patient on the second intervertebral implant device;
determine whether the first load and the second load comply with a predetermined metric;
extract, in response to determining that the first load or the second load do not meet the predetermined metric, a feature vector from the first and/or second implant sensor readings using a machine learning module (116, 264), the feature vector indicative of a target correction for balancing the first load and the second load in compliance with the predetermined metric;
generate implant electrical signals using the machine learning module (116, 264) and based on the feature vector, the machine learning module trained based on patient data sets to generate the implant electrical signals to adjust the first load or the second load; and
transmit the implant electrical signals to at least one of one or more first implant actuators (1474) of the first implant device (1453) and one or more second implant actuators (1474) of the second intervertebral implant device (1430) to move at least one of the first implant device (1453) or the second intervertebral implant device (1430) to a different physical configuration for the target correction.

8. The non-transitory, computer-readable storage medium of claim 7, wherein the feature vector is further indicative of at least one of lumbar lordosis, Cobb angles, coronal parameters, sagittal parameters, pelvic parameters, disc height, segment flexibility, bone quality, or rotational displacement of the spine of the patient.

9. The non-transitory, computer-readable storage medium of claim 7, wherein configuring the first implant device in the different physical configuration comprises:
adjusting at least one of a screw, a cage, a plate, a rod, a disk, a spacer, an expandable device, a stent, a bracket, a tie, a scaffold, a fixation device, an anchor, a nut, a bolt, a rivet, a connector, a tether, a fastener, or a joint replacement of the first implant device using the one or more first implant actuators.

10. The non-transitory, computer-readable storage medium of claim 7, wherein configuring the first implant device in the different physical configuration comprises:
adjusting a reservoir coupled to the first implant device to modify an amount of at least one of a pharmacological, a biological, a biochemical, a narcotic, or a steroid delivered to the patient.

## Patentansprüche

1. Ein System (100), beinhaltend:
einen oder mehrere Computerprozessoren (210); und
ein nicht transitorisches, computerlesbares Speicherungsmedium (250), das Computeranweisungen speichert, die bei Ausführung durch den einen oder die mehreren Computerprozessoren (210) den einen oder die mehreren Computerprozessoren zu Folgendem veranlassen:
Empfangen erster Implantatsensormesswerte von einem oder mehreren ersten Implantatsensoren (1480) einer ersten Implantatvorrichtung (1453), die in einem Patienten implantiert ist und in einer ersten physischen Konfiguration konfiguriert ist, wobei die ersten Implantatsensormesswerte eine erste Last angeben, die durch eine Wirbelsäule des Patienten auf die erste Implantatvorrichtung (1453) ausgeübt wird;
Empfangen zweiter Implantatsensormesswerte von einem oder mehreren zweiten Implantatsensoren (1476) einer zweiten intervertebralen Implantatvorrichtung (1430), die in dem Patienten implantiert ist und in einer zweiten physischen Konfiguration konfiguriert ist, wobei die zweiten Implantatsensormesswerte eine zweite Last angeben, die durch die Wirbelsäule des Patienten auf die zweite intervertebrale Implantatvorrichtung (1430) ausgeübt wird;
Bestimmen, ob die erste Last und die zweite Last eine vorbestimmte Metrik einhalten;
Extrahieren, als Reaktion auf das Bestimmen, dass die erste Last oder die zweite Last die vorbestimmte Metrik nicht erfüllen, eines Merkmalsvektors aus den ersten und/oder zweiten Implantatsensormesswerten unter Verwendung eines Maschinenlernmoduls (116, 264) des Systems, wobei der Merkmalsvektor eine Zielkorrektur gemäß einem Korrekturplan zum Ausbalancieren der ersten Last und der zweiten Last unter Einhaltung der vorbestimmten Metrik angibt;
Erzeugen elektrischer Implantatsignale unter Verwendung des Maschinenlernmoduls (116, 264) und basierend auf dem Merkmalsvektor, wobei das Maschinenlernmodul basierend auf Patientendatensätzen trainiert wird, um die elektrischen Implantatsignale zu erzeugen, um die erste Last oder die zweite Last anzupassen; und
Übertragen der elektrischen Implantatsignale an mindestens einen von einem oder mehreren ersten Implantataktoren (1478) der ersten Implantatvorrichtung (1453) und einem oder mehreren zweiten Implantataktoren (1474) der zweiten intervertebralen Implantatvorrichtung (1430), um mindestens eine von der ersten Implantatvorrichtung oder der zweiten intervertebralen Implantatvorrichtung für die Zielkorrektur in eine unterschiedliche physische Konfiguration zu bewegen.

2. System gemäß Anspruch 1, wobei der Merkmalsvektor ferner mindestens eines von Lendenlordose, Cobb-Winkeln, koronalen Parametern, sagittalen Parametern, Beckenparametern, Bandscheibenhöhe, Segmentflexibilität, Knochenqualität oder Rotationsverschiebung der Wirbelsäule des Patienten angibt.

3. System gemäß Anspruch 1, wobei das Konfigurieren der ersten Implantatvorrichtung in der unterschiedlichen physischen Konfiguration Folgendes beinhaltet:
Anpassen mindestens eines von einer Schraube, einem Käfig, einer Platte, einer Stange, einer Scheibe, einem Abstandshalter, einer aufweitbaren Vorrichtung, einem Stent, einer Klammer, einer Zugstange, einem Gerüst, einer Fixierungsvorrichtung, einem Anker, einer Mutter, einem Bolzen, einem Niet, einem Verbindungselement, einem Halteseil, einem Befestigungselement oder einem Gelenkersatz der ersten Implantatvorrichtung unter Verwendung des einen oder der mehreren ersten Implantataktoren.

4. System gemäß Anspruch 1 wobei das Konfigurieren der ersten Implantatvorrichtung in der unterschiedlichen physischen Konfiguration Folgendes beinhaltet:
Anpassen eines mit der ersten Implantatvorrichtung gekoppelten Reservoirs, um eine Menge von mindestens einem von einem pharmakologischen Präparat, einem biologischen Präparat, einem biochemischen Präparat, einem Narkotikum oder einem Steroid, das dem Patienten verabreicht wird, zu modifizieren.

5. System gemäß Anspruch 1, wobei die Computeranweisungen bei Ausführung durch den einen oder die mehreren Computerprozessoren den einen oder die mehreren Computerprozessoren ferner zu Folgendem veranlassen:
Empfangen von Patientendaten;
Bestimmen einer anatomischen Konfiguration der Wirbelsäule des Patienten basierend auf den Patientendaten und
Identifizieren der Zielkorrektur basierend auf der anatomischen Konfiguration und einer verfügbaren Anpassbarkeit der ersten Implantatvorrichtung und der zweiten intervertebralen Implantatvorrichtung, wobei die Zielkorrektur verwendet wird, um den Merkmalsvektor zu extrahieren.

6. System gemäß Anspruch 1, wobei der Korrekturplan Kriterien zum Betätigen eines oder mehrerer erster Implantataktoren der ersten Implantatvorrichtung beinhaltet.

7. Ein nicht transitorisches, computerlesbares Speicherungsmedium (250), das Computeranweisungen speichert, die bei Ausführung durch einen oder mehrere Computerprozessoren (210) den einen oder die mehreren Computerprozessoren zu Folgendem veranlassen:
Empfangen erster Implantatsensormesswerte von einem oder mehreren ersten Implantatsensoren (1480) einer ersten Implantatvorrichtung (1453), die in einem Patienten implantiert ist und in einer ersten physischen Konfiguration konfiguriert ist, wobei die ersten Implantatsensormesswerte eine erste Last angeben, die durch eine Wirbelsäule des Patienten auf die erste Implantatvorrichtung ausgeübt wird;
Empfangen zweiter Implantatsensormesswerte von einem oder mehreren zweiten Implantatsensoren (1476) einer zweiten intervertebralen Implantatvorrichtung (1430), die in dem Patienten implantiert ist und in einer zweiten physischen Konfiguration konfiguriert ist, wobei die zweiten Implantatsensormesswerte eine zweite Last angeben, die durch die Wirbelsäule des Patienten auf die zweite intervertebrale Implantatvorrichtung ausgeübt wird;
Bestimmen, ob die erste Last und die zweite Last eine vorbestimmte Metrik einhalten;
Extrahieren, als Reaktion auf das Bestimmen, dass die erste Last oder die zweite Last die vorbestimmte Metrik nicht erfüllen, eines Merkmalsvektors aus den ersten und/oder zweiten Implantatsensormesswerten unter Verwendung eines Maschinenlernmoduls (116, 264), wobei der Merkmalsvektor eine Zielkorrektur zum Ausbalancieren der ersten Last und der zweiten Last unter Einhaltung der vorbestimmten Metrik angibt;
Erzeugen elektrischer Implantatsignale unter Verwendung des Maschinenlernmoduls (116, 264) und basierend auf dem Merkmalsvektor, wobei das Maschinenlernmodul basierend auf Patientendatensätzen trainiert wird, um die elektrischen Implantatsignale zu erzeugen, um die erste Last oder die zweite Last anzupassen; und
Übertragen der elektrischen Implantatsignale an mindestens einen von einem oder mehreren ersten Implantataktoren (1474) der ersten Implantatvorrichtung (1453) und einem oder mehreren zweiten Implantataktoren (1474) der zweiten intervertebralen Implantatvorrichtung (1430), um mindestens eine von der ersten Implantatvorrichtung (1453) oder der zweiten intervertebralen Implantatvorrichtung (1430) für die Zielkorrektur in eine unterschiedliche physische Konfiguration zu bewegen.

8. Nicht transitorisches, computerlesbares Speicherungsmedium gemäß Anspruch 7, wobei der Merkmalsvektor ferner mindestens eines von Lendenlordose, Cobb-Winkeln, koronalen Parametern, sagittalen Parametern, Beckenparametern, Bandscheibenhöhe, Segmentflexibilität, Knochenqualität oder Rotationsverschiebung der Wirbelsäule des Patienten angibt.

9. Nicht transitorisches, computerlesbares Speicherungsmedium gemäß Anspruch 7, wobei das Konfigurieren der ersten Implantatvorrichtung in der unterschiedlichen physischen Konfiguration Folgendes beinhaltet:
Anpassen mindestens eines von einer Schraube, einem Käfig, einer Platte, einer Stange, einer Scheibe, einem Abstandshalter, einer aufweitbaren Vorrichtung, einem Stent, einer Klammer, einer Zugstange, einem Gerüst, einer Fixierungsvorrichtung, einem Anker, einer Mutter, einem Bolzen, einem Niet, einem Verbindungselement, einem Halteseil, einem Befestigungselement oder einem Gelenkersatz der ersten Implantatvorrichtung unter Verwendung des einen oder der mehreren ersten Implantataktoren.

10. Nicht transitorisches, computerlesbares Speicherungsmedium gemäß Anspruch 7, wobei das Konfigurieren der ersten Implantatvorrichtung in der unterschiedlichen physischen Konfiguration Folgendes beinhaltet:
Anpassen eines mit der ersten Implantatvorrichtung gekoppelten Reservoirs, um eine Menge von mindestens einem von einem pharmakologischen Präparat, einem biologischen Präparat, einem biochemischen Präparat, einem Narkotikum oder einem Steroid, das dem Patienten verabreicht wird, zu modifizieren.

## Revendications

1. Un système (100), comprenant :
un ou plusieurs processeurs informatiques (210) ; et
un support de stockage non transitoire lisible par ordinateur (250) stockant des instructions informatiques qui, lorsqu'elles sont exécutées par les un ou plusieurs processeurs informatiques (210), amènent les un ou plusieurs processeurs informatiques à :
recevoir des premiers relevés de capteur d'implant en provenance d'un ou de plusieurs premiers capteurs d'implant (1480) d'un premier dispositif d'implant (1453) implanté dans un patient et configuré dans une première configuration physique, les premiers relevés de capteur d'implant étant indicatifs d'une première charge appliquée par une colonne vertébrale du patient sur le premier dispositif d'implant (1453) ;
recevoir des deuxièmes relevés de capteur d'implant en provenance d'un ou de plusieurs deuxièmes capteurs d'implant (1476) d'un deuxième dispositif d'implant intervertébral (1430) implanté dans le patient et configuré dans une deuxième configuration physique, les deuxièmes relevés de capteur d'implant étant indicatifs d'une deuxième charge appliquée par la colonne vertébrale du patient sur le deuxième dispositif d'implant intervertébral (1430) ;
déterminer si la première charge et la deuxième charge sont conformes à une métrique prédéterminée ;
extraire, en réponse à la détermination du fait que la première charge ou la deuxième charge n'atteignent pas la métrique prédéterminée, un vecteur de caractéristiques à partir des premiers et/ou deuxièmes relevés de capteur d'implant à l'aide d'un module d'apprentissage automatique (116, 264) du système, le vecteur de caractéristiques étant indicatif d'une correction cible selon un plan correctif destinée à équilibrer la première charge et la deuxième charge en conformité avec la métrique prédéterminée ;
générer des signaux électriques d'implant à l'aide du module d'apprentissage automatique (116, 264) et sur la base du vecteur de caractéristiques, le module d'apprentissage automatique étant entraîné sur la base d'ensembles de données de patient à générer les signaux électriques d'implant pour ajuster la première charge ou la deuxième charge ; et
transmettre les signaux électriques d'implant à au moins un actionneur parmi un ou plusieurs premiers actionneurs d'implant (1478) du premier dispositif d'implant (1453) et un ou plusieurs deuxièmes actionneurs d'implant (1474) du deuxième dispositif d'implant intervertébral (1430) pour déplacer au moins un dispositif parmi le premier dispositif d'implant et le deuxième dispositif d'implant intervertébral jusqu'à une configuration physique différente pour la correction cible.

2. Le système de la revendication 1, dans lequel le vecteur de caractéristiques est en outre indicatif d'au moins un item parmi une lordose lombaire, des angles de Cobb, des paramètres coronaux, des paramètres sagittaux, des paramètres pelviens, une hauteur discale, une flexibilité segmentaire, une qualité osseuse, ou un déplacement rotationnel de la colonne vertébrale du patient.

3. Le système de la revendication 1, dans lequel la configuration du premier dispositif d'implant dans la configuration physique différente comprend :
l'ajustement d'au moins un item parmi une vis, une cage, une plaque, une tige, un disque, un espaceur, un dispositif expansible, un stent, un boîtier (*bracket*), un lien (*tie*), un échafaudage, un dispositif de fixation, un ancrage, un écrou, un boulon, un rivet, un connecteur, une attache (*tether*), un organe de fixation et un remplacement articulaire du premier dispositif d'implant à l'aide des un ou plusieurs premiers actionneurs d'implant.

4. Le système de la revendication 1 dans lequel la configuration du premier dispositif d'implant dans la configuration physique différente comprend :
l'ajustement d'un réservoir couplé au premier dispositif d'implant pour modifier une quantité d'au moins un item parmi un produit pharmacologique, un produit biologique, un produit biochimique, un narcotique et un stéroïde administrée au patient.

5. Le système de la revendication 1 dans lequel les instructions informatiques, lorsqu'elles sont exécutées par les un ou plusieurs processeurs informatiques, amènent en outre les un ou plusieurs processeurs informatiques à :
recevoir des données de patient ;
déterminer une configuration anatomique de la colonne vertébrale du patient sur la base des données de patient, et
identifier la correction cible sur la base de la configuration anatomique et de l'ajustabilité disponible du premier dispositif d'implant et du deuxième dispositif d'implant intervertébral, la correction cible étant utilisée pour extraire le vecteur de caractéristiques.

6. Le système de la revendication 1, dans lequel le plan correctif comprend des critères pour l'actionnement d'un ou de plusieurs premiers actionneurs d'implant du premier dispositif d'implant.

7. Un support de stockage non transitoire lisible par ordinateur (250) stockant des instructions informatiques qui, lorsqu'elles sont exécutées par un ou plusieurs processeurs informatiques (210), amènent les un ou plusieurs processeurs informatiques à :
recevoir des premiers relevés de capteur d'implant en provenance d'un ou de plusieurs premiers capteurs d'implant (1480) d'un premier dispositif d'implant (1453) implanté dans un patient et configuré dans une première configuration physique, les premiers relevés de capteur d'implant étant indicatifs d'une première charge appliquée par une colonne vertébrale du patient sur le premier dispositif d'implant (1453) ;
recevoir des deuxièmes relevés de capteur d'implant en provenance d'un ou de plusieurs deuxièmes capteurs d'implant (1476) d'un deuxième dispositif d'implant intervertébral (1430) implanté dans le patient et configuré dans une deuxième configuration physique, les deuxièmes relevés de capteur d'implant étant indicatifs d'une deuxième charge appliquée par la colonne vertébrale du patient sur le deuxième dispositif d'implant intervertébral ;
déterminer si la première charge et la deuxième charge sont conformes à une métrique prédéterminée ;
extraire, en réponse à la détermination du fait que la première charge ou la deuxième charge n'atteignent pas la métrique prédéterminée, un vecteur de caractéristiques à partir des premiers et/ou deuxièmes relevés de capteur d'implant à l'aide d'un module d'apprentissage automatique (116, 264), le vecteur de caractéristiques étant indicatif d'une correction cible destinée à équilibrer la première charge et la deuxième charge en conformité avec la métrique prédéterminée ;
générer des signaux électriques d'implant à l'aide du module d'apprentissage automatique (116, 264) et sur la base du vecteur de caractéristiques, le module d'apprentissage automatique étant entraîné sur la base d'ensembles de données de patient à générer les signaux électriques d'implant pour ajuster la première charge ou la deuxième charge ; et
transmettre les signaux électriques d'implant à au moins un actionneur parmi un ou plusieurs premiers actionneurs d'implant (1474) du premier dispositif d'implant (1453) et un ou plusieurs deuxièmes actionneurs d'implant (1474) du deuxième dispositif d'implant intervertébral (1430) pour déplacer au moins un dispositif parmi le premier dispositif d'implant (1453) et le deuxième dispositif d'implant intervertébral (1430) jusqu'à une configuration physique différente pour la correction cible.

8. Le support de stockage non transitoire lisible par ordinateur de la revendication 7, dans lequel le vecteurs de caractéristiques est en outre indicatif d'au moins un item parmi une lordose lombaire, des angles de Cobb, des paramètres coronaux, des paramètres sagittaux, des paramètres pelviens, une hauteur discale, une flexibilité segmentaire, une qualité osseuse, et un déplacement rotationnel de la colonne vertébrale du patient.

9. Le support de stockage non transitoire lisible par ordinateur de la revendication 7, dans lequel la configuration du premier dispositif d'implant dans la configuration physique différente comprend :
l'ajustement d'au moins un item parmi une vis, une cage, une plaque, une tige, un disque, un espaceur, un dispositif expansible, un stent, un boîtier, un lien, un échafaudage, un dispositif de fixation, un ancrage, un écrou, un boulon, un rivet, un connecteur, une attache, un organe de fixation et un remplacement articulaire du premier dispositif d'implant à l'aide des un ou plusieurs premiers actionneurs d'implant.

10. Le support de stockage non transitoire lisible par ordinateur de la revendication 7, dans lequel la configuration du premier dispositif d'implant dans la configuration physique différente comprend :
l'ajustement d'un réservoir couplé au premier dispositif d'implant pour modifier une quantité d'au moins un produit parmi un produit pharmacologique, un produit biologique, un produit biochimique, un narcotique et un stéroïde administrée au patient.
